Europäisches Patentamt

European Patent Office

Office européen des brevets

(11) Publication number: **0 412 841 A1**

## EUROPEAN PATENT APPLICATION

(21) Application number: 90308826.8

(22) Date of filing: 10.08.90

(51) Int. Cl.⁵: **C12P 21/02, C12N 15/12,** C12N 1/21, C12N 5/10, A61K 37/02

The microorganism(s) has (have) been deposited with Agricultural Research Culture Collection under number(s) NRRL-B 15725, NRRL-B 18524 and NRRL-B 18525.

(30) Priority: 11.08.89 US 393617
05.02.90 US 474870

(43) Date of publication of application:
**13.02.91 Bulletin  91/07**

(84) Designated Contracting States:
**AT BE CH DE DK ES FR GB GR IT LI LU NL SE**

(71) Applicant: **ELI LILLY AND COMPANY**
**Lilly Corporate Center**
**Indianapolis Indiana 46285(US)**

(72) Inventor: **Bang, Nils Ulrik**
**706 East 71st Street**
**Indianapolis, Indiana 46220(US)**
Inventor: **Grinnell, Brian William**
**5038 Haynes Court**
**Indianapolis, Indiana 46250(US)**
Inventor: **Hoskins, Jo Ann**
**8229 Tern Court**
**Indianapolis, Indiana 46256(US)**
Inventor: **Moore, Robert Earl, Jr.**
**7419 Lantern Road**
**Indianapolis, Indiana 46256(US)**
Inventor: **Parkinson, John Francis**
**1308 Central Avenue, Apt. Nr. 308**
**Indianapolis, Indiana 46202(US)**

(74) Representative: **Hudson, Christopher Mark et al**
**Erl Wood Manor**
**Windlesham Surrey GU20 6PH(GB)**

(54) **Human thrombomodulin derivatives.**

(57) Soluble human thrombomodulin derivatives comprise the N-terminal, epidermal growth factor homology and serine/threonine-rich regions of human thrombomodulin but lack the transmembrane and cytoplasmic domains of human thrombomodulin. Recombinant vectors and host cells for the production of these derivatives are provided.

EP 0 412 841 A1

# HUMAN THROMBOMODULIN DERIVATIVES

The present invention provides novel human thrombomodulin derivatives and DNA compounds encoding these derivatives. Also provided are recombinant DNA vectors, and host cells transformed with these vectors, the host cells being useful for producing these novel proteins. These thrombomodulin derivatives possess unique properties which make them especially suitable as anticoagulant agents and useful in the treatment or prevention of thrombotic events.

To better understand the invention, the following brief description of the coagulation enzyme system is provided. The coagulation system, sometimes referred to as the "cascade", is best looked at as a chain reaction involving the sequential activation of zymogens into active serine proteases which eventually lead to the production of the enzyme, thrombin. Thrombin, through limited proteolysis, converts plasma fibrinogen into the insoluble gel, fibrin. Two key events in the coagulation cascade are the conversion of clotting Factor X to Xa by clotting factor IXa and the conversion of prothrombin into thrombin by clotting factor Xa.

Both of these reactions occur on cell surfaces, most notably the platelet surface, and both reactions require cofactors. The major cofactors, factors V and VIII, circulate as relatively inactive precursors, but when the first few molecules of thrombin are formed, thrombin activates, by limited proteolysis, the cofactors. The activated cofactors, Va and VIIIa, accelerate, by about five orders of magnitude, both the conversion of prothrombin into thrombin and the conversion of factor X to factor Xa.

Activated protein C overwhelmingly prefers two plasma protein substrates which it hydrolyzes and irreversibly destroys. These plasma protein substrates are the activated forms of clotting cofactors V and VIII (cofactors Va and VIIIa, respectively). Activated protein C only minimally degrades the inactive precursors, clotting factors V and VIII. In dogs, activated protein C has been shown to sharply increase circulating levels of the major physiological fibrinolytic enzyme, tissue plasminogen activator. Activated protein C has been shown in vitro to enhance lysis of fibrin in human whole blood, and recent experiments suggest that this effect is mediated through the interaction with an inhibitor of tissue plasminogen activator. Activated protein C, therefore, is an important in vivo antithrombotic and possibly fibrinolytic agent.

The activation of protein C, however, involves thrombin, the final serine protease in the coagulation cascade, and an endothelial cell membrane-associated glycoprotein, thrombomodulin. Thrombomodulin forms a tight 1:1 stoichiometric complex with thrombin. Thrombomodulin, when complexed with thrombin, modifies substantially the functional properties of thrombin. Thrombin, in the coagulation pathway, normally clots fibrinogen, activates platelets, and converts clotting cofactors V and VIII to their activated forms, Va and VIIIa. Thrombin, alone, acts to activate Protein C, but only very slowly and inefficiently. In contrast, thrombin, when in the 1:1 complex with thrombomodulin, fails to clot fibrinogen, does not activate platelets, and does not convert clotting factors V and VIII to their activated forms. The thrombin:thrombomodulin complex promotes the activation of protein C with the rate constant of protein C activation being as great as 20,000-fold higher for the thrombin:thrombomodulin complex than the rate constant for thrombin alone.

Activated protein C, therefore, is an antithrombotic agent with a wider therapeutic index than other anticoagulants, such as heparin and the oral hydroxycoumarin-type anticoagulants, such as warfarin. Neither protein C nor activated protein C is effective until thrombin is generated at some local site. Activated protein C is virtually ineffective without thrombin, because thrombin is needed to convert clotting factors V to Va and VIII to VIIIa. As noted, the activated forms of these two cofactors are the preferred substrate for activated protein C. The protein C zymogen, when infused into patients, will remain inactive until thrombin is generated and complexed with thrombomodulin. Without the thrombomodulin:thrombin complex, the protein C zymogen is not converted into activated Protein C.

Although the recombinant expression of human protein C is now possible ( See , U. S. Patent No. 4,755,624, issued October 4, 1988), control of its activation in vivo via the thrombin:thrombomodulin complex generally has been limited because thrombomodulin, the essential component for efficient protein C activation, has not been available in significant quantities. In particular, thrombomodulin, until recently, could be purified only in small quantities by a multi-step purification process involving affinity chromatography in which inactivated thrombin was immobilized on agarose. See , for example, Esmon, N. L. et al ., J. Biol. Chem. , 257 , 859 (1982); Suzuki, K. et al ., Biochim. Biophys. Acta , 882 , 343 (1986); and Maruyama, I. et al ., J. Clin. Invest. , 75 , 987 (1985). Purification is complicated further because thrombomodulin binds tightly to cell membranes via its transmembrane domain (see below), is relatively stable to denaturants and detergents and lacks sufficient solubility to be clinically useful as an exogenous antithrombotic agent.

Further, prior to the recent cloning and sequencing of a large portion of bovine thrombomodulin (Jackman, R., Proc. Natl. Acad. Sci. (U.S.A.) , 83 , 8834 (1986)), the structure of thrombomodulin was completely unknown. The entire gene sequence for human thrombomodulin now has been reported. Also, the human genome contains only a single copy of the thrombomodulin gene, which has been localized on human chromosome 20. Further studies reveal that this gene contains no introns suggesting that CDNA and genomic DNA encoding thrombomodulin are identical. ( See , Wen, D. et al ., Biochemistry , 26 , 4350 (1987); Suzuki, et al ., The EMBO Journal , 6 , 1891 (1987); and, Jackman, R. et al ., Proc. Natl. Acad. Sci. (USA) , 84 , 6425 (1987).

This information has allowed the determination of the basic structure of human thrombomodulin. Analysis shows that human thrombomodulin ("human TM") is synthesized as a 575 amino acid protein including a signal peptide portion which is reported to be of 16-, 18- or 21-residues in length. (Jackman, R. et al ., Proc. Natl. Acad. Sci. (USA) , 84 , 6425 (1987); Shirai, T. et al ., J. Biochem. , 103 , 281 (1988); and, Wen, et al ., supra , repectively). Following the signal peptide portion, human TM comprises, sequentially from the amino terminus, the following domains or regions: 1) an amino terminal domain (~223-226 amino acid residues); 2) six EGF ("epidermal growth factor")-like structures (referred to also as the "EGF-homology region"; ~236-240 amino acid residues); 3) a serine/threonine rich region in which several possible O-glycosylation sites are present (~34-37 amino acid residues); 4) a transmembrane region (~23-24 amino acid residues); and, 5) a cytoplasmic domain (~36-38 amino acid residues). One skilled in the art will recognize that the ranges of amino acid residues stated are the result of uncertainty about where a particular domain or region starts and ends. These ranges represent various values reported in the literature for the lengths of each of the domains or regions. See , for example, Suzuki, K. et al ., The EMBO Journal , 6 , 1891 (1987); Wen, D. et al ., supra . As used herein, therefore, "N-terminal region or domain", "epidermal growth factor homology region or domain", "serine/threonine-rich region or domain", "transmembrane region or domain" and "cytoplasmic region or domain" refer to the approximate range of amino acid residues noted above for each region or domain. Further, because in vivo processing will be expected to vary depending upon the expressing transformed host cell, especially a prokaryotic host cell compared to a euakryotic host cell, the term "N-terminal region or domain" optionally may include the human thrombomodulin signal peptide, or a portion thereof.

Because of the transmembrane region in wild-type thrombomodulin, the molecule is retained within cellular membranes. As a consequence, purification of the protein requires the undesirable use of detergents. In addition, the small quantity of purified material available from natural sources seriously hampers the use of wild-type thrombomodulin as a clinically-useful adjunctive anticoagulant or antithrombotic agent.

The present invention overcomes these difficulties by providing soluble thrombomodulin derivatives capable of being produced by recombinant DNA techniques. These derivatives can be obtained in virtually unlimited quantity, are easily handled, and possess desirable clinically-useful characteristics. Thus, these derivatives will provide clinicians with previously unavailable means for the treatment and prevention of thromboses. The benefits and characteristics of the proteins provided by the invention are described in detail below and in the examples.

Besides the terms defined above, for purposes of the present invention, as disclosed and claimed herein, the following terms are defined:

AD2LP--the adenovirus-2 late promoter.

Antibiotic--a substance produced by a microorganism that either naturally or with limited chemical modification, will inhibit the growth of or kill another microorganism or eukaryotic cell.

Antibiotic Resistance-Conferring Gene--a DNA segment that encodes an activity that confers resistance to an antibiotic.

Ap$^r$--the ampicillin-resistant phenotype or a gene conferring ampicillin-resistance.

BK--the BK enhancer element from the human papovavirus, BK Virus, said enhancer capable of increasing the level of transcription from a given promoter.

dhfr--the dihydrofolate reductase gene useful as a selectable marker in dhfr$^-$ cells and which can be used to amplify (increase the copy number of) a DNA segment by exposing the host cell to increasing levels of methotrexate.

EP--a DNA segment comprising the SV40 early promoter of the T-antigen (F) gene, the T-antigen binding sites, and the SV40 origin of replication.

Eukaryotic promoter--any DNA sequence that functions as a promoter in eukaryotic cells.

Host cell--an organism, including prokaryotes and eukaryotes, which can be transformed with a recombinant DNA vector. The term includes, but is not limited to, mammalian cells, as well as viable protoplasts, for example, of Streptomyces.

Hm$^r$--the hygromycin-resistant phenotype or a gene conferring hygromycin-resistance. IVS--DNA encoding an intron, also called an intervening sequence.

MCS--multiple cloning site, also called a polylinker.

ori--a plasmid origin of replication. pA--A DNA sequence encoding a polyadenylation signal.

Promoter--A DNA sequence that directs transcription of DNA into RNA.

Recombinant DNA Vector--any recombinant DNA cloning or expression vector.

Recombinant DNA Cloning Vector--any autonomously replicating or chromosomally integrating agent that comprises a DNA molecule to which one or more additional DNA segments can be or have been added. The term includes, but is not limited to, plasmids, cosmids and phage vectors.

Recombinant DNA Expression Vector--any recombinant DNA cloning vector comprising a promoter and which is capable of expressing a DNA molecule which is part of, or can be inserted into, the vector.

Replicon--a DNA sequence that controls and allows for autonomous replication of a recombinant DNA vector.

Restriction Fragment--any linear DNA generated by the action of one or more restriction enzymes.

Sensitive Host Cell--a host cell that cannot grow in the presence of a given antibiotic or other toxic compound without a DNA segment that confers resistance thereto.

Transformation--the introduction of DNA into a recipient host cell, including a viable protoplast thereof, such that the genotype of the recipient cell is changed.

Transformant--a recipient host cell that has undergone transformation.

Zymogen--an enzymatically inactive precursor of a proteolytic enzyme.

Figure 1 is a flowchart outlining the construction of phdTMD1.

Figure 2 is a restriction site and function map of plasmid pUC18.

Figure 3 is a restriction site and function map of plasmid pUC18TMlinker.

Figure 4 is a restriction site and function map of plasmid pUC18TM.

Figure 5 is a restriction site and function map of plasmid pUC18TMD1.

Figure 6 is a restriction site and function map of plasmid phd.

Figure 7 is a restriction site and function map of plasmid phdTMD1.

Those skilled in the art will appreciate that the representative figures are drawn approximately to scale. The spacing of restriction sites on the map is not exact and actual restriction sites on the vector may vary somewhat from calculated distances. The maps do not provide an exhaustive listing of all the restriction sites on a given vector. These drawings are provided to aid the reader in better understanding the invention.

The present invention provides soluble human thrombomodulin derivatives which are amino acid sequences which, in order from the N-terminus, comprise:

a) the N-terminal;

b) the epidermal growth factor homology; and,

c) serine/threonine rich regions of human thrombomodulin, said amino acid sequence lacking the transmembrane and cytoplasmic domains of human thrombomodulin, said amino acid sequences derivable from an AV12 or 293 host cell transformed with a recombinant DNA vector encoding said amino acid sequence.

In a preferred embodiment of the invention, the amino acid sequence of the soluble thrombomodulin derivative is the amino acid sequence encoded by the thrombomodulin derivative encoding sequence of plasmid pUC18TMD1 or plasmid phdTMD1.

In this preferred embodiment, the amino acid sequence of the soluble thrombomodulin derivative is:

4

EP 0 412 841 A1

H₂N-(R)ₓ(R¹)ᵧ-AlaProAlaGluProGlnProGlyGlySerGlnCysValGluHisAsp
CysPheAlaLeuTyrProGlyProAlaThrPheLeuAsnAlaSerGlnIle
CysAspGlyLeuArgGlyHisLeuMetThrValArgSerSerValAlaAla
AspValIleSerLeuLeuLeuAsnGlyAspGlyGlyValGlyArgArgArg
LeuTrpIleGlyLeuGlnLeuProProGlyCysGlyAspProLysArgLeu
GlyProLeuArgGlyPheGlnTrpValThrGlyAspAsnAsnThrSerTyr
SerArgTrpAlaArgLeuAspLeuAsnGlyAlaProLeuCysGlyProLeu
CysValAlaValSerAlaAlaGluAlaThrValProSerGluProIleTrp
GluGluGlnGlnCysGluValLysAlaAspGlyPheLeuCysGluPheHis
PheProAlaThrCysArgProLeuAlaValGluProGlyAlaAlaAlaAla
AlaValSerIleThrTyrGlyThrProPheAlaAlaArgGlyAlaAspPhe
GlnAlaLeuProValGlySerSerAlaAlaValAlaProLeuGlyLeuGln
LeuMetCysThrAlaProProGlyAlaValGlnGlyHisTrpAlaArgGlu
AlaProGlyAlaTrpAspCysSerValGluAsnGlyGlyCysGluHisAla
CysAsnAlaIleProGlyAlaProArgCysGlnCysProAlaGlyAlaAla
LeuGlnAlaAspGlyArgSerCysThrAlaSerAlaThrGlnSerCysAsn
AspLeuCysGluHisPheCysValProAsnProAspGlnProGlySerTyr
SerCysMetCysGluThrGlyTyrArgLeuAlaAlaAspGlnHisArgCys
GluAspValAspAspCysIleLeuGluProSerProCysProGlnArgCys
ValAsnThrGlnGlyGlyPheGluCysHisCysTyrProAsnTyrAspLeu
ValAspGlyGluCysValGluProValAspProCysPheArgAlaAsnCys
GluTyrGlnCysGlnProLeuAsnGlnThrSerTyrLeuCysValCysAla
GluGlyPheAlaProIleProHisGluProHisArgCysGlnMetPheCys
AsnGlnThrAlaCysProAlaAspCysAspProAsnThrGlnAlaSerCys
GluCysProGluGlyTyrIleLeuAspAspGlyPheIleCysThrAspIle
AspGluCysGluAsnGlyGlyPheCysSerGlyValCysHisAsnLeuPro
GlyThrPheGluCysIleCysGlyProAspSerAlaLeuAlaArgHisIle
GlyThrAspCysAspSerGlyLysValAspGlyGlyAspSerGlySerGly

GluProProProSerProThrProGlySerThrLeuThrProProAlaVal
GlyLeuValHisSer-COOH

wherein
ALA is Alanine, ARG is Arginine, ASN is Asparagine, ASP is Aspartic Acid, CYS is Cysteine, GLN is Glutamine, GLU is Glutamic Acid, GLY is Glycine, HIS is Histidine, ILE is Isoleucine, LEU is Leucine, LYS is Lysine, MET is Methionine, PHE is Phenylalanine, PRO is Proline, SER is Serine, THR is Threonine, TRP is Tryptophan, TYR is Tyrosine, and VAL is Valine;
R is MetLeuGlyValLeuValLeuGlyAlaLeuAlaLeuAla GlyLeuGly-;
R¹ is PhePro-;
x is 0 or 1;
y is 0 or 1, provided that if y = 0, then x must be 0 and if x = 1, then y must be 1.
   In a particularly preferred embodiment of the invention, the amino acid sequence of the soluble

5

thrombomodulin derivative is:

```
H₂N-AlaProAlaGluProGlnProGlyGlySerGlnCysValGluHisAspCys
     PheAlaLeuTyrProGlyProAlaThrPheLeuAsnAlaSerGlnIleCys
     AspGlyLeuArgGlyHisLeuMetThrValArgSerSerValAlaAlaAsp
     ValIleSerLeuLeuLeuAsnGlyAspGlyGlyValGlyArgArgArgLeu
     TrpIleGlyLeuGlnLeuProProGlyCysGlyAspProLysArgLeuGly
     ProLeuArgGlyPheGlnTrpValThrGlyAspAsnAsnThrSerTyrSer
     ArgTrpAlaArgLeuAspLeuAsnGlyAlaProLeuCysGlyProLeuCys
     ValAlaValSerAlaAlaGluAlaThrValProSerGluProIleTrpGlu
     GluGlnGlnCysGluValLysAlaAspGlyPheLeuCysGluPheHisPhe
     ProAlaThrCysArgProLeuAlaValGluProGlyAlaAlaAlaAlaAla
     ValSerIleThrTyrGlyThrProPheAlaAlaArgGlyAlaAspPheGln
     AlaLeuProValGlySerSerAlaAlaValAlaProLeuGlyLeuGlnLeu
     MetCysThrAlaProProGlyAlaValGlnGlyHisTrpAlaArgGluAla
     ProGlyAlaTrpAspCysSerValGluAsnGlyGlyCysGluHisAlaCys
     AsnAlaIleProGlyAlaProArgCysGlnCysProAlaGlyAlaAlaLeu
     GlnAlaAspGlyArgSerCysThrAlaSerAlaThrGlnSerCysAsnAsp
     LeuCysGluHisPheCysValProAsnProAspGlnProGlySerTyrSer
     CysMetCysGluThrGlyTyrArgLeuAlaAlaAspGlnHisArgCysGlu
     AspValAspAspCysIleLeuGluProSerProCysProGlnArgCysVal
     AsnThrGlnGlyGlyPheGluCysHisCysTyrProAsnTyrAspLeuVal
     AspGlyGluCysValGluProValAspProCysPheArgAlaAsnCysGlu
     TyrGlnCysGlnProLeuAsnGlnThrSerTyrLeuCysValCysAlaGlu
     GlyPheAlaProIleProHisGluProHisArgCysGlnMetPheCysAsn
     GlnThrAlaCysProAlaAspCysAspProAsnThrGlnAlaSerCysGlu
     CysProGluGlyTyrIleLeuAspAspGlyPheIleCysThrAspIleAsp
     GluCysGluAsnGlyGlyPheCysSerGlyValCysHisAsnLeuProGly
     ThrPheGluCysIleCysGlyProAspSerAlaLeuAlaArgHisIleGly
     ThrAspCysAspSerGlyLysValAspGlyGlyAspSerGlySerGlyGly


     ProProProSerProThrProGlySerThrLeuThrProProAlaValGly
     LeuValHisSer-COOH
```

wherein
ALA is Alanine, ARG is Arginine, ASN is Asparagine, ASP is Aspartic Acid, CYS is Cysteine, GLN is Glutamine, GLU is Glutamic Acid, GLY is Glycine, HIS is Histidine, ILE is Isoleucine, LEU is Leucine, LYS is Lysine, MET is Methionine, PHE is Phenylalanine, PRO is Proline, SER is Serine, THR is Threonine, TRP is Tryptophan, TYR is Tyrosine, and VAL is Valine.

One will note that in a preferred embodiment of the invention, the thrombomodulin derivatives provided are produced by recombinant technology means, preferably by the transformation of a eukaryotic host cell with a recombinant DNA expression vector encoding the thrombomodulin derivative followed by culturing the cell under conditions suitable for expression of the derivatives of the invention. The expression vector, of course, should be constructed so that the DNA sequence encoding the TM derivatives is properly positioned for expression and in proper translational reading frame in relation to the translation start site. Further, the start site can be derived from the inserted DNA sequence or can be derived from other sources. This sequence can be either heterologous or homologous to the vector, itself, if naturally-occurring, or homologous or heterologous to the promoter used in the construction. For selection purposes, the expression vector can comprise a selectable marker and transformation can be in a host cell normally sensitive to the toxic substance used for selection in the absence of the marker gene.

In this latter embodiment, expression in a eukaryotic cell, one skilled in the art will appreciate that glycosylation of the product is likely to occur. Thus, the invention also provides the thrombomodulin derivatives not only in non-glycosylated form, such as would be expected if the polypeptide was expressed in a prokaryotic microbial host cell, but also in glycosylated form. As used herein, "glycosylation" refers to the addition of one or more sugar moieties to the expressed protein. In addition, as those skilled in the art will appreciate, eukaryotic expression often results in the secretion of the product from the host cell, especially when a signal peptide portion of the expressed polypeptide is present. In addition, other biological processing, for example, N-terminal blocking or subsequent cleavage, or possibly C-terminal cleavage, may occur in a eukaryotic host cell which renders difficult the identification of the final expressed protein. Therefore, in an additional preferred embodiment of the invention, there is provided a polypeptide produced by culturing, under conditions suitable for expression, a host cell, said host cell transformed with a recombinant DNA expression vector comprising the thrombomodulin derivative encoding sequence of plasmid pUC18TMD1 or plasmid phdTMD1. Such a DNA sequence encoding a human thrombomodulin derivative is the following:

```
                10                    30                    50
                 .                     .                     .
   5'-ATGCTTGGGGTCCTGGTCCTTGGCGCGCTGGCCCTGGCCGGCCTGGGGGTTC

                        70                    90
                         .                     .
      CCCGCACCCGCAGAGCCGCAGCCGGGTGGCAGCCAGTGCGTCGAGCACGAC

                110                   130                   150
                 .                     .                     .
      TGCTTCGCGCTCTACCCGGGCCCCGCGACCTTCCTCAATGCCAGTCAGATC

                        170                   190
                         .                     .
      TGCGACGGACTGCGGGGCCACCTAATGACAGTGCGCTCCTCGGTGGCTGCC

                210                   230                   250
                 .                     .                     .
      GATGTCATTTCCTTGCTACTGAACGGCGACGGCGGCGTTGGCCGCCGGCGC

                        270                   290
                         .                     .
      CTCTGGATCGGCCTGCAGCTGCCACCCGGCTGCGGCGACCCCAAGCGCCTC

                310                   330                   350
                 .                     .                     .
      GGGCCCCTGCGCGGCTTCCAGTGGGTTACGGGAGACAACAACACCAGCTAT

                        370                   390                    4
                         .                     .                     .
      AGCAGGTGGGCACGGCTCGACCTCAATGGGGCTCCCCTCTGCGGCCCGTTG

                10                    430                   450
                 .                     .                     .
      TGCGTCGCTGTCTCCGCTGCTGAGGCCACTGTGCCCAGCGAGCCGATCTGG
```

7

470                    490                      510
GAGGAGCAGCAGTGCGAAGTGAAGGCCGATGGCTTCCTCTGCGAGTTCCAC

               530                    550
TTCCCAGCCACCTGCAGGCCACTGGCTGTGGAGCCCGGCGCCGCGGCTGCC

570                    590                      610
GCCGTCTCGATCACCTACGGCACCCCGTTCGCGGCCCGCGGAGCGGACTTC

               630                    650
CAGGCGCTGCCGGTGGGCAGCTCCGCCGCGGTGGCTCCCCTCGGCTTACAG

670                    690                      710
CTAATGTGCACCGCGCCGCCCGGAGCGGTCCAGGGGCACTGGGCCAGGGAG

               730                    750
GCGCCGGGCGCTTGGGACTGCAGCGTGGAGAACGGCGGCTGCGAGCACGCG

770                    790                      810
TGCAATGCGATCCCTGGGGCTCCCCGCTGCCAGTGCCCAGCCGGCGCCGCC

               830                    850
CTGCAGGCAGACGGGCGCTCCTGCACCGCATCCGCGACGCAGTCCTGCAAC

870                    890                      910
GACCTCTGCGAGCACTTCTGCGTTCCCAACCCCGACCAGCCGGGCTCCTAC

               930                    950                      97
TCGTGCATGTGCGAGACCGGCTACCGGCTGGCGGCCGACCAACACCGGTGC

0                      990                      1010
GAGGACGTGGATGACTGCATACTGGAGCCCAGTCCGTGTCCGCAGCGCTGT

```
            1030              1050              1070
GTCAACACACAGGGTGGCTTCGAGTGCCACTGCTACCCTAACTACGACCTG

            1090              1110
GTGGACGGCGAGTGTGTGGAGCCCGTGGACCCGTGCTTCAGAGCCAACTGC

        1130              1150              1170
GAGTACCAGTGCCAGCCCCTGAACCAAACTAGCTACCTCTGCGTCTGCGCC

            1190              1210
GAGGGCTTCGCGCCCATTCCCCACGAGCCGCACAGGTGCCAGATGTTTTGC

    1230              1250              1270
AACCAGACTGCCTGTCCAGCCGACTGCGACCCCAACACCCAGGCTAGCTGT

        1290              1310
GAGTGCCCTGAAGGCTACATCCTGGACGACGGTTTCATCTGCACGGACATC

    1330              1350              1370
GACGAGTGCGAAAACGGCGGCTTCTGCTCCGGGGTGTGCCACAACCTCCCC

        1390              1410              14
GGTACCTTCGAGTGCATCTGCGGGCCCGACTCGGCCCTTGCCCGCCACATT

30              1450              1470
GGCACCGACTGTGACTCCGGCAAGGTGGACGGTGGCGACAGCGGCTCTGGC

        1490              1510              1530
GAGCCCCCGCCCAGCCCGACGCCCGGCTCCACCTTGACTCCTCCGGCCGTG

GGGCTCGTGCATTCG-3',
```

wherein
A is deoxyadenyl, G is deoxyguanyl, C is deoxycytidyl, and T is thymidyl.

The preferred host cell for this embodiment is a eukaryotic host cell, preferably a mammalian cell. The most preferred host cell is either a human 293 ("293 cell") or Syrian Hamster AV12-664 cell ("AV12"). In this embodiment, as described in more detail below, two forms of the polypeptide have been observed, particularly when the derivatives are expressed in the 293 or AV12 cells. These polypeptides are designated the high molecular weight thrombomodulin derivative and the low molecular weight thrombomodulin derivative. The high molecular weight form has a molecular weight of about 95 to about 110 kD (reducing conditions) and about 76-94 kD (non-reducing conditions). Upon chemical deglycosylation with anhydrous TFMS (trifluoromethanesulfonic acid) ( See Sojar, et al ., Arch Biochem. Biophys . 259 , 52 (1987)), the high molecular weight form produced a protein of about 68kD and 65kD (under reducing conditions), after two and three hours, respectively. In addition, this form is susceptible to chondroitinase ABC cleavage to produce a protein which prolongs thrombin clotting time but at a level less than that of the untreated form. The susceptibility to chondroitinase treatment suggests that the high molecular weight form of the soluble thrombomodulin derivative of the invention may possess a glyco saminoglycan moiety. Further, this form has the following additional characteristics:

a) an optimal $Ca^{++}$ concentration of about 1.0 to about 5.0 mM; and

b) a $K_d$ for thrombin of about 2.0 to about 3.0 nM

The low molecular weight form has a molecular weight of about 73 to about 77 kD (reducing conditions) and about 58-66 kD (non-reducing conditions). Upon TFMS deglycosylation, this form of the thrombomodulin derivative produced, after 3 hours, a protein of about 63 to about 64 kD (reducing conditions). This derivative also has the following characteristics:

a) an optimal $Ca^{++}$ concentration of about .1 to about .5 mM; and,

b) a $K_d$ for thrombin of about 10.0 to about 25.0 nM.

Also provided by the invention are DNA sequences, recombinant DNA vectors, host cells and a method for producing the desired soluble thrombomodulin derivatives of the invention. These additional aspects of the invention will be defined in more detail below.

The DNA sequence encoding the thrombomodulin derivatives of the invention is the following:

$$5'-(R')_x(R^{1'})_y\text{-GCACCCGCAGAGCCGCAGCCGGGTGGCAGCCAGTGCGTCGAGCACGAC}$$

TGCTTCGCGCTCTACCCGGGCCCCGCGACCTTCCTCAATGCCAGTCAGATC

TGCGACGGACTGCGGGGCCACCTAATGACAGTGCGCTCCTCGGTGGCTGCC

GATGTCATTTCCTTGCTACTGAACGGCGACGGCGGCGTTCGCCGCCGGCGC

CTCTGGATCGGCCTGCAGCTGCCACCCGGCTGCGGCGACCCCAAGCGCCTC

GGGCCCCTGCGCGGCTTCCAGTGGGTTACGGGAGACAACAACACCAGCTAT

AGCAGGTGGGCACGGCTCGACCTCAATGGGGCTCCCCTCTGCGGCCCGTTG

TGCGTCGCTGTCTCCGCTGCTGAGGCCACTGTGCCCAGCGAGCCGATCTGG

GAGGAGCAGCAGTGCGAAGTGAAGGCCGATGGCTTCCTCTGCGAGTTCCAC

GAGGAGCAGCAGTGCGAAGTGAAGGCCGATGGCTTCCTCTGCGAGTTCCAC

TTCCCAGCCACCTGCAGGCCACTGGCTGTGGAGCCCGGCGCCGCGGCTGCC

```
                 570                      590                        610
                  .                        .                         .
         GCCGTCTCGATCACCTACGGCACCCCGTTCGCGGCCCGCGGAGCGGACTTC

                       630                      650
                        .                        .              .
         CAGGCGCTGCCGGTGGGCAGCTCCGCCGCGGTGGCTCCCCTCGGCTTACAG

             670                      690                      710
              .                        .                        .
         CTAATGTGCACCGCGCCGCCCGGAGCGGTCCAGGGGCACTGGGCCAGGGAG

                       730                      750
              .                        .              .            .
         GCGCCGGGCGCTTGGGACTGCAGCGTGGAGAACGGCGGCTGCGAGCACGCG

             770                      790                      810
              .                        .                        .
         TGCAATGCGATCCCTGGGGCTCCCCGCTGCCAGTGCCCAGCCGGCGCCGCC

                       830                      850
              .            .              .            .            .
         CTGCAGGCAGACGGGCGCTCCTGCACCGCATCCGCGACGCAGTCCTGCAAC

         870                      890                      910
          .            .              .            .
         GACCTCTGCGAGCACTTCTGCGTTCCCAACCCCGACCAGCCGGGCTCCTAC

                       930                      950                     97
              .            .              .            .            .
         TCGTGCATGTGCGAGACCGGCTACCGGCTGGCGGCCGACCAACACCGGTGC

         0                       990                      1010
          .                        .                        .       .
         GAGGACGTGGATGACTGCATACTGGAGCCCAGTCCGTGTCCGCAGCGCTGT

             1030                     1050                     1070
              .                        .                        .
         GTCAACACACAGGGTGGCTTCGAGTGCCACTGCTACCCTAACTACGACCTG

                       1090                     1110
              .            .              .            .            .
         GTGGACGGCGAGTGTGTGGAGCCCGTGGACCCGTGCTTCAGAGCCAACTGC
```

1130            1150            1170

```
GAGTACCAGTGCCAGCCCCTGAACCAAACTAGCTACCTCTGCGTCTGCGCC
```

1190            1210

```
GAGGGCTTCGCGCCCATTCCCCACGAGCCGCACAGGTGCCAGATGTTTTGC
```

1230            1250            1270

```
AACCAGACTGCCTGTCCAGCCGACTGCGACCCCAACACCCAGGCTAGCTGT
```

1290            1310

```
GAGTGCCCTGAAGGCTACATCCTGGACGACGGTTTCATCTGCACGGACATC
```

1330            1350            1370

```
GACGAGTGCGAAAACGGCGGCTTCTGCTCCGGGGTGTGCCACAACCTCCCC
```

1390            1410            14

```
GGTACCTTCGAGTGCATCTGCGGGCCCGACTCGGCCCTTGCCCGCCACATT
```

30            1450            1470

```
GGCACCGACTGTGACTCCGGCAAGGTGGACGGTGGCGACAGCGGCTCTGGC
```

1490            1510            1530

```
GAGCCCCCGCCCAGCCCGACGCCCGGCTCCACCTTGACTCCTCCGGCCGTG
```

```
GGGCTCGTGCATTCG-3',
```

in which

R′ is 5′-ATGCTTGGGGTCCTGGTCCTTGGCGCGCTG GCCCTGGCCGGCCTGGGG-3′;

R1′ is 5′-TTCCCC-3′;

x is 0 or 1;

y is 0 or 1, provided that if y = 0, then x must be 0, and if x = 1, then y must be 1;

A is deoxyadenyl,

G is deoxyguanyl,

C is deoxycytidyl, and T is thymidyl.

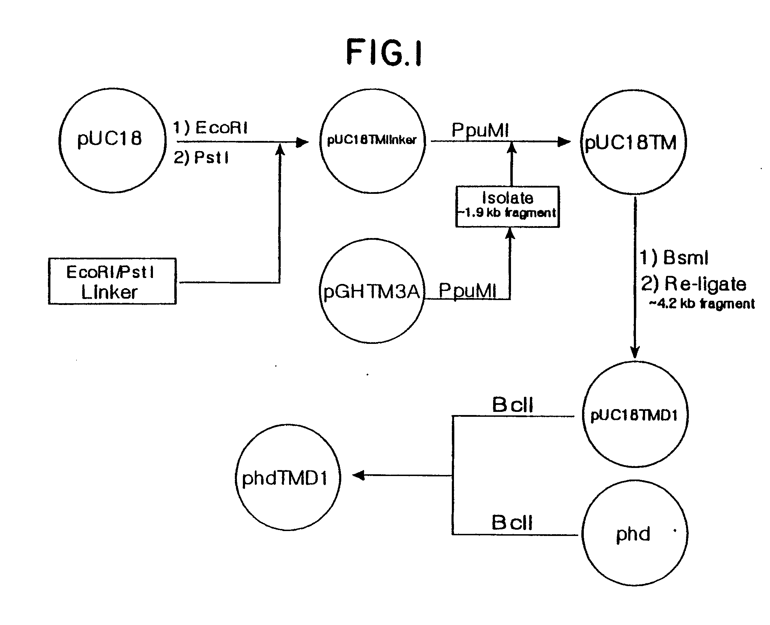

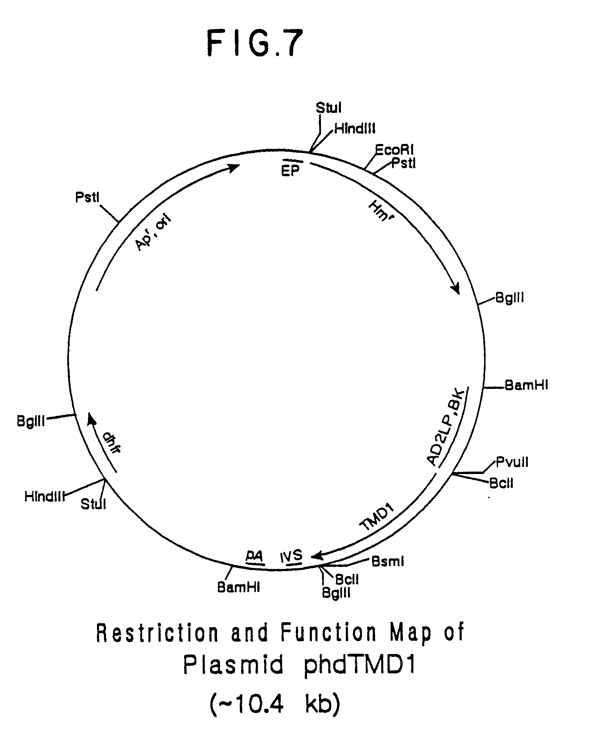

As noted, the derivatives of the invention preferably are prepared using recombinant DNA techniques. Thus, the invention provides novel DNA sequences, recombinant DNA vectors and host cells for expressing the desired products. As noted earlier, a preferred method for preparing the soluble thrombomodulin derivatives is by culturing a eukaryotic host cell with an expression vector containing the desired DNA sequence. The preferred recombinant expression vector for this purpose is plasmid phdTMD1, the restriction site and function map of which is provided in Figure 7. The overall construction of plasmid phdTMD1 is outlined in the flowchart of Figure 1. Each of the steps in this construction will be discussed in detail.

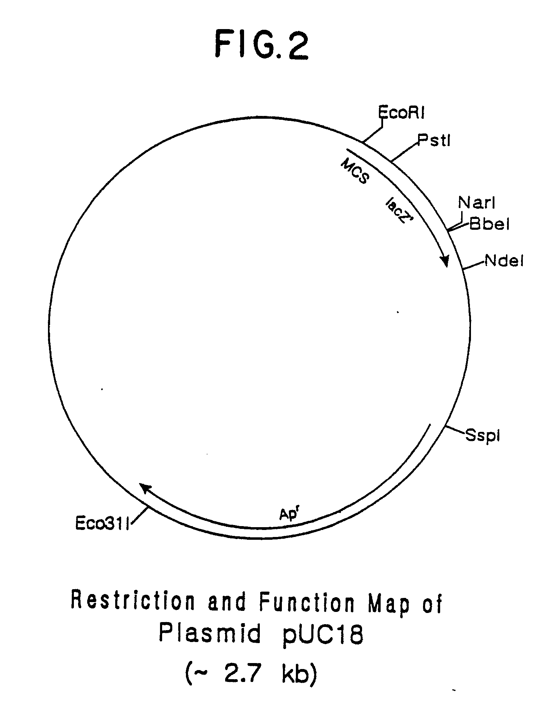

Starting plasmid, plasmid pUC18, as well-known by those skilled in the art, is a commercially available ( e.g. from Boehringer-Mannheim, Indianapolis, IN) recombinant DNA cloning vector containing a multiple cloning site ("polylinker") within its included lac gene. Alternatively, one skilled in the art could use plasmid pUC19, also commercially available. In addition, these plasmids comprise a modified ampicillin-resistance gene and origin of replication derived from pBR322. These vectors are especially useful because they allow the cloning of doubly digested DNA restriction fragments, separately and in the desired orientation with respect to the LAC promoter. The restriction site and function map of pUC18 is provided in Figure 2.

The specific scheme outlined in Figure 1 for preparing plasmid phdTMD1 requires the presence of several restriction sites not present within the TM derivative coding sequence. Therefore, a linker sequence, designated the Eco RI-Pst I linker, was prepared. The sequence with its restriction sites marked is the following:

```
         BclI                    BsmI                      PstI

     EcoRI            PpuMI                        BclI
5'-AATTCTGATCATGCTTGGGGTCCTTGCATTCGTAATTAATTAATGATCACTGCA-3'
   IIIIIIIIIIIIIIIIIIIIIIIIIIIIIIIIIIIIIIIIIIIIIIIIIIIIII
3'-GACTAGTACGAACCCCAGGAACGTAAGCATTAATTAATTACTAGTG-5'
```

Each of the noted restriction sites will be useful in constructing plasmid phdTMD1 as will become clearer by reference to Figure 1 and the discussion below.

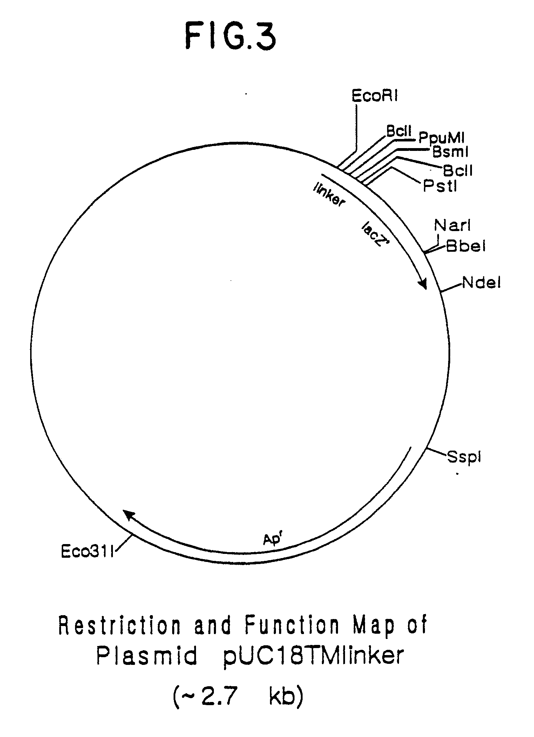

Intermediate vector, pUC18TMlinker then is prepared by doubly digesting pUC18 with restriction enzymes Eco RI and Pst I, followed by ligation of the resulting DNA with the Eco RI-Pst I linker described above. The restriction site and function map of the resulting vector, pUC18TMlinker, is provided in Figure 3.

The coding sequence for the soluble thrombomodulin derivatives of the invention is derived from genomic DNA encoding the entire thrombomodulin gene. This gene has been localized on chromosome 20 of the human genome and is reported to be intron-free. ( See , Wen D. et al . Biochemistry , 26 , 4350 (1987); and, Jackman, R. et al ., Proc. Natl. Acad. Sci.(USA) , 84 , 6425 (1987)). Using oligonucleotide probes designed and based upon the bovine thrombomodulin DNA sequence ( See , Jackman, R. et al , Proc. Natl. Acad. Sci. (USA) , 83 , 8834 (1986)), a clone, GHTM3A, carrying the entire human thrombomodulin gene, was isolated from a human chromosome 20 library carried in lambda phage Charon 21A. This chromosomal library is available commercially from the American Type Culture Collection (ATCC), 12301 Parklawn Drive, Rockville MD 20852, under the accession number ATCC 57712 (ID Code LL20NS01).

Once obtained, GHTM3A was found to contain the entire thrombomodulin gene on an ~6.4 kb Hin dIII fragment packaged in the Charon 21A vector. Thus, the entire coding region is obtained by treating the clone with Hin dIII restriction endonuclease and isolating the ~6.4 kb restriction fragment.

The ~6.4 kb Hin dIII fragment from GHTM3A then was ligated to Hin dIII-digested puc19. The resulting plasmid, designated plasmid pGHTM3A, was isolated and used for further constructions.

The next step in the construction of phdTMD1 involves obtaining the ~1.9 kb Ppu MI restriction fragment from plasmid pGHTM3A. This fragment also contains all but the first few base pairs of the human thrombomodulin coding region. The missing pairs are reconstructed by ligating the ~1.9 kb Ppu MI fragment of pGHTM3A to Ppu MI digested pUC18TMlinker, as described below. The Ppu MI sites for isolating the human TM sequence, however, overlap the dcm methylase recognition sequence, 5'-CCTGG-3'. Methylation of the internal cytosine of this sequence would block cleavage by Ppu MI. One skilled in the art, therefore, will recognize that cloning of the desired sequences must be performed in dcm ⁻ cells. A preferred host for this purpose is E. coli K12 GM48, deposited November 23, 1983, now available under the terms of the Budapest Treaty from the permanent stock culture collection of the Northern Regional Research Laboratory ("NRRL"), 1815 North University Street, Peoria, Illinois 61604, under the accession number NRRL B-15725. For later purposes, one skilled in the art will note that this strain is also dam ⁻.

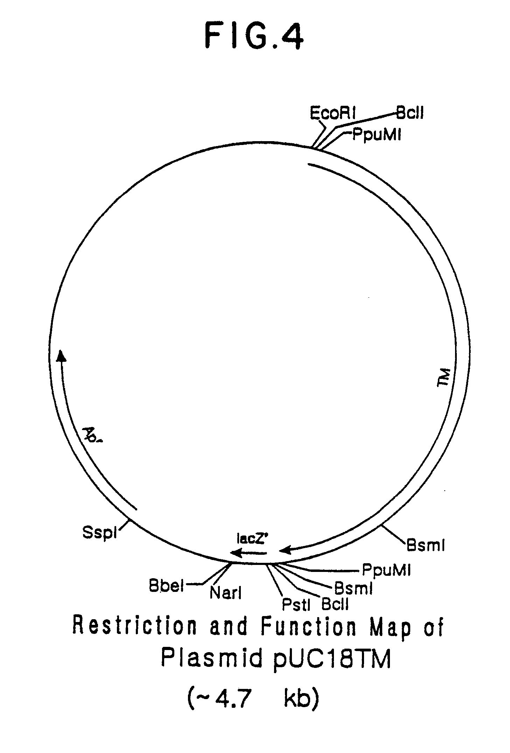

Plasmid pGHTM3A is isolated from the E. coli K12 GM48 transformants, is treated with restriction enzyme Ppu MI, and the ~1.9 kb restriction fragment, containing the human TM gene, is isolated. Plasmid pUC18TMlinker, prepared in the manner described above, likewise is treated with restriction enzyme Ppu MI. The resulting DNA was isolated and then ligated to the ~1.9 kb Ppu MI restriction fragment of pGHTM3A. The recircularized plasmid, designated pUC18TM, was isolated and contains the entire human TM coding sequence. Plasmid pUC18TM has been transformed into E. coli K12 DH5αF' as the preferred source and stock reservoir of the plasmid. The plasmid can be isolated, using conventional means, from this strain which has been deposited, under the terms of the Budapest Treaty, and made a part of the permanent stock culture collection of the NRRL. This strain is available under the accession number NRRL B-18524 deposited July 20, 1989. A restriction site and function map of plasmid pUC18TM is provided in Figure 4 of the drawings.

Plasmid pUC18TMD1 comprises the sequence ("TMD1") encoding the soluble thrombomodulin derivatives of the invention. This vector is prepared by deleting from the full length TM sequence located on pUC18TM about 500 base pairs from the 3'-end of the gene. The deletion is performed by treating pUC18TM with restriction enzyme Bsm I followed by recircularization. This deletion occurs at the serine/threonine rich/transmembrane domain junction, effectively cleaving the transmembrane and cytoplasmic encoding regions from the vector. Based upon published sequences for human thrombomodulin, this cleavage, upon religation, would be expected to produce upon expression of the encoded protein, and absent any additional in vivo processing, a polypeptide with the following structure:

Signal Peptide/NH$_2$-terminal region/EGF homology region/Threonine/Serine region/. . ./ - ProAlaValGlyLeuValHisSer-COOH

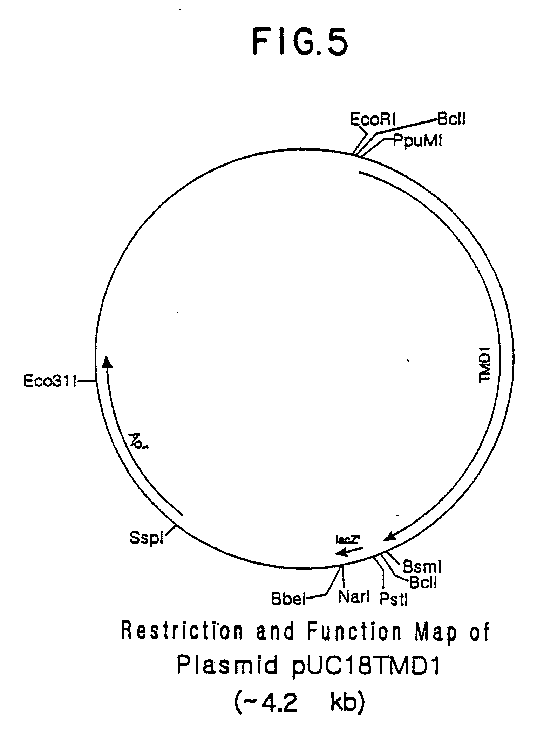

The entire TMD1 sequence, encoding the soluble thrombomodulin derivatives of the invention, is contained on the ~1.5 kb Bcl I fragment of pUC18TMD1. This fragment, except as noted below, is isolated by conventional methods. The restriction site and function map of plasmid pUC18TMD1 is provided in Figure 5 of the drawings.

As was the case for the Ppu MI restriction of plasmid pGHTM3A, subsequent Bcl I digestion, as will be necessary, requires that the internal deoxyadenyl residue in the recognition sequence not be methylated. Therefore, cloning of plasmid pUC18TMD1 requires tranformation into a host strain deficient in adenine methylase activity ( dam ⁻). As noted, the strain used earlier, E. coli K12 GM48 (NRRL B-15725), is suitable for this purpose.

The ~1.5 Bcl I fragment of plasmid pUC18TMD1 then is inserted into the eukaryotic recombinant DNA expression vector, plasmid phd. Plasmid phd is a convenient eukaryotic expression vector because it is a cassette vector which contains the BK virus enhancer sequence ("BK") immediately upstream from the adenovirus-2 late promoter ("AD2LP"). In addition, plasmid phd contains a single Bcl I restriction enzyme recognition sequence positioned for insertion of a desired DNA sequence so that it is expressed from the BK enhancer-AD2LP promoter system. This construction allows for high level transcription and subsequent expression of an inserted gene sequence. Plasmid phd also contains a hygromycin resistance-conferring sequence, as well as a dihydrofolate reductase (dhfr) cistron, both of which can be used as selectable markers in hygromycin sensitive or dhfr negative host cells, respectively. The construction of plasmid phd is described in detail in European Patent Application, EP A 0 245 949 (Appl. No. 87303083.7), published November 19, 1987.

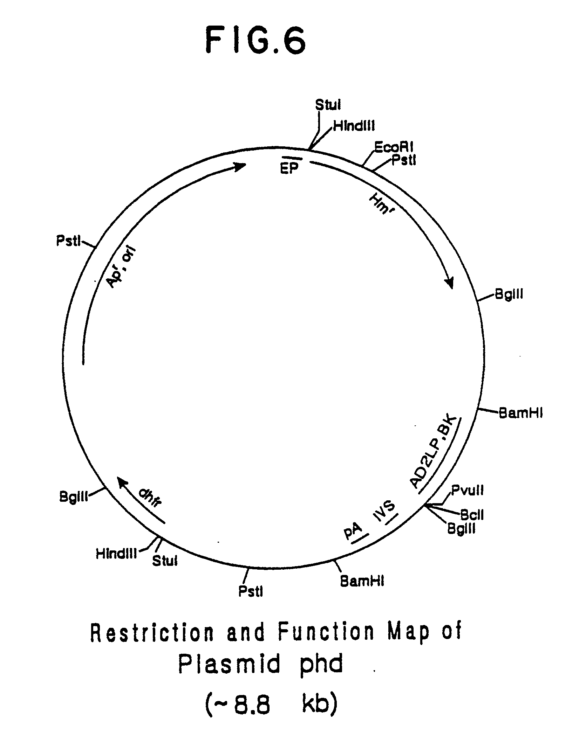

Plasmid phd has been transformed into E. coli K12 GM48, as the preferred source and stock reservoir of the plasmid. The plasmid can be isolated by conventional means from this strain which has been deposited and made a part of the permanent stock culture collection of the NRRL. The strain is available under the accession number NRRL B-18525 deposited July 26, 1989. A restriction site and function map of phd is shown in Figure 6 of the drawings.

To prepare recombinant DNA expression plasmid phdTMD1, the ~1.5 kb Bcl I fragment of pUCTMDI was isolated and ligated to Bcl I-digested plasmid phd. The recircularized DNA is the desired product, plasmid phdTMD1. A restriction site and function map of plasmid phdTMD1 is provided in Figure 7 of the drawings.

As one skilled in the art will appreciate, the specific vectors used and described above are not to be construed as the only way to clone and express the DNA sequences encoding soluble thrombomodulin derivatives of the invention. In particular, one skilled in the art now can construct vectors containing any desired restriction enzyme recognition sequence(s) to prepare both eukaryotic and prokaryotic recombinant DNA vectors which are useful in the invention. In addition, one skilled in the art is fully familiar with the degeneracy of the genetic code. Consequently, the skilled artisan can modify the DNA sequences provided by the invention to provide homologous proteins having the same or improved physiological characteristics compared to those polypeptides specifically provided herein. Also, one skilled in the art can modify the DNA sequences to express an identical protein to those provided, albeit expressed at higher levels. Furthermore, one skilled in the art is familiar with means to prepare synthetically, either partially or in whole, DNA sequences which would be useful in preparing recombinant DNA vectors or soluble TM encoding sequences which are encompassed by the invention. Additionally, recombinant means for modifying the DNA sequences provided may include, for example, site-directed deletion or site-directed mutagenesis. These techniques are well known to those skilled in the art and require no further elaboration here. Consequently, as used herein, "constructed" includes within its scope DNA which is isolated from natural sources, prepared synthetically or semi-synthetically (from natural and synthetic sources) or which are modified by recombinant DNA methods.

Likewise, those skilled in the art will recognize that the polypeptides expressed recombinantly, using the noted constructions, may be synthesized, as well, either in whole or in part by conventional known techniques, e.g. solid phase synthesis. Thus, the present invention should not to be construed as necessarily limited to the specific vector constructions or means for production of the ultimate human thrombomodulin derivatives either defined above or in the examples. These alternate means for preparing the present thrombomodulin derivatives are meant to be encompassed by the present invention.

As one will appreciate, the preferred method for producing the soluble human thrombomodulin derivatives of the invention is by expressing in a host cell a recombinant DNA vector comprising the thrombomodulin derivative encoding sequence of plasmid pUC18TMD1 or plasmid phdTMD1. The following is that thrombomodulin derivative encoding sequence:

```
           10                    30                    50
            .                     .                     .
   5'-ATGCTTGGGGTCCTGGTCCTTGGCGCGCTGGCCCTGGCCGGCCTGGGGTTC


                         70                    90
            .             .         .           .         .
   CCCGCACCCGCAGAGCCGCAGCCGGGTGGCAGCCAGTGCGTCGAGCACGAC


          110                   130                   150
            .         .           .         .           .
   TGCTTCGCGCTCTACCCGGGCCCCGCGACCTTCCTCAATGCCAGTCAGATC


                        170                   190
            .             .         .           .         .
   TGCGACGGACTGCGGGGCCACCTAATGACAGTGCGCTCCTCGGTGGCTGCC


          210                   230                   250
            .         .           .         .           .
   GATGTCATTTCCTTGCTACTGAACGGCGACGGCGGCGTTGGCCGCCGGCGC


                        270                   290
            .             .         .           .         .
   CTCTGGATCGGCCTGCAGCTGCCACCCGGCTGCGGCGACCCCAAGCGCCTC
```

310                        330                        350

GGGCCCCTGCGCGGCTTCCAGTGGGTTACGGGAGACAACAACACCAGCTAT

370                        390                        4

AGCAGGTGGGCACGGCTCGACCTCAATGGGGCTCCCCTCTGCGGCCCGTTG

10                        430                        450

TGCGTCGCTGTCTCCGCTGCTGAGGCCACTGTGCCCAGCGAGCCGATCTGG

470                        490                        510

GAGGAGCAGCAGTGCGAAGTGAAGGCCGATGGCTTCCTCTGCGAGTTCCAC

530                        550

TTCCCAGCCACCTGCAGGCCACTGGCTGTGGAGCCCGGCGCCGCGGCTGCC

570                        590                        610

GCCGTCTCGATCACCTACGGCACCCCGTTCGCGGCCCGCGGAGCGGACTTC

630                        650

CAGGCGCTGCCGGTGGGCAGCTCCGCCGCGGTGGCTCCCCTCGGCTTACAG

670                        690                        710

CTAATGTGCACCGCGCCGCCCGGAGCGGTCCAGGGGCACTGGGCCAGGGAG

730                        750

GCGCCGGGCGCTTGGGACTGCAGCGTGGAGAACGGCGGCTGCGAGCACGCG

770                        790                        810

TGCAATGCGATCCCTGGGGCTCCCCGCTGCCAGTGCCCAGCCGGCGCCGCC

830                        850

CTGCAGGCAGACGGGCGCTCCTGCACCGCATCCGCGACGCAGTCCTGCAAC

```
        870                    890                      910
             .                     .                        .
GACCTCTGCGAGCACTTCTGCGTTCCCAACCCCGACCAGCCGGGCTCCTAC

            930                    950                      97
             .                     .                        .
TCGTGCATGTGCGAGACCGGCTACCGGCTGGCGGCCGACCAACACCGGTGC

       0                   990                      1010
        .                     .                        .
GAGGACGTGGATGACTGCATACTGGAGCCCAGTCCGTGTCCGCAGCGCTGT

           1030                   1050                     1070
             .                     .                        .
GTCAACACACAGGGTGGCTTCGAGTGCCACTGCTACCCTAACTACGACCTG

            1090                   1110
             .                     .                        .
GTGGACGGCGAGTGTGTGGAGCCCGTGGACCCGTGCTTCAGAGCCAACTGC

       1130                   1150                     1170
        .                     .                        .
GAGTACCAGTGCCAGCCCCTGAACCAAACTAGCTACCTCTGCGTCTGCGCC

            1190                   1210
             .                     .                        .
GAGGGCTTCGCGCCCATTCCCCACGAGCCGCACAGGTGCCAGATGTTTTGC

       1230                   1250                     1270
        .                     .                        .
AACCAGACTGCCTGTCCAGCCGACTGCGACCCCAACACCCAGGCTAGCTGT

            1290                   1310
             .                     .                        .
GAGTGCCCTGAAGGCTACATCCTGGACGACGGTTTCATCTGCACGGACATC

   1330                   1350                     1370
        .                     .                        .
GACGAGTGCGAAAACGGCGGCTTCTGCTCCGGGGTGTGCCACAACCTCCCC

            1390                   1410                       14
             .                     .                        .
GGTACCTTCGAGTGCATCTGCGGGCCCGACTCGGCCCTTGCCCGCCACATT

       30                    1450                     1470
        .                     .                        .
GGCACCGACTGTGACTCCGGCAAGGTGGACGGTGGCGACAGCGGCTCTGGC

            1490                   1510                     1530
             .                     .                        .
GAGCCCCCGCCCAGCCCGACGCCCGGCTCCACCTTGACTCCTCCGGCCGTG

                 .
GGGCTCGTGCATTCG-3',
```

wherein

A is deoxyadenyl, G is deoxyguanyl, C is deoxycytidyl, and T is thymidyl.

This latter sequence was previously described. This sequence, or a portion thereof, can be expressed in both prokaryotic and eukaryotic cells if inserted in properly designed expression vectors. For example, E. coli, Bacillus and Streptomyces expression vectors are now well-known in the art. The noted thrombomodulin derivative encoding sequence can be prepared as described above and in the examples and then can be inserted in proper orientation for transcription and expression from a given promoter in the appropriate prokaryotic expression vector, cloned under conditions which allow selection of properly transformed host cells and then expressed, under conditions suitable for expression of the inserted sequence, followed by recovery of the product.

Expression in a prokaryotic host cell, especially in E. coli, of the desired thrombomodulin derivatives of the invention is not limited to the use of a particular promoter because a specific promoter is not crucial to the operablility of the invention. Promoters which can be used may include, for example, the lipoprotein (" lpp ") promoter, the E. coli trp promoter, bacteriophage lambda promoters, or the E. coli lactose (" lac ") promoter. In addition, one or more promoters can be used in tandem, such as for example, the trp and lac promoters. In addition, hybrid promoter/translational activating sequences can be prepared such as the tac promoter, to drive expression of the TMD1 coding sequence provided by the invention. All of these promoters have been previously characterized, are well-known in the art, and can be constructed either synthetically or from known plasmids.

In addition, it may be advantageous to use a thermoinducible runaway replicon such as that disclosed in U.K. Patent Publication 1,557,774 and in Uhlin et al ., Gene , 6 , 91 (1979). At temperatures below 30° C, especially 25° C, the replicon maintains a relatively low copy number of about 10 to 15 copies per cell. When the temperature is raised to 37° C, copy number control is lost and plasmids containing the replicon amplify to approximately 1000-2000 copies per cell.

As noted below, the cloning of a foreign gene, such as the soluble thrombomodulin derivatives of the invention, into vectors comprising a runaway replicon results, upon induction and loss of copy number control, in a greatly increased rate of protein synthesis and the concomitant formation of intracellular proteinaceous granules ("inclusion bodies"). The granules are highly homogenous in their protein composition, with the desired protein product comprising up to and often exceeding 80% by dry weight of the granule. These granules can be easily isolated from cell lysates and are stable to washing in low concentrations of urea or detergents. Washing removes proteins that bind non-specifically to the granule.

The present invention, however, is not limited to the use of a runaway replicon-containing plasmid for the cloning and expression in prokaryotic cells of the soluble thrombomodulin derivatives. Many replicons such as those from pBR322, pBR328, pACYC184, and the like are know in the art and are suitable for the construction of recombinant DNA vectors designed to drive expression of the thrombomodulin derivative-encoding DNA sequences of the invention.

One skilled in the art would also be familiar with the veg promoter from B. subtilis should expression in Bacillus be desired. In addition, the reader is referred to European Patent Publication, EP A 0 116 411 (Published August 22, 1984), as well as U.S. Patent No. 4,559,300, both herein incorporated by reference, for their teaching of vectors and methods for expressing polypeptides in Bacillus . In addition, U.S. Patent No. 4,559,300 discloses expression vectors for use in Streptomyces . Other such expression vectors are now well known as well. See, for example, Horinouchi et al ., Mol. Gen. Genet ., 210 , 468 (1987) and Bibb, M. et al ., Experimental Manipulation of Gene Expression , Chapter 4, "Developments in Streptomyces Cloning", Academic Press (1983).

In addition, the use of particular selectable markers is not crucial to the operation of the invention. A wide variety of selectable markers exist for use in either or both of eukaryotic or prokaryotic host cells.

One skilled in the art will recognize that when expressed in a prokaryotic cell such as, for example, E. coli, Bacillus or Streptomyces, the product normally would not be expected to be glycosylated or even processed further by the intracellular biosynthetic machinery. Consequently, in such cells, the thrombomodulin derivatives of the invention may not be secreted from the cell. Instead, as often seen, particularly in E. coli, the product may be deposited as "inclusion bodies" as described earlier. The purification of the product, in such a case, may require the disruption of the host cell membrane, denaturation of the product, followed possibly by refolding of the protein, and, if desired, cleavage of any desired portion of the signal sequence using appropriate proteases. Alternatively, the DNA sequence for the signal peptide can be modified so as to encode a specific cleavage site recognized by a given enzyme so that site-specific cleavage of the product would be possible. The means for such purification, refolding, and cleavage steps now are well-known to those skilled in the art and will not be elaborated further.

The product, when expressed from a prokaryotic host cell, either in its full length form or a portion thereof, may possess significant thrombomodulin-like activity and could be used for this purpose or for the development of other useful derivatives. In addition, these products could be used as antigens for the production of human thrombomodulin antibodies which could be used in a variety of assays. Many such assays use competitive antibody-binding to measure levels of a protein in a sample. Thus, radioactively (or other) labelling of the prokaryotic cell-produced thrombomodulin derivatives can be used as the "competing molecule" in an assay for thrombomodulin in blood plasma. Such an assay would be important for the diagnosis of patients with coagulation problems. Also, products produced from a prokaryotic cell could be used for structure analysis, folding characteristics, etc. for the development of further improved thrombomodulin derivatives.

The preferred host cell for production of the thrombomodulin derivatives of the invention, however, is a eukaryotic host cell. Usually, mammalian and other eukaryotic host cells, such as some yeasts ( e.g. Saccharomyces, Kluyveromyces, or Pichia), possess the necessary cellular machinery for the recognition and proper processing of the signal peptide portion present on the amino-terminus of the expressed product. Some mammalian host cells also provide post-translational modifications, such as glycosylation, etc., as observed in wild-type thrombomodulin in plasma. A wide variety of vectors exist for the transformation of eukaryotic host cells, and as indicated above, the specific vectors provided herein are in no way intended to limit the scope of the invention.

For example, a wide variety of pSV2-type vectors comprise segments of the SV40 genome and which constitute a defined eukaryotic transcription unit including a promoter ( e.g. , EP), intervening sequences (IVS), and polyadenylation (pA) sites. In the absence of SV40 T-antigen, the pSV2-type vectors transform mammalian and other eukaryotic host cells by integrating into the host cell chromosomal DNA. A variety of plasmid pSV2-type vectors have been constructed ( See Eukaryotic Viral Vectors , edited by Gluzman, Cold Spring Harbor Laboratories, N.Y., 1982) such as plasmids pSV2-gpt, pSV2-neo, pSV2-dhfr, and pSV2-$\beta$-globin, in which the SV40 promoter drives transcription of an inserted gene. The construction and use of these vectors is now well-known to those skilled in the art. Such vectors are available from the American Type Culture Collection, Rockville, MD or from the Northern Regional Research Laboratory (NRRL), Peoria, IL.

Furthermore, other plasmids useful for expressing the derivatives of the invention can use promoters other than the SV40 early promoter. The present invention is in no way limited to the use of any particular promoter exemplified herein. Other promoters, such as the SV40 late promoter or promoters from eukaryotic genes such as, for example, the estrogen-inducible chicken ovalbumin gene, the interferon genes, the glucocorticoid-inducible tyrosine aminotransferase gene, the thymidine kinase gene and the major early and late adenovirus genes, can be readily isolated and modified for use on recombinant DNA expression vectors designed to produce the thrombomodulin derivatives of the invention. Eukaryotic promoters can be used also in tandem to drive expression of the desired final product.

Furthermore, a large number of retroviruses are know which infect a wide range of eukaryotic host cells. Long terminal repeats in retroviral DNA often encode promoter activity and can be used in place of the SV40 early promoter described above, to drive transcription and translation of the soluble thrombomodulin derivative encoding sequences provided by the invention. For example, plasmid pRSVcat (available from the ATCC under the accession number ATCC 37152) comprises portions of the long terminal repeat of Rous Sarcoma Virus (RSV), a virus known to infect chicken and other host cells. The RSV long terminal repeat sequences can be isolated on an ~.76 kb Nde I-Hin dIII restriction fragment of plasmid pRSVcat. This promoter can be isolated and inserted in an appropriate vector such that it is positioned correctly to drive transcription and expression of the thrombomodulin derivative encoding sequence of the invention.

In addition, other eukaryotic or mammalian expression systems are known, similar to those provided by phd, described above. The reader is referred, for example, to published European Patent Application No. 87303083.7, published as EP A 0 245 949 (published November 19, 1987) and corresponding to U.S. Ser. No. 06/849999 (filed April 9, 1986), incorporated herein by reference. The preferred expression vector for the present thrombomodulin derivatives, however, is phdTMD1.

Also, should expression in a yeast cell be desirable, the means for preparing yeast expression systems are now well-described in the art. The reader is referred to the following references if such expression is desired: U.S. Patent No. 4,775,622 (issued October 4, 1988); European Patent No. EP B 0 073 635 (granted April 20, 1988); U.S. Patent No. 4,615,974 (issued October 7, 1986); and European Patent Publication EP A 0 183 070 (published June 4, 1986).

As noted, the eukaryotic expression vectors specifically provided, especially phdTMD1, or any other eukaryotic vector constructed as indicated above and which includes the TMD1 coding sequence, can be transformed into and expressed in a variety of eukaryotic, especially mammalian, host cells. Vectors which contain no selectable marker with which to isolate and identify stable transformants, would be useful for transient assay or for purposes of cotransformation, such as in the procedure outlined in U.S. Pat. No.

4,399,216, issued August 26, 1983, incorporated herein by reference. The vectors of the invention may include sequences which allow for replication in E. coli because it usually more efficient to prepare plasmid DNA in E. coli than in other host organisms, and, if desired, transform the vectors into the desired host.

In any case, expression of the desired thrombomodulin derivative encoding sequences of the invention occurs in those host cells in which the particular promoter associated with the inserted gene functions. The promoters noted above, and in particular, the adenovirus-2 late promoter used in phdTMD1, function in a wide variety of host cells. Although the invention is not limited to any particular host cell, preferred host cells for expression of the thrombomodulin derivatives of the invention are provided in Table 1:

Table 1

Preferred Eukaryotic Host Cells for Plasmid phdTMD1

| Host Cell | Origin | Source | Comments |
|---|---|---|---|
| HepG-2 | Human Liver Hepatoblastoma | *ATCC # HB 8065 | U.S. Patent 4,393,133 describes the use of this cell line |
| Aedes aegypti | Mosquito Larvae | ATCC # CCL 125 | |
| CV-1 | African Green Monkey Kidney | ATCC # CCL 70 | |
| LLC-MK$_2$ original | Rhesus Monkey Kidney | ATCC # CCL 7 | |
| LLC-MK$_2$ derivative | Rhesus Monkey Kidney | ATCC # CCL 7.1 | Grows faster than ATCC # CCL 7 |
| 3T3 | Mouse Embryo Fibroblasts | ATCC # CCL 92 | |
| CHO-K1 | Chinese Hamster Ovary | ATCC # CCL 61 | Proline-requiring Derivatives of CHO-K1, such as the dhfr⁻ derivative DXB11, can be generated from this host |

EP 0 412 841 A1

Table 1 -(cont'd)

Preferred Eukaryotic Host Cells for Plasmid phdTMD1

| Host Cell | Origin | Source | Comments |
|---|---|---|---|
| Antheraea eucalypti | Moth Ovarian Tissue | ATCC # CCL 80 | |
| HeLa | Human Cervix Epitheloid | ATCC # CCL 2 | |
| RPMI8226 | Human Myeloma | ATCC # CCL 155 | IgG lambda-type light chain secreting |
| H4IIEC3 | Rat Hepatoma | ATCC # CRL 1600 | Derivatives, such as 8-azaguanine-resistant FAZA host cells, can be generated from this host |
| C127I | Mouse Fibroblast | ATCC # CRL 1616 | |
| HS-Sultan | Human Plasma Cell Plasmocytoma | ATCC # CRL 1484 | |
| BHK-21 | Syrian Hamster Kidney | ATCC # CCL 10 | |
| COS-1 | Monkey Kidney (SV40 transformed) | ATCC # CRL 1650 | |

*ATCC = American Type Culture Collection, 12301 Parklawn Drive, Rockville, Maryland 20852-1776

Especially preferred host cells include human 293 ("293 cells") cells and Syrian Hamster AV12 ("AV12") cells, available from the American Type Culture Collection, Rockville, MD, under the accession numbers ATCC CRL 1573 and CRL 9595, respectively. The AV12 cells were deposited (November 24, 1987) under the terms of the Budapest Treaty and the 293 cells are listed in the ATCC catalog as presently available to the public.

When expressed in either AV12 or 293 cells, phdTMD1 produces two forms of the soluble thrombomodulin derivatives of the invention. These are designated as the high molecular weight and low molecular weight forms. The particular physical and functional characteristics of each form are described in detail in the examples below. In addition, N-terminal sequencing of the low and high molecular weight derivatives indicates that cleavage in the signal peptide portion of the molecule occurs predominantly at the carboxy terminus of amino acid 18 (proline) leaving the following as the predicted amino acid of the product, assuming no further processing occurs subsequent to expression:

$H_2$N-AlaProAlaGluProGlnProGlyGlySerGlnCysValGluHisAspCys

PheAlaLeuTyrProGlyProAlaThrPheLeuAsnAlaSerGlnIleCys

AspGlyLeuArgGlyHisLeuMetThrValArgSerSerValAlaAlaAsp

ValIleSerLeuLeuLeuAsnGlyAspGlyGlyValGlyArgArgArgLeu

TrpIleGlyLeuGlnLeuProProGlyCysGlyAspProLysArgLeuGly

ProLeuArgGlyPheGlnTrpValThrGlyAspAsnAsnThrSerTyrSer

ArgTrpAlaArgLeuAspLeuAsnGlyAlaProLeuCysGlyProLeuCys

ValAlaValSerAlaAlaGluAlaThrValProSerGluProIleTrpGlu

GluGlnGlnCysGluValLysAlaAspGlyPheLeuCysGluPheHisPhe

ProAlaThrCysArgProLeuAlaValGluProGlyAlaAlaAlaAlaAla

ValSerIleThrTyrGlyThrProPheAlaAlaArgGlyAlaAspPheGln

AlaLeuProValGlySerSerAlaAlaValAlaProLeuGlyLeuGlnLeu

MetCysThrAlaProProGlyAlaValGlnGlyHisTrpAlaArgGluAla

ProGlyAlaTrpAspCysSerValGluAsnGlyGlyCysGluHisAlaCys

AsnAlaIleProGlyAlaProArgCysGlnCysProAlaGlyAlaAlaLeu

GlnAlaAspGlyArgSerCysThrAlaSerAlaThrGlnSerCysAsnAsp

LeuCysGluHisPheCysValProAsnProAspGlnProGlySerTyrSer

CysMetCysGluThrGlyTyrArgLeuAlaAlaAspGlnHisArgCysGlu

AspValAspAspCysIleLeuGluProSerProCysProGlnArgCysVal

AsnThrGlnGlyGlyPheGluCysHisCysTyrProAsnTyrAspLeuVal

AspGlyGluCysValGluProValAspProCysPheArgAlaAsnCysGlu

TyrGlnCysGlnProLeuAsnGlnThrSerTyrLeuCysValCysAlaGlu

GlyPheAlaProIleProHisGluProHisArgCysGlnMetPheCysAsn

GlnThrAlaCysProAlaAspCysAspProAsnThrGlnAlaSerCysGlu

CysProGluGlyTyrIleLeuAspAspGlyPheIleCysThrAspIleAsp

GluCysGluAsnGlyGlyPheCysSerGlyValCysHisAsnLeuProGly

ThrPheGluCysIleCysGlyProAspSerAlaLeuAlaArgHisIleGly

ThrAspCysAspSerGlyLysValAspGlyGlyAspSerGlySerGlyGly

ProProProSerProThrProGlySerThrLeuThrProProAlaValGly

LeuValHisSer-COOH

wherein

ALA is Alanine, ARG is Arginine, ASN is Asparagine, ASP is Aspartic Acid, CYS is Cysteine, GLN is Glutamine, GLU is Glutamic Acid, GLY is Glycine, HIS is Histidine, ILE is Isoleucine, LEU is Leucine, LYS is Lysine, MET is Methionine, PHE is Phenylalanine, PRO is Proline, SER is Serine, THR is Threonine, TRP is Tryptophan, TYR is Tyrosine, and VAL is Valine.

N-terminal sequencing of the low molecular weight form also indicates a minor population of product in which the signal peptide portion of the N-terminus is cleaved at the carboxy terminus of amino acid 16 (glycine). The predicted amino acid sequence of this product, assuming no further processing occurs subsequent to expression, is the following:

```
PhePro-AlaProAlaGluProGlnProGlyGlySerGlnCysValGluHisAspCys
        PheAlaLeuTyrProGlyProAlaThrPheLeuAsnAlaSerGlnIleCys
        AspGlyLeuArgGlyHisLeuMetThrValArgSerSerValAlaAlaAsp
        ValIleSerLeuLeuLeuAsnGlyAspGlyGlyValGlyArgArgArgLeu
        TrpIleGlyLeuGlnLeuProProGlyCysGlyAspProLysArgLeuGly
        ProLeuArgGlyPheGlnTrpValThrGlyAspAsnAsnThrSerTyrSer
        ArgTrpAlaArgLeuAspLeuAsnGlyAlaProLeuCysGlyProLeuCys
        ValAlaValSerAlaAlaGluAlaThrValProSerGluProIleTrpGlu
        GluGlnGlnCysGluValLysAlaAspGlyPheLeuCysGluPheHisPhe
        ProAlaThrCysArgProLeuAlaValGluProGlyAlaAlaAlaAlaAla


        ValSerIleThrTyrGlyThrProPheAlaAlaArgGlyAlaAspPheGln
        AlaLeuProValGlySerSerAlaAlaValAlaProLeuGlyLeuGlnLeu
        MetCysThrAlaProProGlyAlaValGlnGlyHisTrpAlaArgGluAla
        ProGlyAlaTrpAspCysSerValGluAsnGlyGlyCysGluHisAlaCys
        AsnAlaIleProGlyAlaProArgCysGlnCysProAlaGlyAlaAlaLeu
        GlnAlaAspGlyArgSerCysThrAlaSerAlaThrGlnSerCysAsnAsp
        LeuCysGluHisPheCysValProAsnProAspGlnProGlySerTyrSer
        CysMetCysGluThrGlyTyrArgLeuAlaAlaAspGlnHisArgCysGlu
        AspValAspAspCysIleLeuGluProSerProCysProGlnArgCysVal
        AsnThrGlnGlyGlyPheGluCysHisCysTyrProAsnTyrAspLeuVal
        AspGlyGluCysValGluProValAspProCysPheArgAlaAsnCysGlu
        TyrGlnCysGlnProLeuAsnGlnThrSerTyrLeuCysValCysAlaGlu
        GlyPheAlaProIleProHisGluProHisArgCysGlnMetPheCysAsn
        GlnThrAlaCysProAlaAspCysAspProAsnThrGlnAlaSerCysGlu
        CysProGluGlyTyrIleLeuAspAspGlyPheIleCysThrAspIleAsp
        GluCysGluAsnGlyGlyPheCysSerGlyValCysHisAsnLeuProGly
        ThrPheGluCysIleCysGlyProAspSerAlaLeuAlaArgHisIleGly
        ThrAspCysAspSerGlyLysValAspGlyGlyAspSerGlySerGlyGly
        ProProProSerProThrProGlySerThrLeuThrProProAlaValGly
        LeuValHisSer-COOH
```

wherein
ALA is Alanine, ARG is Arginine, ASN is Asparagine, ASP is Aspartic Acid, CYS is Cysteine, GLN is Glutamine, GLU is Glutamic Acid, GLY is Glycine, HIS is Histidine, ILE is Isoleucine, LEU is Leucine, LYS is Lysine, MET is Methionine, PHE is Phenylalanine, PRO is Proline, SER is Serine, THR is Threonine, TRP is Tryptophan, TYR is Tyrosine, and VAL is Valine.

All of these forms are meant to be encompassed by the invention. As noted in the examples provided, the present derivatives offer useful properties previously unavailable to the skilled clinician. The thrombomodulin derivatives of the invention are easily prepared and purified because they are soluble and secreted from the eukaryotic host cell. The unexpected solubility in detergent-free buffers provides a distinct advantage over wild-type thrombomodulin. In addition, the products provide significant physiological activity which could not have been predicted. In particular, especially for the high molecular species, optimal calcium concentrations approximate those present under normal physiological conditions and observed for the wild-type thrombomodulin. Further, the products, most strikingly the high molecular weight form, inhibit the clotting cascade through two distinct and different mechanisms. First, the derivatives in consort with thrombin, activate the Protein C anticoagulant pathway, thereby reducing the activity of the major cofactors in the clotting system, Factors Va and VIIIa. Secondly and unexpectedly, the derivatives, particularly the high molecular weight form, substantially inhibit thrombin coagulant activity. Also, unexpectedly, the derivatives, particularly the high molecular weight derivative, inhibit the normal capability of thrombin to activate platelets. This is unexpected since the literature suggests that full-length human thrombomodulin isolated from natural sources has little, if any, inhibitory effect on thrombin coagulant and platelet activation activities. ( See , e.g. Maruyama, I., et al ., J. Clin. Invest . 75 , 987 (1985); Salem, H.H., et al ., J. Biol. Chem . 259 , 12246 (1984)).

The soluble, secretable thrombomodulin derivatives of the invention will have substantial value in the prevention and treatment of thrombotic disorders. These disorders include a wide variety of acquired disease states involving intravascular coagulation, including deep vein thrombosis, pulmonary embolism, peripheral arterial thrombosis, emboli originating from the heart or peripheral arteries, acute myocardial infarction, thrombotic strokes, and disseminated intravascular coagulation. Disseminated intravascular coagulation occurs as a complication to numerous disease states including major trauma, major surgery, heat stroke, septicemia, acute and chronic liver disease, malignancies including solid tumors, leukemias and lymphomas, a wide variety of bacterial, fungal, parasitic and viral infections, obstetrical complications, hemolytic processes, cardiogenic shock, circulatory collapse from any cause, severe progressive strokes, snake bites, collagen vascular disorders, purpura fulminans, acute pancreatitis, allergic vasculitis, polycythemia vera, thrombocythemia, and ulcerative colitis among others.

Although not yet discovered, congenital deficiencies in the expression of the thrombomodulin may be demonstrated in patients with thromboembolic problems. Acute episodes in such patients will be amenable to treatment with the thrombomodulin derivatives provided.

Experimental and clinical data suggest that conventional anticoagulants, particularly warfarin, are useful in the treatment of invasive cancers and act to prevent or reduce the distal metastatic lesions of these malignancies. The present thrombomodulin derivatives represent an attractive alternative to conventional anticoagulants in these clinical situations for the reasons detailed below.

Deep vein thrombosis and pulmonary embolism can be treated with conventional anticoagulants, but a far more attractive clinical approach is to prevent the occurrence of thromboembolic complications in identified high-risk patients, such as, for example, patients undergoing surgery, patients who are chronically bedridden and patients with congestive heart failure. Over 50% of surgical patients age 50 and over and 20% of all patients in general suffer from deep vein thrombosis following surgery. About 20% of all postsurgical cases of deep vein thrombosis are complicated by one or more pulmonary emboli. Presently, low doses of heparin (e.g. 5,000 units every 8 hours) are administered both, pre-and post-surgery, to prevent deep vein thrombosis. Low-dose heparin occasionally causes heavy bleeding during and after surgery. Among the thrombomodulin derivatives, the low molecular weight derivative causes less thrombin inactivation and less inhibition of platelet activation than the high molecular weight form thrombomodulin derivatives. The low molecular weight form, in particular, is more selective than heparin and less likely to cause bleeding complications, being active only when and where thrombin is generated and fibrin thrombi are formed. Thrombomodulin derivatives would be more effective and less likely to cause bleeding complications than heparin when used prophylactically for the prevention of deep vein thrombosis. The dose of recombinant-produced thrombomodulin derivatives for prevention of deep vein thrombosis is in the range from 0.5 to 100 mg/day. Preferably, administration of the thrombomodulin derivative should begin 6 hours prior to surgery and continue until the patient becomes mobile. In established, objectively-documented, deep vein thrombosis and/or pulmonary embolism, the dose of thrombomodulin derivative (preferably the high molecular weight form) ranges from 1-30 mg as a loading dose, followed by a continuous infusion in amounts ranging from 3-300 mg/day. Similar dosage schedules are applicable for the treatment of peripheral arterial thrombi. Because of the lower likelihood of bleeding complications from infusions of thrombomodulin derivative, these proteins can replace heparin, intra-and post-surgically, in conjunction with thrombectomies or embolectomies, surgical procedures which are often necessary to save ischemic limbs from amputation in the setting of an acute arterial obstruction.

Arterial emboli originating from the heart are frequent complications in heart diseases involving heart valves, in patients with artificial heart valves, in acute myocardial infarction, and in certain types of heart arrhythmias. The treatment of these problems with conventional oral anticoagulants is not always entirely effective, and, as always, when oral anticoagulants are used, the risk of bleeding complications is substantial. The present thrombomodulin derivatives, administered long-term in doses comparable to those for the treatment of established deep vein thrombosis-pulmonary embolism, through continuous infusion using, for example, a portable pump system, has substantial utility in the prevention of cardiogenic emboli.

Similarly, emboli originating from thrombi in peripheral arteries, most notably the carotid arteries, are not treated or prevented satisfactorily with currently used regimens, which include drugs capable of suppressing platelet function, oral anticoagulants, or combinations thereof. As in the case of cardiogenic emboli, the thrombomodulin derivatives, administered long-term in the same manner as outlined for cardiogenic emboli, has major potential in the prevention of emboli originating from carotid artery thrombi and resulting in embolic strokes.

The thrombomodulin derivatives (most probably the low molecular weight form) is also useful in the treatment of thrombotic strokes. Today, strokes are not usually treated with conventional anticoagulants. Treatment of strokes with either heparin or oral anti-coagulants, although occasionally beneficial, carries a high risk for bleeding into the infarcted brain area, thereby aggravating the neurological deficit accompanying the stroke. Because of their low potential for causing bleeding complications and their selectivity, thrombomodulin variants can be given to stroke victims and are beneficial in preventing the local extension of the occluding arterial thrombus, thereby reducing the neurological deficit resulting from the stroke. The amount of thrombomodulin variants administered will vary with each patient depending on the nature and severity of the stroke.

Further, the present thrombomodulin derivatives (most preferably the high molecular weight variant) will be useful in the treatment of acute myocardial infarction because there is evidence that the major mechanism of action of thrombomodulin and derivatives provided, that is, the activation of the protein C anticoagulant pathway, constitutes a highly effective means of achieving antithrombotic effects on the arterial side of the circulation. In current trials with thrombolytic agents in acute myocardial infarction and from animal experiments, it would appear that heparin, as adjunct therapy, is relatively ineffective, as an antithrombotic agent on the arterial side of the circulation. The present thrombomodulin derivatives can be given with thrombolytic agents during the acute phases of the myocardial infarction. This will result in a shorter time to reperfusion than if the thrombolytic agent is given by itself or in combination with heparin. After the occluding coronary thrombus is dissolved, the thrombomodulin derivatives can be given for several additional days to prevent coronary reocclusion. In acute myocardial infarction, the patient is given the loading dose of 3-30 mg of thrombomodulin variants at the time thrombolytic treatment is initiated followed by a continuous infusion of thrombomodulin variants in amounts ranging from 1-100 mg/day.

Also, the present thrombomodulin derivatives are useful in the treatment of disseminated intravascular coagulation ("DIC"). There is experimental evidence that inflammatory mediators such as IL-1, TNF, and LPS endotoxin sharply downregulate the expression of thrombomodulin on endothelial cells leading in turn to defective activation of the protein C anticoagulant pathways. Heparin and the oral anticoagulants have been given to patients with disseminated intravascular coagulation in extensive clinical trials, but the results of these trials have been disappointing. Characteristically, patients with disseminated intravascular coagulation have widespread thrombi involving the microcirculation with concomitant and often severe bleeding problems, which result from "consumption" of essential clotting factors, which have been first activated and then inactivated during the formation of widespread microcirculatory fibrin thrombi. In disseminated intravascular coagulation, thrombomodulin has a distinct advantage over conventional anticoagulants. Because of their selectivity, the thromomodulin derivatives of the invention will not aggravate the bleeding problems associated with disseminated intravascular coagulation, as do heparin and the oral anticoagulants, but instead will retard or inhibit the formation of additional microvascular fibrin deposits. Infused thrombomodulin will block the substantial quantities of thrombin present in the circulation. The high molecular variant will block thrombin's procoagulant and platelet activating activity and speed up the conversion of protein C to activated protein C. The doses required are comparable to those used in established deep vein thrombosis-pulmonary embolism. Treatment will continue until definitive measures can be taken to treat the underlying causes of the DIC syndrome.

Evidence exists that conventional anticoagulant drugs, particularly warfarin, are useful in the treatment of invasive malignant tumors. Many tumor cells produce substances which trigger the activation of the coagulation system resulting in local fibrin deposits. These fibrin deposits function as "nests" in which cancer cells can divide to form metastatic lesions. In one clinical study, it was shown that patients receiving warfarin in addition to cancer chemotherapy for treatment of small cell carcinoma of the lung live longer and have less extensive metastatic lesions than patients receiving chemotherapy alone. However, the cancer chemotherapy utilized in this study was less intensive than that considered optimal in clinical oncology today. The more intensive forms of cancer chemotherapy almost always produce a sharp decline in the platelet count, and thrombocytopenia combined with warfarin therapy puts the patient in an unacceptably high risk for serious bleeding complications. Thrombomodulin variants, being more selective than conventional anticoagulants and having a higher therapeutic index than either heparin or the oral anticoagulant, can be given relatively safely to the thrombocytopenic patient, thus making possible the treatment of patients with invasive cancers with effective intensive chemotherapy in combination with thrombomodulin variants. Treatment of invasive cancers with thrombomodulin variants will follow a dosage regimen comparable to that used in deep vein thrombosis-pulmonary embolism.

In view of this discussion, the invention provides in another aspect, a method for the treatment or prevention of thrombotic events in a patient in need thereof, which comprises administering to said patient a therapeutically-effective amount of a thrombomodulin derivative provided herein.

The compounds of the present invention can be formulated according to known methods to prepare pharmaceutically-useful compositions. In particular, a thrombomodulin derivative of the present invention is combined in admixture with a pharmaceutically acceptable carrier, diluent or excipient. Suitable carriers, diluents or excipients and their formulation, inclusive of other human proteins, e.g., human serum albumin, are described, for example, in Remington's Pharmaceutical Sciences 16th ed., 1980, Mack Publishing Co., edited by Osol et al., which is hereby incorporated by reference. Such compositions will contain a therapeutically- effective amount of the thrombomodulin derivative, together with a suitable amount of carrier, excipient, or diluent, vehicle in order to prepare pharmaceutically acceptable compositions suitable for effective administration to the patient. The thrombomodulin compositions can be administered parenterally, or by other methods well-known to those skilled in the art, so as to ensure their delivery to the bloodstream in an effective form.

The following non-limiting examples are provided to further illustrate and describe the invention. The invention is not to be construed as limited in scope by reason of the descriptions given in any of the examples. Sources of reagents are provided merely for convenience and are not meant to be limiting on the invention.

## Example 1

## A. Isolation and Identification of Human Thrombomodulin-Containing DNA Sequences

The thrombomodulin gene has been localized to chromosome 20 in the human genome (Wen, D. et al . Biochemistry , 26 , 4350 (1987). Further, this gene is reported to contain no introns (Jackman, R. et al . Proc. Natl. Acad. Sci. (USA) , 84 , 6425 (1987). Based upon this information, a human chromosome 20 library carried in lambda phage Charon 21A was purchased from American Type Culture Collection (ATCC #57712, ID code LL20NS01, Rockville, MD 20852). E. coli strain C600 cells (commercially available from Stratagene, LaJolla, CA 92037) were grown to an optical density of 0.6-0.8 at 600 nm in TY broth (10 g Bacto-tryptone, 5 g Bacto-yeast extract, 10 g NaCl/liter, pH 7.4); 600 $\mu$l of cells were infected with 50,000 phage. A total of 600,000 phage (three chromosome equivalents) were plated on twelve 150 mm dishes, each containing 75 ml TY agar (TY broth plus 15 g/liter Bacto-agar). Plaques were well-formed after 6 hours at 37° C. The phage were transferred to Colony/Plaque Screen filters (New England Nuclear, Boston, MA) and prepared for hybridization using the supplier's recommendations.

Several different 50 base oligomers were designed based upon the published bovine thrombomodulin DNA sequence (Jackman, R. et al ., Proc. Natl. Acad. Sci. (USA) , 83 , 8834 (1986)) and these probes were radiolabeled with T4 kinase using techniques familiar to those skilled in the art. The filters first were hybridized with a mix of three of the probes using known protocols and positive signals were identified by exposure to film for 6 hours at -70° C. The original probe was removed from these filters by heating in 0.1X SSPE, 0.1% SDS for 10 minutes at 75° C. SSPE (20X) is prepared by dissolving 174 g of NaCl, 27.6 g of $NaH_2PO_4 \cdot H_2O$ and 7.4 g of EDTA in 800 ml of $H_2O$ after which the pH is adjusted to 7.4 with NaOH (~6.5 ml of a 10 N solution) and the volume adjusted to 1 liter. The filters then were exposed to film for 24 hours at -70° C to prove that all of the original probe mixture had been removed. These filters then were hybridized again using a different probe mix. The second films were compared to the first set and only plaques that hybridized with both sets of probes were chosen for a second round of screening. During the second round of screening one plaque was found to hybridize to a probe homologous to the 5'-end of the DNA sequence and also to a separate probe homologous to the 3'-end of the gene. This clone contained the entire coding sequence. This clone, GHTM3A, was purified and used as the basis for all further studies.

### B. Isolation of TM cDNA Insert from GHTM3A

Large amounts of purified phage DNA were isolated using the plate lysate technique (modified from the procedure published by R. W. Davis, D. Botstein and J. R. Roth, A Manual for Genetic Engineering , Advanced Bacterial Genetics , Cold Spring Harbor, N.Y., 1980, page 106). Ten 150 mm dishes each were plated with E. coli C600 infected with about $10^6$ plaque-purified phage. Confluent lysis was achieved after seven hours at 37° C. Each plate was flooded with 10 ml of lambda dilution buffer (10 mM Tris (pH 7.5), 10 mM $MgSO_4$ ) and gently rocked at 4° C overnight. The buffer was removed carefully the next morning and treated with a few drops of chloroform to lyse any remaining cells. Phage particles were removed from the supernatant by centrifugation at 20,000 rpm for 3 hours at 18° C and the pellet was resuspended in 1 ml of lambda dilution buffer.

The phage particles were purified further using cesium chloride gradients: the 1 ml phage sample was layered over a step gradient of 1 ml 5 M CsCl, 10 mM $MgSO_4$ , 0.1 mM disodium EDTA, 10 mM Tris (pH = 8.0); and 3 ml of 3 M CsCl, 10 mM $MgSO_4$, 0.1 mM disodium EDTA, 10 mM Tris (pH = 8.0). After 60 min at 30,000 rpm (18° C), a faint blue band, corresponding to the phage particles, could be seen at the interface.

Using a syringe and needle, 0.5 ml of the solution at the interface was removed. Phage DNA was released from the protein coat with the addition of an equal volume of deionized formamide. After 30 min at room temperature the solution was diluted with 0.5 ml of water and the DNA precipitated with two volumes of room temperature ethanol. The DNA was collected by centrifugation for 10 minutes at 10,000 rpm; the pellet was rinsed with 5 ml of 68% ethanol at -20° C, dried and resuspended in 500 $\mu$1 of TE (10 mM Tris (pH = 8.0), 1 mM EDTA). A total of 200-1000 $\mu$g of phage DNA was recovered.

The human genomic DNA had been inserted into the Hin dIII site of the Charon 21A vector and so could be released by digesting the recombinant clone with Hin dIII. Fifty $\mu$g of purified phage DNA were digested in a volume of 200 ul of core buffer with 50 units of Hin dIII (buffer and enzyme supplied by Bethesda Research Laboratories, Gaithersburg, Maryland 20877) for 90 minutes at 37° C. The samples were precipitated with ethanol and then separated by electrophoresis on a 1.0% agarose gel along with lambda DNA cut with Hin dIII (Bethesda Research Laboratories) as a size marker. The DNA was visualized using ethidium bromide and the size of each band was calculated based upon its migration in the gel. The insert size for GHTM3A was estimated to be ~6.4 kb.

### C. Isolation of the ~6.4 kb HindIII fragment of phage GHTM3A

50 $\mu$g of the phage GHTM3A DNA isolated in Example 1B is mixed with 5 $\mu$l (~50 units) of restriction enzyme Hin dIII, 10 $\mu$1 of 10X Hin dIII reaction buffer (500 mM NaCl, 500 mM Tris-HCl (pH 8.0), 100 mM $MgCl_2$) and 85 $\mu$l of $H_2O$ and is incubated at 37° C for about 2 hours. The Hin dIII digested phage GHTM3A DNA is then electrophoresed on a 0.6% agarose gel until the ~6.4 kb Hin dIII fragment is separated from the other digestion products. The DNA bands are visualised by first staining the gel with a dilute solution of ethidium bromide and then viewing the gel with ultraviolet light.

The region of the gel containing the ~6.4 kb Hin dIII fragment is cut from the gel, wrapped in a piece of plastic film and frozen at -70° C for about 10 minutes. The frozen gel slice then is gently squeezed at room temperature and the gel slurry poured into a 1.5 ml centrifuge tube. An equal volume of phenol is added and the mixture vigorously vortexed and frozen again at -70° C. The mixture is thawed at room temperature and the aqueous phase removed after centrifugation at 13,000xg for 10 minutes. The aqueous phase is extracted once more with phenol and then twice with $CHCl_3$. After addition of 1/10 volume of 3M Na Acetate, the DNA is precipitated by addition of 2 volumes of ethanol, incubation at -20° C for 20 minutes and followed by centrifugation.

Approximately 10 $\mu$g of the ~6.4 kb Hin dIII restriction fragment were obtained by this procedure. The purified fragment is resuspended in 10 $\mu$l of TE buffer and stored at -20° C.

D. Isolation of HindIII-digested plasmid pUC19

Approximately 10 μg of plasmid pUC19 (available from Boehringer-Mannheim, Indianapolis, IN 46250 or "BRL" (Bethesda Research Laboratories, Inc., Gaithersburg, MD 20760)) were digested with Hin dIII as described in Example 1C. Hin dIII digested pUC19 DNA was isolated by extraction of the digest mixture with phenol, then with $CHCl_3$, followed by ethanol precipitation as described in Example IC. The approximately 10 μg of Hin dIII-digested DNA was resuspended in 10 μl of TE buffer and stored at -20° C.

E. Ligation of Fragments to Construct Plasmid pGHTM3A

One μl of the Hin dIII-digested pUC19 plasmid DNA, as prepared in Example 1D, and 1 μl of the ~6.4 kb Hin dIII fragment isolated in Example 1C were mixed together and then incubated with 1 μl of 10X ligase buffer (300mM Tris-HCl, pH 7.8; 10mM $MgCl_2$; 100mM dithiothreitol and 1 mg/ml BSA), 1 μl of 10mM ATP, 1 μl T4 DNA ligase (~10 units) and 6 μl of $H_2O$ at 16° C overnight. The ligated DNA constituted the desired plasmid, plasmid pGHTM3A.

F. Transformation of E. coli with pGHTM3A

A 50 ml culture of E. coli K12 DH5αF' (commercially available from Bethesda Research Laboratories, "BRL", Gaithersburg, MD 20877) in TY-broth is grown to an O.D. 590 nm of ~0.2. The culture is chilled on ice for ten minutes, and the cells are collected by centrifugation. The cell pellet is resuspended in 25 ml of cold 100 mM $CaCl_2$ and incubated on ice for 25 minutes. The cells are once again pelleted by centrifugation and the pellet is resuspended in 2.5 ml of cold 100 mM $CaCl_2$ and incubated on ice overnight.

Two hundred μl of this cell suspension are mixed with the ligated DNA prepared in Example 1E and incubated on ice for 20 minutes. The mixture then is incubated at 42° C for 2 minutes, followed by a 10 minute incubation at room temperature. Three ml of TY-broth are added to the cell mixture, and then the cells are incubated in an air-shaker at 37° C for two hours.

Aliquots of the cell mixture are plated on TY-agar (TY-broth with 15 g/l agar) plates containing 100 μg/ml ampicillin, and the plates then are incubated at 37° C. E. coli K12 DH5αF'/pGHTM3A transformants are verified by restriction enzyme analysis of their plasmid DNA.

EXAMPLE 2

Culture of E. coli K12 DH5αF'/pGHTM3A and Isolation of Plasmid pGHTM3A

A. Culture of E. coli K12 DH5αF'/pGHTM3A

One liter of TY-broth (10 g Bacto-tryptone, 5 g Bacto-yeast extract, 10 g NaCl, pH 7.5) containing 50 μg/ml ampicillin was incubated with a culture of E. coli DH5αF'/pGHTM3A and incubated in an air-shaker at 37° C until the optical density (O.D.) at 590 nm was ~1 absorbance unit, at which time 150 mg of chloramphenicol were added to the culture. The incubation was continued for about 16 hours; the chloramphenicol addition inhibits protein synthesis, and thus inhibits further cell division, but allows plasmid replication to continue.

B. Isolation of Plasmid pGHTM3A

The culture prepared in Example 2A was centrifuged in a Sorvall GSA rotor (Dupont Co., Instrument Products, Biomedical Division, Newtown, CN 06470) at 600 rpm for 5 minutes at 4° C. The resulting supernatant was discarded, and the cell pellet was washed in ~50 ml of TES buffer (10 mM Tris-HCl, pH = 7.5; 10 mM NaCl; and 1 mM EDTA) and then repelleted. After discarding the supernatant again, the cell pellet was frozen in a dry ice-ethanol bath and then thawed. The thawed cell pellet was resuspended in 10 ml of a 25% sucrose/50 mM EDTA solution. After adding and mixing 1 ml of a 5 mg/ml lysozyme solution; 3 ml of 0.25M EDTA, pH = 8.0; and 100 μl of 10 mg/ml RNAse A, the solution was incubated on ice for 15 minutes. Three ml of lysing solution (prepared by mixing 3 ml 10% Triton-X 100; 75 ml 0.25M EDTA, pH = 8.0; 15 ml of 1M Tris-HCl, pH = 8.0; and 7 ml of water) were added to the lysozyme-treated cells, mixed, and the resulting solution incubated on ice for another 15 minutes. The lysed cells were frozen in a dry ice-ethanol bath and then thawed.

The cellular debris was removed from the solution by centrifugation at 25,000 rpm for 40 minutes in an SW27 rotor (Beckman 7360 N. Lincoln Ave., Lincolnwood, IL 60646). After adding 30.44 g of CsCl and ~1 ml of a 5 mg/ml ethidium bromide solution, the solution volume was adjusted to 40 ml and decanted into a Vti50 ultracentrifuge tube (Beckman). After sealing the tube, the solution was centrifuged in a Vti50 rotor at 42,000 rpm for ~16 hours. The resulting plasmid band, visualized with ultraviolet light, was isolated and then placed in a Ti75 tube and rotor (Beckman) and centrifuged at 55,000 rpm for 16 hours. Any necessary volume adjustments were made using TES containing 0.761 g/ml CsCl. The plasmid band was again isolated, the ethidium bromide extracted with salt-saturated isopropanol, and diluted 1:3 with TES buffer. Two volumes of ethanol then were added to the solution, followed by incubation overnight at -20°C. The plasmid DNA was pelleted by centrifuging the solution in an SS34 rotor (Sorvall) for 15 minutes at 10,000 rpm.

The ~1 mg of plasmid pGHTM3A DNA obtained by this procedure was suspended in 1 ml of TE buffer (10 mM Tris-HCl, pH = 8.0 and 1 mM EDTA) and stored at -20°C.

EXAMPLE 3

Construction of plasmid pUC18TM

A. Isolation of the ~1.9 kb PpuMI restriction Fragment of Plasmid pGHTM3A

The Ppu MI sites used for isolating the coding sequence of human thrombomodulin both overlap the dcm methylase recognition sequence, 5'-CCTGG-3'. Methylation of the internal cytosine residue of this recognition sequence results in blockage of DNA cleavage. For this reason, it was necessary to isolate plasmid pGHTM3A from E. coli K12 GM48. E. coli K12 GM48, a strain deficient in the cytosine methylase, originally was deposited November 23, 1983, and now is available, under the terms of the Budapest Teaty, from the permanent stock culture collection of the Northern Regional Research Laboratory ("NRRL"), Peoria, Illinois 61604, under the accession number NRRL B-15725.

E. coli K12 GM48 was prepared for transformation and then transformed with plasmid pGHTM3A in substantial accordance with the procedure of Example 1F. Plasmid pGHTM3A was isolated from the E. coli K12 GM48/pGHTM3A transformants in substantial accordance with the teaching of Example 2B.

Twenty μg of the plasmid pGHTM3A DNA isolated from the E. coli K12 GM48/pGHTM3A transformants were dissolved in 10 μl 10X Ppu MI reaction buffer (500 mM NaCl; 100 mM Tris-HCl, pH 7.4; 100 mM MgCl₂; 100 mM 2-mercaptoethanol; 1 mg/ml bovine serum albumin), 10 μl (~15 units) restriction enzyme Ppu MI, and 85 μl of H₂O and the resulting reaction was incubated at room temperature overnight. The Ppu MI-digested plasmid pGHTM3A DNA was loaded onto a 1% agarose gel, and the desired ~1.9 kb Ppu MI restriction fragment was isolated and purified in substantial accordance with the teaching of Example 1. The ~2 μg of fragment obtained were dissolved in 10 μl of TE buffer and stored at -20°C.

B. Construction of the EcoRI-PstI Linker

The DNA fragments used in the construction of the linker were synthesized either by using a Systec 1450A DNA Synthesizer (Systec Inc., 3816 Chandler Drive, Minneapolis, MN) or an ABS 380A DNA Synthesizer (Applied Biosystems, Inc., 850 Lincoln Centre Drive, Foster City, CA 94404). Many DNA synthesizing instruments are known in the art and can be used to make the desired fragments. In addition, the fragments can also be conventionally prepared in substantial accordance with the procedures of Itakura et al ., 1977, Science , 198:1056 and Crea et al ., 1978, Proc. Nat. Acad. Sci. (USA) , 75:5765.

Five hundred picomoles of each single strand of the linker were kinased in 20 μl of reaction buffer containing: 15 units (~0.5 μl) T4 polynucleotide kinase, 2 μl 10X ligase buffer (300 mM Tris-HCl, pH = 7.8; 100 mM MgCl₂; 100 mM dithiothreitol; and 1 mg/ml BSA), 10 μl 500 μM ATP, and 7.5 μl H₂O. The kinase reaction was incubated at 37°C for 30 minutes, and the reaction was terminated by incubation at 100°C for 10 minutes. In order to ensure complete kination, the reaction was chilled on ice, 2 μl of 0.2M dithothreitol, 2.5 μl of 5 mM ATP, and 15 units of T4 polynucleotide kinase were added, mixed, and the reaction mix incubated another 30 minutes at 37°C. The reaction was stopped by another 10 minute incubation at 100°C and then chilled on ice.

Although kinased separately, the two single strands of the DNA linker were mixed together after the kinase reaction. In order to anneal the strands, the kinase reaction mixture was incubated at 100°C for 10 minutes in a water bath containing ~150 ml of water. After this incubation, the water bath was shut off and allowed to cool to room temperature, a process taking about 3 hours. The water bath, still containing the tube of kinased DNA, then was placed in a 4°C refrigerator overnight. This process annealed the single strands. The synthesized linker had the following structure:

```
                 BclI                 BsmI                   PstI
         EcoRI          PpuMI                          BclI
     5'-AATTCTGATCATGCTTGGGGTCCTTGCATTCGTAATTAATTAATGATCACTGCA-3'
         |||||||||||||||||||||||||||||||||||||||||||||||||||
     3'-GACTAGTACGAACCCCAGGAACGTAAGCATTAATTAATTACTAGTG-5'
```

The linker was stored at -20°C until needed.


## C. Isolation of EcoRI-PstI-Digested Plasmid pUC18

Approximately 10 μg of plasmid pUC18 DNA (Boehringer-Mannheim, Indianapolis, IN 46250; or BRL, Gaithersburg, MD 20760) were dissolved in 10 μl 10X Eco RI reaction buffer (500 mM NaCl, 1M Tris-HCl, pH 7.5, 50 mM MgCl₂, 1 mg/ml bovine serum albumin), 5 μl (~50 units) restriction enzyme Eco RI, and 85 μl H₂O, and the reaction was placed at 37°C for 2 hours. The reaction mixture was then made 0.25M in NaOAc, and after adding two volumes of ethanol and chilling in a dry ice-ethanol bath, the DNA was pelleted by centrifugation.

The DNA pellet was dissolved in 10 μl 10X Pst I reaction buffer (1M NaCl, 100 mM Tris-HCl, pH 7.5, 100 mM MgCl₂, 1 mg/ml bovine serum albumin), 5 μl (~50 units) restriction enzyme Pst I, and 85 μl H₂O, and the reaction was placed at 37°C for two hours.

After digestion with Pst I, the reaction mixture was extracted once with phenol, once with CHCl₃ and the digested DNA precipitated and pelleted as described above. The DNA pellet which constituted Eco RI-Pst I-digested plasmid pUC18 was resuspended in 10 μl of TE buffer and stored at -20°C until use.


## D. Ligation of Fragments to Construct Plasmid pUC18TMlinker

One μl of the Eco RI-Pst I linker prepared in Example 3B and 1 μl of the Eco RI-Pst I-digested plasmid pUC18, prepared in 3C, were mixed together and then incubated with 1 μl 10X ligase buffers 1 μl 10 mM ATP, 1 μl T4 DNA ligase (~10 units), and 7 μl of H₂O at 16°C overnight. The ligated DNA constituted the desired plasmid, plasmid pUC18TMlinker. A restriction site and function map of the plasmid is presented in Figure 3 of the accompanying drawings.


## E. Isolation of Plasmid pUC18TMLinker

The ligated DNA constituting plasmid pUC18TMlinker prepared in Example 3D was used to transform E. coli K12 DH5αF' cells substantially as described in Example IF. E. coli K12 DH5αF'/pUC18TMlinker transformants were verified by restriction enzyme analysis of their plasmid DNA. A single E. coli K12 DH5αF'/pUCTMlinker transformant was cultured as described in Example 2A and plasmid pUC18TMlinker DNA was isolated from this culture as described in Example 2B.


### F. Isolation of PpuMI-Digested Plasmid pUC18TMlinker

Approximately 10 μg of plasmid pUC18TMlinker DNA were digested with Ppu MI restriction enzyme substantially as described in Example 3A. After phenol extraction and CHCl₃ extraction, the Ppu MI-digested DNA was ethanol precipitated as described in Example 3C. The DNA pellet, which constituted ~10 μg of Ppu MI-digested pUC18TMlinker DNA, was resuspended in 10 μl of TE buffer and stored at -20°C until ready for use.


### G. Ligation of Fragments to Construct Plasmid pUC18TM

One μg of the ~1.9 kb Ppu MI restriction fragment prepared in Example 3A was mixed with 1 μg of Ppu MI-digested pUC18TMlinker DNA prepared in Example 3F and then ligated substantially as described in Example 3D.

The ligated DNA constituted the desired plasmid pUC18TM. A restriction site and function map of the plasmid is presented in Figure 4 of the accompanying drawings. Plasmid pUC18TM has been transformed into E. coli K12 DH5αF′, as the preferred source and stock reservoir of the plasmid. The plasmid can be conventionally isolated from the strain which has been deposited (July 20, 1989), and made a part of the permanent stock culture collection of the NRRL. The strain is available under the accession number NRRL B-18524.


### H. Isolation of Plasmid pUC18TM

The ligated DNA constituting plasmid pUC18TM prepared in Example 3G was used to transform E. coli K12 DH5αF′ cells as described in Example 1. E. coli K12 DH5αF′/pUC18TM transformants were verified by restriction enzyme analysis of the plasmid DNA. A single K12 DH5αF′/pUC18TM transformant was cultured as described in Example 2A and plasmid pUC18TM DNA was isolated from this culture as described in Example 2B.


### EXAMPLE 4


### Construction of Plasmid phdTMD1


### A. Isolation of BsmI-Digested Plasmid pUC18TM

Ten μg of plasmid pUC18TM prepared and isolated as described in Example 3H were dissolved in 10 μl 10X Bsm I reaction buffer (500 mM NaCl, 100 mM Tris-HCl, pH 7.4, 100 mM MgCl₂, 100 mM 2-mercaptoethanol, 1 mg/ml bovine serum albumin), 10 μl of BsmI (~50 units, New England Biolabs) and 85 μl of H₂O. The reaction mixture was incubated at 65°C under paraffin oil, extracted once with phenol, once with CHCl₃ and the DNA was precipitated as described in Example 3C.

The Bsm I-digested pUC18TM DNA was electrophoresed in a 1% agarose gel until the ~4.2 kb and ~.46 kb fragment were separated clearly. The ~4.2 kb fragment was isolated and purified in accordance with Example 1C.

The ~8 μg of purified fragment, which constituted Bsm I-digested buffer pUC18TM DNA, was resuspended in 10 μl of TE buffer and stored at -20°C.


### B. Ligation of BsmI-Digested pUC18TM to Construct Plasmid pUC18TMD1

Half a μg of the Bsm I-digested pUC18TM DNA prepared in Example 4A was recircularized by ligation substantially as described in Example 3D. The ligated DNA constituted the desired plasmid, plasmid pUC18TMD1. A restriction site and function map of the plasmid is presented in Figure 5 of the accompanying drawings.

### C. Isolation of Plasmid pUC18TMD1

The ligated DNA constituting plasmid pUC18TMD1 prepared in Example 4B was used to transform E. coli K12 DH5αF′ cells as described in Example 1. E. coli K12 DH5αF′/pUC18TMD1 transformants were verified by restriction enzyme analysis of the plasmid DNA. A single E. coli K12 DH5αF′/pUC18TMD1 transformant was cultured as described in Example 2A and plasmid pUC18TMD1 was isolated from this culture substantially as described in Example 2B.

### D. Isolation of the ~1.5 kb BclI Restriction Fragment of Plasmid pUC18TMD1

In order to digest DNA completely with restriction enzyme Bcl I, the deoxyadenyl residue in the recognition sequence must not be methylated. When preparing plasmid DNA in E. coli for subsequent digestion with Bcl I, it is necessary to use a strain deficient in adenine methylase, such as E. coli K12 GM48, NRRL B-15725, described earlier.

E. coli K12 GM48 was prepared for transformation and then transformed with plasmid pUC18TMD1 in substantial accordance with the procedure of Example 1. Plasmid pUC18TMD1 DNA was isolated from the E. coli K12 GM48/pUC18TMD1 transformants in substantial accordance with the teaching of Example 2B.

Fifty μg of the plasmid pUC18TMD1 DNA isolated from the E. coli K12 GM48/pUC18TMD1 transformants were dissolved in 10 μl 10X Bcl I reaction buffer (750 mM KCl; 60 mM Tris-HCl, pH = 7.4; 100 mM MgCl₂; 10 mM dithiothreitol; and 1 mg/mL BSA), 5 μl (~50 units) restriction enzyme BclI, and 85 μl of H₂O, and the resulting reaction was incubated at 50° C for two hours. The Bcl I-digested plasmid pUC18TMD1 DNA was loaded onto a 1% agarose gel, and the desired ~1.5 kb Bcl I restriction fragment was isolated and purified in substantial accordance with the teaching of Example 3A. The ~10 μg of fragment obtained were dissolved in 10 μl of TE buffer and stored at -20° C.

### E. Isolation of BclI-Digested Plasmid phd

E. coli K12 GM48 was prepared for transformation and then transformed with mammalian expression plasmid phd, in substantial accordance with the procedure of Example 1. A restriction site and function map of plasmid phd is presented in Figure 6 of the accompanying drawings. E. coli K12 GM48/phd has been deposited in the NRRL and is available under the accession number NRRL B-18525 (July 26, 1989). Plasmid phd DNA was isolated from the E. coli K12 GM48/phd transformants in substantial accordance with the teaching of Example 1.

Fifty μg of the plasmid phd DNA isolated from the E. coli K12 GM48/phd transformants were dissolved in 10 μl 10X Bcl I reaction buffer (750 mM KCl; 60 mM Tris-HCl, pH = 7.4; 100 mM MgCl₂; 10 mM dithiothreitol; and 1 mg/mL BSA), 5 μl (~50 units) restriction enzyme Bcl I, and 85 μl of H₂O, and the resulting reaction was incubated at 50° C for two hours. After extraction with phenol and then extraction with CHCl₃ the digested DNA was ethanol precipitated as described in Example 3C. The pellet which constituted BclI-digested plasmid phd was resuspended in 10 μl of TE buffer and stored at -20° C.

### F. Ligation of BclI Fragments to Construct Plasmid phdTMD1

One μg of the ~1.5 kb Bcl I fragment prepared in Example 4D was mixed with 0.5 μg of Bcl I-digested plasmid phd prepared in Example 4E and ligated substantially as described in Example 3D. The ligated DNA constituted plasmid phdTMD1. A restriction site and function map of the plasmid is presented in Figure 7 of the accompanying drawings.

### G. Isolation of Plasmid phdTMD1

34

The ligated DNA constituting phdTMD1 prepared in Example 4F was used to transform E. coli K12 DH5αF' cells as described in Example 1. E. coli K12 DH5αF'/phdTMD1 transformants were verified by restriction enzyme analysis of the plasmid DNA. Transformants containing the correct orientation of the 1.5 kb Bcl I insert yielded the following restriction fragments following digestion of 1 μg plasmid DNA with Bgl II in $\overline{100}$ mM NaCl, 50 mM Tris-HCl, pH 8.0, 10 mM MgCl$_2$ at 37°C for 1 hour: ~4.6 kb, ~2.6 kb, ~1.6 kb and ~1.4 kb. Fragments were identified by electrophoresis in 1% agarose and ethidium bromide staining in the manner described in Example 1.

A single E. coli K12 DH5αF'/phdTMD1 transformant containing the correct orientation was cultured in TY-broth as described in Example 2A. Plasmid DNA was isolated from the culture as described in Example 2B. To remove residual traces of CsCl, the ~l mg of isolated plasmid phdTMD1 was resuspended in 10 ml of TE buffer and ethanol precipitated twice in accordance with Example 3C. The DNA pellet, which constituted plasmid phdTMD1 was resuspended in 1 ml of TE buffer and stored at -20°C.

## G. Isolation of Plasmid phdTMD1

The ligated DNA constituting phdTMD1 prepared in Example 4F was used to transform E. coli K12 DH5αF' cells as described in Example 1. E. coli K12 DH5αF'/phdTMD1 transformants were verified by restriction enzyme analysis of the plasmid DNA. Transformants containing the correct orientation of the 1.5 kb Bcl I insert yielded the following restriction fragments following digestion of 1 μg plasmid DNA with Bgl II in $\overline{100}$ mM NaCl, 50 mM Tris-HCl, pH 8.0, 10 mM MgCl$_2$ at 37°C for 1 hour: ~4.6 kb, ~2.6 kb, ~1.6 kb and ~1.4 kb. Fragments were identified by electrophoresis in 1% agarose and ethidium bromide staining in the manner described in Example 1.

A single E. coli K12 DH5αF'/phdTMD1 transformant containing the correct orientation was cultured in TY-broth as described in Example 2A. Plasmid DNA was isolated from the culture as described in Example 2B. To remove residual traces of CsCl, the ~1 mg of isolated plasmid phdTMD1 was resuspended in 10 ml of TE buffer and ethanol precipitated twice in accordance with Example 3C. The DNA pellet, which constituted plasmid phdTMD1 was resuspended in 1 ml of TE buffer and stored at -20°C.

## EXAMPLE 5

### Construction of AV12/phdTMD1 and 293/PhdTMD1 Transformants

The following procedure describes the construction of AV12/phdTMD1 and 293/phdTMD1 transformants. Furthermore, the procedure given is generally applicable to the cell lines previously listed as preferred cell lines in the present specification. Transformation procedures for eukaryotic host cells are well known in the art. See , e.g. , Wigler, et al , 1979, Proc. Natl. Acad. Sci. (USA) 76 , 1373 and Graham, et al ., 1973, Virology 52 , 456.

## A. Preparation of the Cells

Cultures of human 293 cells ("293 cells"), and Syrian hamster AV12~664 cells ("AV12 cells"), available from the American Type Culture Collection under the accession numbers ATCC CRL 1573 and CRL 9595, respectively, were passaged one day prior to transformation and plated at a density of 1-2 X 10$^6$ cells/100 mM tissue-culture dish in growth medium comprising Dulbecco's Modified Eagle's Medium (Gibco Laboratories Life Technologies, Inc., 3175 Stanley Road, Grand Island, NY 14072) supplemented with 10% v/v fetal calf serum (Hyclone Laboratories, 1725 South State Hwy 89-91, Logan UT 84321) and 50 μg/ml gentamycin (Gibco Laboratories Life Technologies, Grand Island, NY 14072). The medium was replaced 4 hours prior to transformation.

## B. Preparation of the DNA

Ten to twenty $\mu$g of plasmid phdTMD1 DNA were added to 62.5 $\mu$l of 2M CaCl$_2$ and 437.5 $\mu$l of H$_2$O. The 0.5 ml of DNA were then added dropwise to 0.5 ml of 2X HeBS (10 g/L Hepes (pH = 7.5); 16 g/L NaCl; 0.74 g/L KCl; 0.25 g/L Na$_2$PO$_4$; and 2 g/L dextrose), forming a precipitate. The mixture was allowed to stand for 30-40 minutes at room temperature before it was added to the cells. A longer incubation time may result in a coarser precipitate that does not transform well, but sometimes a longer incubation time may be necessary to form a precipitate.

C. Tranformation of the Cells

The 1 ml DNA solution prepared in Example 5B was added to a 100 mm dish of the 293 or AV12 cells with gentle agitation and incubated at 37°C for 3-4 hours. Using care not to detach the cells, the cells were washed twice with the growth medium described in Example 5A and returned to a 37°C incubator. Since plasmid phdTMD1 comprises a selectable marker for resistance to hygromycin B, stable transformants were selected by the addition, 72 hours post-transformation, to the growth medium of 200 $\mu$g/ml hygromycin B. Cells were allowed to propagate in this selection medium for 2-3 weeks with a change of medium every 2 days. Individual hygromycin-resistant transformants then were isolated for further propagation and analysis.

EXAMPLE 6

A. Assay of the Expressed Thrombomodulin Derivatives

Wild-type thrombomodulin forms a 1:1 stoichiometric high-affinity complex with thrombin at the endothelial cell-surface. This complex is able to rapidly convert protein C zymogen to activated protein C at physiological calcium concentrations. In the absence of thrombomodulin, thrombin-dependent activation of protein C is strongly inhibited by physiological calcium concentrations, occurring at rates greater than 1000-fold slower relative to the thrombomodulin:thrombin complex.

The assay for the expressed soluble human thrombomodulin activity produced by the vectors of the invention transformed in 293 or AV12 cells in conditioned culture medium exploits the above-mentioned properties of the thrombomodulin:thrombin complex relative to thrombin alone. This assay is performed as follows:

When screening for the soluble thrombomodulin activity, stable 293 or AV12/phdTMD1 transformants were propagated in 24-well tissue culture clusters in growth medium (described in Example 5A). When wells were >80% confluent, growth medium was removed, the cell monolayers were washed twice with Hanks Balanced Salt Solution (Gibco Laboratories Life Technologies, Inc., Grand Island, NY 14072) and 1 ml of 3:1 serum-free medium added. Serum free medium is 3 parts Dulbecco's Modified Eagles Medium to 1 part F12 Medium (both from Gibco Laboratories Life Technologies, Inc.) with 10$^{-8}$ M selenium, 5 x 10$^{-5}$ M ethanolamine and 1 $\mu$g/ml Vitamin K (all from Sigma Chemical Co., St. Louis, MO 63178); 2.4 g/l NaHCO$_3$ and 1 $\mu$g/ml transferrin (Miles Scientific, Naperville, IL 60566); 2 x 10$^{-2}$ M HEPES (Sigma Chemical Co., St. Louis, MO 63178); 1 $\mu$g/ml insulin and 1 $\mu$g/ml tobramycin (Eli Lilly and Co., Indianapolis, IN 46285; Sigma Chemical Co., St. Louis, MO 63178). After incubation overnight at 37°C, the conditioned serum-free medium was removed and centrifuged at 13,000 g to remove cellular debris and particulates.

Ten $\mu$l of conditioned medium from each sample then was added to 100 $\mu$l of assay buffer (0.02M Tris (pH 7.4), 0.1M NaCl, 3.0 mM CaCl$_2$) in 96-well plates. To each sample was added 50 $\mu$l of a solution of 50 $\mu$g/ml human protein C zymogen (obtainable according to the procedure of Grinnell, B. et al ., Biotechnology , 5 , 1189 (1987)) in assay buffer, followed by 20 $\mu$l of a solution of 2 N.I.H. units/ml of bovine thrombin (Enzyme Research Laboratories, 300 N. Michigan St. South Bend, IN 46601) in assay buffer. The samples then were incubated on a rotating plate at 37°C for 30 minutes. To block thrombin serine protease activity, 20 $\mu$l of a solution of 50 antithrombin units/ml of hirudin (Sigma Chemical Co., St. Louis, MO 63178) in assay buffer was added and the samples incubated for a further 5 minutes at 37°C. Twenty-five $\mu$l of a solution of 4 mM chromogenic substrate S-2366 (Helena Laboratories, Beaumont, TX ) in assay buffer were added and optical density readings were taken at 405 nm using a UVmax automatic plate reader (Molecular Devices Corp., Palo Alto, CA 94394). Since substrate S-2366 shows specificity for activated protein C, increases in O.D. at 405 nm are proportional to the amount of activated protein C in the sample.

Thrombomodulin activity in conditioned medium could be measured in units of μg activated protein C/ml/min by comparing O.D. readings of the samples to a standard curve of freshly prepared activated recombinant human protein C. Alternatively, an estimation of the concentration of thrombomodulin in the samples in units of μg/ml could be determined by comparison to a standard curve of purified detergent-solubilized rabbit thrombomodulin.

EXAMPLE 7

Purification of Soluble Derivatives of Human Thrombomodulin

A. Clone Expansion and Collection of Conditioned Serum-Free Medium

Using the thrombomodulin assay described in Example 6, 293 or AV12/phdTMD1 transformants were found to secrete soluble thrombomodulin activity into conditioned serum-free medium, the components of which are described in Example 6A. Thrombomodulin expression levels were found in the range 0.2-2.5 μg/ml after overnight conditioning at 37°C.

Both 293 and AV12/phdTMD1 transformants expressing ~2.0 μg/ml of the soluble human thrombomodulin derivatives of the invention, were expanded until confluent monolayers were obtained in 10 roller bottles (~850 cm²). At this stage, growth medium was replaced with 100 ml 3:1 serum-free medium/roller bottle and the cultures returned to 37°C for 24 hours. This initial 100 ml of conditioned medium was then discarded and replaced with 100 ml fresh serum-free medium. Collections of serum-free conditioned medium subsequently were made every 24 hours. The pooled conditioned medium from 10 roller bottles (~1 litre) was centrifuged at 13,000xg for 30 minutes at 4°C, decanted and stored at -20°C. Generally, 8-10 collections, providing 8-10 litres of serum-free conditioned medium were made before cultures were discarded due to overgrowth. The conditioned medium was allowed to thaw at 4°C and centrifuged at 13,000xg for 30 minutes at 4°C prior to purification procedures described below.

B. Fast-Flow Q Sepharose Anion Exchange Chromatography

A Fast-flow Q-sepharose (Pharmacia LKB Biotechnology, Inc. P. O. Box 1327, Piscataway, NJ) column was used as the initial step in the purification of the soluble human thrombomodulins of the invention. The column (1.6 x 20 cm) was pre-equilibrated with 20 column volumes of 0.1M NaCl, 0.02M Tris-HCl (pH 7.4), 0.1 mM EDTA and 0.02% w/v NaN₃, at a flow rate of 2.0 ml/min. Two litres of serum-free conditioned medium then was applied overnight at 4°C at a flow rate of 2 ml/min. The column then was washed with 10 column volumes of equilibration buffer. The thrombomodulin activity was recovered from the column in a volume of 60-100 ml by step elution with 1M NaCl, 0.02M Tris-HCl (pH 7.4), 0.1 mM EDTA, 0.02% (w/v) NaN₃.

C. Affinity Chromatography of Soluble Recombinant Thrombomodulin

Ten ml of Affigel-10 (N-hydroxysuccinimide ester of derivatized cross-linked agarose) affinity gel matrix (BioRad, Richmond, CA 94804) were washed with 200 ml ice-cold distilled H₂O. To this 10 ml of gel were added 25 ml of a solution of 2 mg/ml bovine thrombin (Enzyme Research Laboratories, South Bend, IN 46601) in 50 mM HEPES pH 7.6. The thrombin was allowed to couple with the gel matrix by gentle agitation at 4°C for 4 hours. Coupling efficiency, as determined by measuring O.D. 280 nm of the supernatant following sedimentation of the gel, was typically about 95%. Additional non-reacted sites on the affinity matrix were blocked by addition of 1.0 ml of 1M glycine (pH 8.0) and incubation overnight at 4°C. After washing the gel 10 times in 50 mM HEPES (pH 7.6) to remove residual unbound thrombin, the affinity matrix was treated with 50 mM diisopropylfluorophosphate for 10 minutes at 4°C. Treatment of the matrix in this manner inhibits the serine protease activity of bound thrombin without destroying its ability to bind recombinant thrombomodulin.

The diisopropylphosphorothrombin affinity column then was extensively washed to remove excess diisopropylfluorophosphate and equilibrated with 0.02M NaCl, 0.02M Tris-HCl (pH 7.4), 1.0 mM CaCl$_2$, 0.02% (w/v) NaN$_3$ at a flow rate of 2 ml/minute at 4° C.

The Fast flow Q-sepharose eluate prepared in Example 7B was concentrated and desalted by ultrafiltration using 0.02M NaCl, 0.02M Tris-HCl (pH 7.4), 0.001M CaCl$_2$, 0.02% (w/v) NaN$_3$. This desalted sample, containing about 70 mg protein in 40 ml buffer then was applied to the diisopropylphosphorothrombin affinity column at a flow rate of 0.5 ml/min. Unbound material was reapplied to the column by means of a loop and peristaltic pump. In this manner continuous flow of the sample over the affinity column could be maintained overnight at 4° C, so as to maximize binding of the recombinant thrombomodulin to the affinity column.

The column then was washed with 10 volumes cf equilibration buffer and bound recombinant thrombomodulin obtained in 30-50 ml by step elution with 1M NaCl, 0.02M Tris-HCl (pH 7.4), 0.0001M EDTA, 0.02% (w/v) NaN$_3$. This diisopropylphosphorothrombin affinity-purified soluble thrombomodulin then was concentrated down to a volume of 1-2 ml (10-20 mg/ml) by ultrafiltration.

## D. Size Exclusion Chromatography

The affinity purified human thrombomodulin derivatives prepared in Example 7C were subjected to size-exclusion chromatography using a Superose-6 HR 10/30 column (Pharmacia LKB Biotechnology, Inc., Piscataway, NJ 08855). The column was equilibrated overnight at room temperature with mobile phase buffer (50 mM Na$_2$PO$_4$ (pH 7.5), 0.1M NaCl, 0.1% (w/v) NaN$_3$, 0.05% (v/v) Tween-20) at a flow rate of 0.1 ml/min. The concentrated affinity-purified thrombomodulin prepared in Example 7C was applied to the column in 200 $\mu$l aliquots (3-6 purification runs). The column was eluted with mobile phase buffer at a flow rate of 0.5 ml/min. Fractions (0.5 ml) were collected and assayed for thrombomodulin activity as described in Example 6A.

The majority (>90%) of soluble recombinant thrombomodulin activity eluted from this column in fractions 25 (12.5 ml) to 40 (20 ml), and could be resolved into two major peaks based on molecular size. Consequently, two pools of soluble recombinant thrombomodulin were made. Pool #1 contained the larger molecular species (retention time ~15 ml) eluting in fractions 25-33 and Pool #2 contained the smaller molecular species (retention time ~17 ml) eluting in fractions 34-40.

## E. Anion Exchange Chromatography of the Soluble Thrombomodulin Derivatives

Both the high molecular weight and low molecular weight forms of the thrombomodulin derivatives were purified further by anion exchange chromatography on a Mono-Q HR 5/5 column (Pharmacia LKB Biotechnology, Inc., Piscataway, NJ 08855). In both cases the column was equilibrated using 0.02M NaCl, 0.02M Tris-HCl (pH 7.8), 0.001M EDTA, 0.02% NaN$_3$.

Pool #1 containing the high molecular size form of soluble thrombomodulin derivative prepared in Example 7D, was applied to the Mono-Q column at a flow rate of 1 ml/min. Greater than 95% of thrombomodulin activity was bound to the column and could be eluted with a linear 20-ml gradient from 0.02 to 2M NaCl in 0.02M Tris-HCl (pH 7.8), 0.0001M EDTA, 0.02% (w/v) NaN$_3$. A broad peak of recombinant thrombomodulin activity was eluted in a volume of ~6 ml from 1 to 1.6M NaCl. This material constituted the high molecular weight form of soluble human thrombomodulin derivative, the properties of which are described below.

Pool #2 containing the low molecular weight form of soluble human thrombomodulin derivative prepared in Example 7D was applied to and eluted from the Mono-Q column in the same manner as described for Pool #1. In this case, a sharp peak of thrombomodulin activity was eluted in a volume of ~2 ml between 0.45 and 0.65M NaCl. This material constituted the low molecular weight form of soluble human thrombomodulin, the properties of which are described below.

Using the purification procedures described above and in Examples 7B-7D, approximately 1 mg of each form of recombinant thrombomodulin can be isolated from 2 liters of serum-free conditioned medium produced from either AV12/phdTMD1 or 293/phdTMD1 transformants. The material obtained was usually greater than 95% pure.

## Example 8

Functional Characteristics of Soluble Recombinant Human Thrombomodulin

A. Determination of the Michaelis Constant ($K_m$) and the Dissociation Constant ($K_d$)

Wild-type detergent solubilized human thrombomodulin forms a high affinity 1:1 stoichiometric complex with thrombin, with a dissociation constant ($K_d$) of approximately 0.4 nM (Suzuki, K. et al . J. Biochem. , 104:628 1988). The dissociation constants of the purified human thrombomodulin derivatives, prepared as described in Example 7, were determined as follows:

The high and low molecular weight recombinant thrombomodulin derivatives, purified from both 293 and AV12 transformants, were diluted to a concentration of 500 ng/ml in 0.1 M NaCl, 0.02 M Tris-HCl (pH 7.5), 3 mM CaCl$_2$ ("Buffer A"). Twenty $\mu$l of each sample then was mixed in 96-well plates with 100 $\mu$l of Buffer A and 50 $\mu$l of a solution of 50 $\mu$l/ml of Protein C ( See , Grinell, B. supra ) in Buffer A. Ten $\mu$l of bovine thrombin (Enzyme Research Laboratories, South Bend, IN 46601) then was added from serial dilutions in Buffer A to obtain thrombin concentrations in the range of 100-0.05 nM. The reaction mixtures then were incubated at 37°C for 10 minutes on a rotating plate. After the incubation, a four-fold molar excess of hirudin (in a 20 $\mu$l volume) (Sigma Chemical Co., St. Louis, MO 63178) was added to each well. Control wells containing Buffer A, Protein C and thrombin were treated in the same way. After incubation at room temperature for an additional five minutes, 25 ml of a 4 mM solution of chromogenic substrate S2366 (Helena Laboratories, Beaumont, TX) in Buffer A was added and the O.D. at 405 nm was determined, after a two minute incubation period, using a kinetic microplate reader (UVmax, Molecular Devices Corp., Palo Alto CA 94394).

The O.D.$_{405\ nm}$ readings in the thrombomodulin-containing wells were corrected for background by subtraction of the O.D.$_{405\ nm}$ reading obtained in thrombin control wells. Under the conditions used, the O.D.$_{405\ nm}$ reading thus obtained was proportional to the amount of activated Protein C generated by the thrombomodulin:thrombin complex. The amount of activated Protein C, therefore, could be calculated from a standard curve of freshly prepared activated Protein C. By this means, the rate of Protein C activation ($\mu$g activated Protein C per minute) could be determined as a function of thrombin concentration. The data then could be fitted by non-linear regression analysis using a commercially available program (Enzfitter, Elsevier-Biosoft, Cambridge, U.K.), according to Michaelis-Menten type kinetics. This data analysis provided an estimate of the $K_m$ of thrombomodulin for thrombin and $V_{max}$ at saturating thrombin concentrations.

For determination of the $K_d$, the amount of free thrombin and thrombin bound to thrombomodulin at each concentration was determined. By definition, under $V_{max}$ conditions, the proportion of thrombomodulin complexed to thrombin was 100%. Assuming a linear relationship between the concentration of thrombomodulin:thrombin complex and the rate of Protein C activation, the amount of free and bound thrombin at each thrombin concentration could be determined. Under the conditions described, the concentration of thrombomodulin used was 0.75 nm (assuming a molecular weight for wild-type thrombomodulin of 74,000 daltons; Suzuki, et al ., J. Biochem. , 140:628 (1988)). Thus, at any given thrombin concentration:

$$[\text{Thrombin}]_{bound} = V/V_{max} \times 0.75$$

and

$$[\text{Thrombin}]_{free} = [\text{Thrombin}]_{total} - [\text{Thrombin}]_{bound}$$

where V is the rate of Protein C activation to a given thrombin concentration and $V_{max}$ is the maximal rate of Protein C activation.

Data obtained by this means were plotted as a function of [Thrombin]$_{bound}$ against [Thrombin]$_{free}$ using a one-ligand binding site non-linear regression analysis (Enzfitter, see above ), and the dissociation constant ($K_d$) was obtained.

The $K_d$ values, determined by this method, of the high and low molecular weight thrombomodulin derivatives purified from the 293 and AV12 transformants are summarized in Table 2, which follows.

B. Determination of the $K_m$ for Protein C of the Thrombomodulin:Thrombin Complex

The rate of Protein C activation by the thrombomodulin:thrombin complex has been shown to be dependent on the concentration of Protein C. The $K_m$ of wild-type human thrombomodulin:thrombin complex for Protein C has been determined to be about 0.7 $\mu$M (Suzuki, K. et al ., J. Biochem. 140:628 1988). The $K_m$ for Protein C of the thrombomodulin:thrombin complex was determined, for each of the derivatives purified according to the procedures of Example 7, in the following manner.

Soluble thrombomodulin derivatives were diluted and mixed with Buffer A as described in Example 8A, except that the concentration of Protein C was varied from 70-0.07 $\mu$M, and the thrombin concentration was kept constant at 2.2 nM. Control wells contained Buffer A, thrombin and varying Protein C concentrations. After incubation for 10 minutes at 37° C and inhibition of thrombin by addition of a four-fold molar excess of hirudin, the rate of Protein C activation was determined using chromogenic S-2366, as described in Example 8A. Readings at O.D.$_{405\ nm}$ in thrombomodulin-containing wells were corrected by subtraction of the O.D.$_{405\ nm}$ reading in Protein C control wells. Data obtained were analyzed by non-linear regression analysis using the Enzfitter program described in Example 8A. The $K_m$ of recombinant thrombomodulin:thrombin complex for Protein C obtained by this method are summarized in Table 2, which follows.

C. Determination of the Ca$^2$ Optimum for the Thrombomodulin Derivatives

Wild-type thrombomodulin displays saturable kinetics with respect to Ca$^2$, with an optimum at 1-5 mM (Esmon, N. L., et al ., J. Biol. Chem. , 259:12246 1984). The Ca$^2$ dependence of the thrombomodulin derivatives was determined by varying the Ca$^2$ concentration of assay buffers. For these experiments, the thrombomodulin derivatives, Protein C and thrombin were diluted to the appropriate concentrations in Buffer A containing from 5 mM to 0.025 mM CaCl$_2$ and mixed together essentially as described in Example 8A. The thrombomodulin derivatives were used at 0.75 nM, thrombin at 2.2 nM and Protein C at 1.4 $\mu$M. Control reactions containing Protein C, thrombin and varying amounts of CaCl$_2$ also were prepared. After activation for 10 minutes at 37° C and hirudin inhibition of thrombin, O.D. readings were obtained at 405 nm as described in Example 8A. One skilled in the art will note that the activation of Protein C by thrombomodulin:thrombin complex is Ca$^2$-dependent whereas the spontaneous activation of Protein C by thrombin alone increases with decreasing Ca$^2$ concentration. For this reason, control reactions in the absence of thrombomodulin must be performed to provide an accurate control for thrombomodulin-independent Protein C activation by thrombin. The O.D.$_{405\ nm}$ readings from such reactions were subtracted from thrombomodulin-containing reactions prior to data analysis.

When such data were plotted as the rate of Protein C activation against the Ca$^2$ concentration of reaction mixtures, significant differences were observed between the high and low molecular weight human thrombomodulin derivatives. For the high molecular weight form of human thrombomodulin purified from either 293 or AV12 transformants, the Ca$^2$-dependence showed typical Michaelis-type saturation with optimal values of Protein C activation at 1-5 mM Ca$^2$ concentrations. This value, approximates normal physiological Ca$^2$ concentrations. For the low molecular weight form purified form either 293 or AV12 transformants, the rate of Protein C activation was bimodal. In particular, there was observed a dramatic increase up to concentrations of 200-500 $\mu$M followed by progressive inhibition at higher Ca$^2$ concentrations.

The optimal Ca$^2$ concentrations for the various purified recombinant thrombomodulin derivatives are summarized in Table 2.

TABLE 2

| KINETIC PROPERTIES OF HUMAN RECOMBINANT THROMBOMODULIN DERIVATIVES | | | | |
|---|---|---|---|---|
| Form of TM | Producing Cell Line | $K_d$ for Thrombin (nM) | $K_m$ for Protein C ($\mu$M) | Optimal Ca$^{++}$ Concentration |
| High M. W. | AV12 | 2.99 ± 0.19 | 0.74 ± 0.08 | 1-5 mM |
| High M. W. | 293 | 2.66 ± 0.19 | 1.02 ± 0.12 | 1-5 mM |
| Low M. W. | AV12 | 15.6 ± 1.1 | 0.76 ± 0.06 | 200-500 $\mu$M |
| Low M. W. | 293 | 16.3 ± 1.4 | 0.78 ± 0.03 | 200-500 $\mu$M |

D. Prolongation of Human Plasma Clotting Times

A number of standardized clinical clotting assays exit for the evaluation of clotting factor deficiencies and levels of circulating anticoagulants. The recombinant thrombomodulin derivatives were tested for anticoagulant activity in two clotting tests: the activated partial thromboplastin time ("APTT"; see Koepke, J.E., Am. J. Clin. Pathol ., 63:990 (1975)) and, the thrombin clotting time ("TCT", see Koepke, J. A. Am. J. Clin. Pathol ., 68:191 (1977)). The "APTT test" quantifies the activation of the Protein C anticoagulant pathway, and measures the diminished clotting activity arising as a consequence of the proteolytic degradation of cofactors Va and VIIIa by activated Protein C. The thrombin clotting time test (TCT) is normal even with full activation of the Protein C anticoagulant pathway. Rather, a prolongation of the TCT reflects the inhibition of thrombin's procoagulant activity by thrombomobulin and the thrombomodulin derivatives described herein. Human plasma used for these studies was collected by atraumatic venipuncture and prepared by mixing 9 volumes of whole blood with 1 volume of 0.11 M sodium citrate, followed by centrifugation at 2,000xg for 10 minutes. The supernatant plasma was stored on ice until use. Both the APTT and TCT tests were performed using an automated clotting timer and disposable plastic cuvettes (both from American Labor, Largo, FL 33543). For use in the APTT test, 50 $\mu$l of plasma or plasma containing a given concentration of recombinant thrombomodulin derivative (1-5 $\mu$g/ml) was mixed with 50 $\mu$l of actin activated cephaloplastin reagent (Baxter Healthcare Corp., McGaw Park, IL 60085) for 3 minutes at 37° C. Fifty $\mu$l of 0.02 M CaCl$_2$ then was added and the clotting time determined. The APTT test results for high and low molecular weight thrombomodulin derivatives purified from both AV12 and 293 cells are summarized in Table 3.

For the TCT test, 50 $\mu$l of plasma or plasma containing recombinant thrombomodulin derivative were mixed with 100 $\mu$l of 0.05 M imidazole buffer (pH 7.3) and incubated at 37° C for 120 seconds. Clotting was initiated by the addition of 50 $\mu$l of thrombin (Parke-Davis, Detroit, MI). Thrombin was freshly diluted in distilled water and used at a concentration sufficient to give a clotting time of approximately 20 seconds. The TCT test results for the various recombinant human thrombomodulin derivatives are summarized in Table 4.

As one skilled in the art will appreciate from the results shown in Tables 3 and 4, both high and low molecular weight forms of recombinant thrombomodulin from both 293 and AV12 transformants possessed substantial anticoagulant activity. In both the APTT test which reflects activation of the Protein C anticoagulant pathway and in the TCT test, which reflects direct inhibition of the procoagulant activity of thrombin, the high molecular weight form of recombinant thrombomodulin was found to be a more effective anticoagulant than the low molecular weight form. Particularly striking is the effect of the high molecular weight form on the thrombin clotting time which, in fact, exceeds that achievable with rabbit thrombomodulin purified from natural sources. Concentrations of high molecular weight recombinant thrombomodulin above 5 $\mu$g/ml (APTT test) and 3 $\mu$g/ml (TCT test) prolonged the clotting time indefinitely ( i.e ., no clot formation was observed).

TABLE 3

| Effect of Purified Human Thrombomodulin Derivatives On the Activated Partial Thromboplastin Time (APTT) of Human Plasma ** | | | | | |
|---|---|---|---|---|---|
| | | Clotting Time (Seconds) | | | |
| Form of TM | Producing Cell Line | 0 $\mu$g/ml * | 1.0 $\mu$g/ml * | 2.0 $\mu$g/ml * | 4.0 $\mu$g/ml * |
| High M. W. | AV12 | 33.4 ± 1.2 | 53.7 ± 1.7 | 67.1 ± 1.6 | 93.9 ± 2.6 |
| High M. W. | 293 | 32.3 ± 0.5 | 45.4 ± 0.8 | 59.4 ± 1.0 | 70.9 ± 2.1 |
| Low M. W. | AV12 | 33.0 ± 1.0 | 35.9 ± 0.4 | 36.0 ± 0.7 | 44.8 ± 0.9 |
| Low M. W. | 293 | 32.3 ± 0.4 | 35.6 ± 0.6 | 37.5 ± 0.2 | 43.6 ± 0.8 |

* Amount of Sample added to human plasma
** Clotting times are shown as the mean ± the standard deviation from 3 experiments

TABLE 4

| Effect of Purified Human Thrombomodulin Derivatives On the Thrombin Clotting Time (TCT) of Human Plasma ** | | | | | |
|---|---|---|---|---|---|
| | | Clotting Time (Seconds) | | | |
| Form of TM | Producing Cell Line | 0 μg/ml * | 1.0 μg/ml * | 2.0 μg/ml * | 3.0 μg/ml * |
| High M. W. | AV12 | 19.7 ± 0.8 | 25.6 ± 0.4 | 40.2 ± 1.8 | 86.7 ± 2.9 |
| High M. W. | 293 | 19.9 ± 0.9 | 24.4 ± 0.6 | 34.2 ± 1.2 | 58.3 ± 1.4 |
| Low M. W. | AV12 | 19.9 ± 0.9 | 20.8 ± 0.4 | 23.4 ± 0.4 | 24.4 ± 1.2 |
| Low M. W. | 293 | 20.0 ± 0.6 | 21.3 ± 0.4 | 24.2 ± 0.2 | 29.0 ± 1.4 |

\* Amount of Sample added to human plasma

\*\* Clotting times are shown as the mean ± the standard deviation from 3 experiments

E. Inhibition of Thrombin-dependent Platelet Activation

Wild type thrombomodulin has been shown to inhibit thrombin-dependent platelet activation as measured by inhibition of thrombin-induced platelet serotonin release (Esmon, N. L., et al ., J. Biol. Chem ., 258:12238, (1983) and, Maruyama, I., et al ., J. Clin. Invest ., 75:987, (1985)). The influence of the various purified human thrombomodulin derivatives on thrombin-induced serotonin release from platelets was determined as follows:

Whole blood was collected as described in Example 8D and platelet-rich plasma was prepared by centrifugation at 1000xg for 3 minutes. The platelet-rich plasma was mixed with an equal volume of modified Tangen's buffer (0.145 M NaCl, 0.005 M KCl, 0.05 mM $CaCl_2$, 0.1 mM $MgCl_2$, 0.0055 M glucose, 0.015 M HEPES (pH 7.4) and 1 mg/ml bovine serum albumin), and platelets pelleted by centrifugation at 900xg for 10 minutes. The plates were gently resuspended in Tangen's buffer at one-fifth the original volume of plasma and slowly equilibrated at 37°C. The platelet suspension was incubated with 0.1 μCi/ml $^{14}$C-5-hydroxytryptamine (Amersham Corp., Arlington Heights, IL 60005) for 1 hour at 37°C. Excess radiolabel was removed by washing the platelet suspension three times in Tangen's buffer.

The platelet pellet finally obtained was gently resuspended to the original plasma volume in Tangen's buffer and kept at room temperature until use. The final suspension contained $5 \times 10^8$ platelets per ml, as counted by use of a hemocytometer.

For measurement of $^{14}$C-serotonin release, 0.5 ml of the platelet suspension was pre-warmed to 37°C with constant stirring for 5 minutes. Thrombin then was added at a concentration of 1 nM and the incubation continued for 3 minutes. Aliquots 0.1 ml then were removed and mixed with 0.9 ml of ice-cold stop buffer (0.05 M sodium phosphate (pH 7.4), 0.2% (v/v) glutaraldehyde and 0.005 M EDTA). After centrifugation for 30 seconds at 8000xg, a 0.5 ml aliquot was removed and mixed with 9 ml Aquasol scintillation cocktail (New England Nuclear, Boston, MA 02118) and the $^{14}$C was counted using a Beckman LS3801 Scintillation Counter. The total counts were determined by mixing 0.1 ml of the platelet suspension with 0.9 ml stop buffer and using a 0.5 ml aliquot of this mixture for scintillation counting. The background released counts were determined by incubating the platelet suspension for 3 minutes at 37°C in the absence of thrombin.

$^{14}$C-serotonin release was determined using the following equation:

$$\%release = \frac{(released\ counts-background)}{(total\ counts-background)} \times 100$$

The data obtained for $^{14}$C-serotonin release in the presence 1 nM thrombin mixed with various concentration of the human thrombomodulin derivatives are summarized in Table 5. The data clearly indicate that both the high and low molecular weight forms of thrombomodulin derivative are effective in preventing activation of platelets as reflected by serotonin release. The high molecular weight from is particularly effective in this regard. The high molecular weight form, in particular, consistently was found to inhibit more efficiently thrombin-dependent platelet activation than did rabbit thrombomodulin. This result was unexpected because TM prepared from human placenta was thirty-fold less efficient in this regard. ( See , Maruyama, et al ., J. Clin. Invest . 75 , 987 (1985)).

Table 5

| The Effect of Human Thrombomodulin Derivatives on Thrombin-Induced $^{14}$C-Serotonin Release From Human Platelets | | | | |
|---|---|---|---|---|
| | | * %$^{14}$C-Serotonin Release Thrombomodulin Concentration | | |
| Form of TM | Producing Cell Line | 0 nM | 10 nM | 20 nM |
| High M. W. | AV12 | 80 | 16 | 7 |
| High M. W. | 293 | 80 | 14 | 12.5 |
| Low M. W. | AV12 | 80 | 64 | 57 |
| Low M. W. | 293 | 80 | 64 | 54 |

* The %$^{14}$C-serotonin released was determined in the presence of 1 nM thrombin for 3 minutes at 37° C.

F. Inhibition of Thrombin-Induced Platelet Aggregation

Wild-type detergent-solubilized thrombomodulin has been shown to inhibit thrombin-induced platelet activation as measured by inhibition of thrombin-induced platelet aggregation (Esmon, N.L. et al ., J. Biol. Chem . 258:12238-12242, 1983). The ability of the human thrombomodulin derivatives of the invention to inhibit platelet aggregation was evaluated in platelet-rich human plasma as follows:

Nine volumes of freshly drawn human blood were mixed with 1 volume of 3.8% (w/v) sodium citrate and centrifuged at 100xg for 25 minutes to obtain platelet-rich human plasma. A sample of platelet-poor plasma was also prepared by centrifugation of whole blood at 2000xg for 15 minutes. Aggregation studies were performed using a commercially available aggregometer and aggregometer cuvettes according to the manufacturer's instructions (Helena Monitor IV, Helena Laboratories, Beaumont, TX). For each experiment, correction for background absorbance due to plasma was performed by placing a 0.5 ml sample of platelet-poor plasma in an aggregometer cuvette and zeroing the instrument. A 0.5 ml sample of platelet-rich plasma then was placed in the instrument and brought to 37° C by continuous stirring. Thrombin then was added at a concentration of 2 nM and platelet aggregation monitored automatically as the decrease in absorbance over time (as platelets aggregate there is less light scattering and the absorbance of platelet-rich plasma will decrease). Maximal platelet aggregation typically was observed between 30 and 60 seconds following addition of thrombin. When either the low or high molecular weight thrombomodulin derivatives (from both 293 and AV12 transformants) was premixed with thrombin at a thrombomodulin to thrombin ratio of 50:1, complete inhibition of the platelet aggregation response was observed. Addition of recombinant thrombomodulin to the aggregometer cuvette after the addition of thrombin resulted in decreased inhibition of the aggregation response, with nearly maximal aggregation occurring if thrombomodulin was added more than 20 seconds after the addition of thrombin.

G. Estimation of Molecular Weight by Electophoretic Properties

Wild-type thrombomodulin exhibits anomalous behavior on sodium dodecyl sulfate polyacrylamide gel electrophoresis (SDS-PAGE). The molecular weight of the protein ranges from 74-84 kD before and 88-110 kD after disulfide bond reduction (Esmon, N. L. et al ., J. Biol. Chem ., 257:859 (1981) and, Suzuki, K. et al ., J. Biochem ., 104:628 (1988)).

The molecular weights of the various human thrombomodulin derivatives purified as described in Example 7 were determined by SDS-PAGE under both reducing and non-reducing conditions. The electrophoresis was performed with ultra-thin 10-15% gradient gels using a commerically available automated electophoresis system (PhastSystem, Pharmacia LKB, Uppsala, Sweden) according to the manufacturer's instructions. Molecular weight standards and protein standards (BioRad Laboratories, Richmond, CA 94804) were stained using the PhastSystem silver staining method according to the manufacturer's instruction. Typically, 0.2 to 1.0 $\mu$g of each recombinant thrombomodulin derivative were required to obtain good staining visibility.

Molecular weights of the recombinant thrombomodulin derivatives were determined by reference to a plot of molecular weights of standard proteins against the distance the proteins migrated through the gel. The values obtained using this procedure are summarized in Table 6.

TABLE 6

| Molecular Weight Determination of Recombinant Thrombomodulin Derivatives | | | |
|---|---|---|---|
| | | Molecular Weight (kDaltons) | |
| Form of TM | Producing Cell Line | Reduced | Non-Reduced |
| High M. W. | AV12 | 95-110 | 78-94 |
| High M. W. | 293 | 95-110 | 76-94 |
| Low M. W. | AV12 | 73-77 | 61-66 |
| Low M. W. | 293 | 73-77 | 58-64 |

EXAMPLE 9

A. Chondroitinase ABC Treatment of Soluble Thrombomodulin Derivatives

200 $\mu$l (75 $\mu$g/ml) of the soluble thrombomodulin derivatives purified from 293 cells were incubated overnight at 37°C both with and without chondroitinase ABC (50 milliunits, Sigma Chemical Co., St. Louis, MO) in 100 mM NaCl, 50mM Tris-HCl, 30 $\mu$M sodium acetate (pH 8.0), in the presence of 2.5mM 1,10-phenanthroline, 10 $\mu$g/ml pepstatin and 20 $\mu$g/ml leupeptin.

B. Thrombin Clotting Time of Chondroitinase Treated Thrombomodulin Derivatives

The chondrioitinase treated derivatives of Example 9A were used to determine the effect on thrombin clotting time of human fibrinogen.

The clotting reactions contained 3 mg/ml of human fibrinogen in 150mM NaCl, 20mM Tris-HCl, 3mM CaCl$_2$, pH 7.4 at 37°C. The clotting time was measured after the addition of 16nM thrombin. The chondroitinase treated thrombomodulin derivatives of Example 9A were added at a concentration of 100nM. The results of this study are shown in Table 7.

Table 7

| Effect of Chondroitinase ABC on Soluble Thrombomodulin Derivatives | |
| --- | --- |
| Sample | Clotting Time (secs) |
| Thrombin | 22 ± 1 |
| Thrombin + Chondroitinase | 24 ± 1 |
| Thrombin + low m.w. Derivative | 53 ± 1 |
| Thrombin + low m.w. Derivative + Chondroitinase | 52 ± 1 |
| Thrombin + high m.w. Derivative | 250 ± 6 |
| Thrombin + high m.w. Derivative + Chondroitinase | 102 ± 4 |

**Claims**

1. An amino acid sequence which, in order from the N-terminus, comprises:
a) the N-terminal;
b) epidermal growth factor homology; and
c) serine/threonine rich
regions of human thrombomodulin, said amino acid sequence lacking the transmembrane and cytoplasmic domains of human thrombomodulin, said amino acid sequence derivable from an AV12 or 293 host cell transformed with a recombinant DNA vector encoding said amino acid sequence.

2. An amino acid sequence which is:

$$H_2N-(R)_x(R^1)_y-\text{AlaProAlaGluProGlnProGlyGlySerGlnCysValGluHisAsp}$$

CysPheAlaLeuTyrProGlyProAlaThrPheLeuAsnAlaSerGlnIle

CysAspGlyLeuArgGlyHisLeuMetThrValArgSerSerValAlaAla

AspValIleSerLeuLeuLeuAsnGlyAspGlyGlyValGlyArgArgArg

LeuTrpIleGlyLeuGlnLeuProProGlyCysGlyAspProLysArgLeu

GlyProLeuArgGlyPheGlnTrpValThrGlyAspAsnAsnThrSerTyr

SerArgTrpAlaArgLeuAspLeuAsnGlyAlaProLeuCysGlyProLeu

CysValAlaValSerAlaAlaGluAlaThrValProSerGluProIleTrp

GluGluGlnGlnCysGluValLysAlaAspGlyPheLeuCysGluPheHis

PheProAlaThrCysArgProLeuAlaValGluProGlyAlaAlaAlaAla

AlaValSerIleThrTyrGlyThrProPheAlaAlaArgGlyAlaAspPhe

GlnAlaLeuProValGlySerSerAlaAlaValAlaProLeuGlyLeuGln

LeuMetCysThrAlaProProGlyAlaValGlnGlyHisTrpAlaArgGlu

AlaProGlyAlaTrpAspCysSerValGluAsnGlyGlyCysGluHisAla

```
CysAsnAlaIleProGlyAlaProArgCysGlnCysProAlaGlyAlaAla
LeuGlnAlaAspGlyArgSerCysThrAlaSerAlaThrGlnSerCysAsn
AspLeuCysGluHisPheCysValProAsnProAspGlnProGlySerTyr
SerCysMetCysGluThrGlyTyrArgLeuAlaAlaAspGlnHisArgCys
GluAspValAspAspCysIleLeuGluProSerProCysProGlnArgCys
ValAsnThrGlnGlyGlyPheGluCysHisCysTyrProAsnTyrAspLeu
ValAspGlyGluCysValGluProValAspProCysPheArgAlaAsnCys
GluTyrGlnCysGlnProLeuAsnGlnThrSerTyrLeuCysValCysAla
GluGlyPheAlaProIleProHisGluProHisArgCysGlnMetPheCys
AsnGlnThrAlaCysProAlaAspCysAspProAsnThrGlnAlaSerCys
GluCysProGluGlyTyrIleLeuAspAspGlyPheIleCysThrAspIle
AspGluCysGluAsnGlyGlyPheCysSerGlyValCysHisAsnLeuPro
GlyThrPheGluCysIleCysGlyProAspSerAlaLeuAlaArgHisIle
GlyThrAspCysAspSerGlyLysValAspGlyGlyAspSerGlySerGly
GluProProProSerProThrProGlySerThrLeuThrProProAlaVal
GlyLeuValHisSer-COOH
```

wherein

ALA is Alanine, AFG is Arginine, ASN is Asparagine, ASP is Aspartic Acid, CYS is Cysteine, GLN is Glutamine, GLU is Glutamic Acid, GLY is Glycine, HIS is Histidine, ILE is Isoleucine, LEU is Leucine, LYS is Lysine, MET is Methionine, PHE is Phenylalanine, PRO is Proline, SER is Serine, THR is Threonine, TRP is Tryptophan, TYR is Tyrosine, and VAL is Valine;

R is MetLeuGlyValLeuValLeuGlyAlaLeuAlaLeuAla GlyLeuGly-;

$R^1$ is PhePro-;

x is 0 or 1;

y is 0 or 1, provided that if y = 0, then x must be 0 and if x = 1, then y must be 1.

3. The amino acid sequence of Claim 1 or Claim 2 which is glycosylated.

4. The amino acid sequence of Claim 1 or Claim 2 which is not glycosylated.

5. The amino acid sequence of Claim 1 or Claim 2 which is

```
H₂N-AlaProAlaGluProGlnProGlyGlySerGlnCysValGluHisAspCys
      PheAlaLeuTyrProGlyProAlaThrPheLeuAsnAlaSerGlnIleCys
      AspGlyLeuArgGlyHisLeuMetThrValArgSerSerValAlaAlaAsp
      ValIleSerLeuLeuLeuAsnGlyAspGlyGlyValGlyArgArgArgLeu
      TrpIleGlyLeuGlnLeuProProGlyCysGlyAspProLysArgLeuGly
      ProLeuArgGlyPheGlnTrpValThrGlyAspAsnAsnThrSerTyrSer
      ArgTrpAlaArgLeuAspLeuAsnGlyAlaProLeuCysGlyProLeuCys
      ValAlaValSerAlaAlaGluAlaThrValProSerGluProIleTrpGlu
      GluGlnGlnCysGluValLysAlaAspGlyPheLeuCysGluPheHisPhe
      ProAlaThrCysArgProLeuAlaValGluProGlyAlaAlaAlaAlaAla
      ValSerIleThrTyrGlyThrProPheAlaAlaArgGlyAlaAspPheGln
      AlaLeuProValGlySerSerAlaAlaValAlaProLeuGlyLeuGlnLeu
      MetCysThrAlaProProGlyAlaValGlnGlyHisTrpAlaArgGluAla
      ProGlyAlaTrpAspCysSerValGluAsnGlyGlyCysGluHisAlaCys
      AsnAlaIleProGlyAlaProArgCysGlnCysProAlaGlyAlaAlaLeu
      GlnAlaAspGlyArgSerCysThrAlaSerAlaThrGlnSerCysAsnAsp
      LeuCysGluHisPheCysValProAsnProAspGlnProGlySerTyrSer
      CysMetCysGluThrGlyTyrArgLeuAlaAlaAspGlnHisArgCysGlu
      AspValAspAspCysIleLeuGluProSerProCysProGlnArgCysVal
      AsnThrGlnGlyGlyPheGluCysHisCysTyrProAsnTyrAspLeuVal


      AspGlyGluCysValGluProValAspProCysPheArgAlaAsnCysGlu
      TyrGlnCysGlnProLeuAsnGlnThrSerTyrLeuCysValCysAlaGlu
      GlyPheAlaProIleProHisGluProHisArgCysGlnMetPheCysAsn
      GlnThrAlaCysProAlaAspCysAspProAsnThrGlnAlaSerCysGlu
      CysProGluGlyTyrIleLeuAspAspGlyPheIleCysThrAspIleAsp
      GluCysGluAsnGlyGlyPheCysSerGlyValCysHisAsnLeuProGly
      ThrPheGluCysIleCysGlyProAspSerAlaLeuAlaArgHisIleGly
      ThrAspCysAspSerGlyLysValAspGlyGlyAspSerGlySerGlyGly
      ProProProSerProThrProGlySerThrLeuThrProProAlaValGly
      LeuValHisSer-COOH
```

wherein
ALA is Alanine, ARG is Arginine, ASN is Asparagine, ASP is Aspartic Acid, CYS is Cysteine, GLN is Glutamine, GLU is Glutamic Acid, GLY is Glycine, HIS is Histidine, ILE is Isoleucine, LEU is Leucine, LYS is Lysine, MET is Methionine, PHE is Phenylalanine, PRO is Proline, SER is Serine, THR is Threonine, TRP is Tryptophan, TYR is Tyrosine, and VAL is Valine.

6. The amino acid sequence of Claim 5 which is glycosylated.

7. The amino acid sequence of Claim 5 which is not glycosylated.

8. A polypeptide product produced by culturing, under conditions suitable for expression, a host cell, said host cell transformed with a recombinant DNA expression vector comprising a DNA sequence, said DNA sequence being:

70                                    90

5'-(R')$_x$(R$^{1'}$)$_y$-GCACCCGCAGAGCCGCAGCCGGGTGGCAGCCAGTGCGTCGAGCACGAC

110                130                150

TGCTTCGCGCTCTACCCGGGCCCCGCGACCTTCCTCAATGCCAGTCAGATC

170                190

TGCGACGGACTGCGGGGCCACCTAATGACAGTGCGCTCCTCGGTGGCTGCC

210                230                250

GATGTCATTTCCTTGCTACTGAACGGCGACGGCGGCGTTGGCCGCCGGCGC

270                290

CTCTGGATCGGCCTGCAGCTGCCACCCGGCTGCGGCGACCCCAAGCGCCTC

310                330                350

GGGCCCCTGCGCGGCTTCCAGTGGGTTACGGGAGACAACAACACCAGCTAT

370                390                4

AGCAGGTGGGCACGGCTCGACCTCAATGGGGCTCCCCTCTGCGGCCCGTTG

10                 430                450

TGCGTCGCTGTCTCCGCTGCTGAGGCCACTGTGCCCAGCGAGCCGATCTGG

470                490                510

GAGGAGCAGCAGTGCGAAGTGAAGGCCGATGGCTTCCTCTGCGAGTTCCAC

530                550

TTCCCAGCCACCTGCAGGCCACTGGCTGTGGAGCCCGGCGCCGCGGCTGCC

570                590                610

GCCGTCTCGATCACCTACGGCACCCCGTTCGCGGCCCGCGGAGCGGACTTC

630                        650

CAGGCGCTGCCGGTGGGCAGCTCCGCCGCGGTGGCTCCCCTCGGCTTACAG

670                        690                        710

CTAATGTGCACCGCGCCGCCCGGAGCGGTCCAGGGGCACTGGGCCAGGGAG

730                        750

GCGCCGGGCGCTTGGGACTGCAGCGTGGAGAACGGCGGCTGCGAGCACGCG

770                        790                        810

TGCAATGCGATCCCTGGGGCTCCCCGCTGCCAGTGCCCAGCCGGCGCCGCC

830                        850

CTGCAGGCAGACGGGCGCTCCTGCACCGCATCCGCGACGCAGTCCTGCAAC

870                        890                        910

GACCTCTGCGAGCACTTCTGCGTTCCCAACCCCGACCAGCCGGGGCTCCTAC

930                        950                        97

TCGTGCATGTGCGAGACCGGCTACCGGCTGGCGGCCGACCAACACCGGTGC

0                        990                        1010

GAGGACGTGGATGACTGCATACTGGAGCCCAGTCCGTGTCCGCAGCGCTGT

1030                        1050                        1070

GTCAACACACAGGGTGGCTTCGAGTGCCACTGCTACCCTAACTACGACCTG

1090                        1110

GTGGACGGCGAGTGTGTGGAGCCCGTGGACCCGTGCTTCAGAGCCAACTGC

1130                        1150                        1170

GAGTACCAGTGCCAGCCCCTGAACCAAACTAGCTACCTCTGCGTCTGCGCC

                    1190                    1210

GAGGGCTTCGCGCCCATTCCCCACGAGCCGCACAGGTGCCAGATGTTTTGC

         1230              1250              1270

AACCAGACTGCCTGTCCAGCCGACTGCGACCCCAACACCCAGGCTAGCTGT

              1290              1310

GAGTGCCCTGAAGGCTACATCCTGGACGACGGTTTCATCTGCACGGACATC

    1330              1350              1370

GACGAGTGCGAAAACGGCGGCTTCTGCTCCGGGGTGTGCCACAACCTCCCC

              1390              1410              14

GGTACCTTCGAGTGCATCTGCGGGCCCGACTCGGCCCTTGCCCGCCACATT

    30              1450              1470

GGCACCGACTGTGACTCCGGCAAGGTGGACGGTGGCGACAGCGGCTCTGGC

              1490              1510              1530

GAGCCCCCGCCCAGCCCGACGCCCGGCTCCACCTTGACTCCTCCGGCCGTG

              GGGCTCGTGCATTCG-3'

in which
R′ is 5′-ATGCTTGGGGTCCTGGTCCTTGGCGCGCTG GCCCTGGCCGGCCTGGGG-3′;
R¹′ is 5′-TTCCCC-3′;
x is 0 or 1;
y is 0 or 1, provided that if y = 0, then x must be 0, and if x = 1, then y must be 1;
A is deoxyadenyl,
G is deoxyguanyl,
C is deoxycytidyl, and T is thymidyl.

9. The polypeptide product of Claim 8 when expressed in a prokaryotic host cell.

10. The polypeptide product of Claim 8 when expressed in a eukaryotic host cell.

11. The polypeptide product of Claim 10 when expressed in a 293 or AV12 cell.

12. The polypeptide product of Claim 11 having a molecular weight of about 110 kD (reducing conditions) and about 76-94 kD (non-reducing conditions).

13. The polypeptide product of Claim 11 having a molecular weight of about 73 to about 77 kD (reducing conditions) and about 58-66 kD (non-reducing conditions).

14. A constructed DNA compound which comprises the sequence:

70                                        90

$5'-(R')_x(R^{1'})_y$-GCACCCGCAGAGCCGCAGCCGGGTGGCAGCCAGTGCGTCGAGCACGAC

110                     130                     150

TGCTTCGCGCTCTACCCGGGCCCCGCGACCTTCCTCAATGCCAGTCAGATC

170                     190

TGCGACGGACTGCGGGGCCACCTAATGACAGTGCGCTCCTCGGTGGCTGCC

210                     230                     250

GATGTCATTTCCTTGCTACTGAACGGCGACGGCGGCGTTGGCCGCCGGCGC

270                     290

CTCTGGATCGGCCTGCAGCTGCCACCCGGCTGCGGCGACCCCAAGCGCCTC

310                     330                     350

GGGCCCCTGCGCGGCTTCCAGTGGGTTACGGGAGACAACAACACCAGCTAT

370                     390                     4

AGCAGGTGGGCACGGCTCGACCTCAATGGGGCTCCCCTCTGCGGCCCGTTG

10                      430                     450

TGCGTCGCTGTCTCCGCTGCTGAGGCCACTGTGCCCAGCGAGCCGATCTGG

470                     490                     510

GAGGAGCAGCAGTGCGAAGTGAAGGCCGATGGCTTCCTCTGCGAGTTCCAC

530                     550

TTCCCAGCCACCTGCAGGCCACTGGCTGTGGAGCCCGGCGCCGCGGCTGCC

570                     590                     610

GCCGTCTCGATCACCTACGGCACCCCGTTCGCGGCCCGCGGAGCGGACTTC

                    630                      650

CAGGCGCTGCCGGTGGGCAGCTCCGCCGCGGTGGCTCCCCTCGGCTTACAG

              670              690                      710

CTAATGTGCACCGCGCCGCCCGGAGCGGTCCAGGGGCACTGGGCCAGGGAG

                      730              750

GCGCCGGGCGCTTGGGACTGCAGCGTGGAGAACGGCGGCTGCGAGCACGCG

              770              790                      810

TGCAATGCGATCCCTGGGGCTCCCCGCTGCCAGTGCCCAGCCGGCGCCGCC

                      830              850

CTGCAGGCAGACGGGCGCTCCTGCACCGCATCCGCGACGCAGTCCTGCAAC

      870                      890                      910

GACCTCTGCGAGCACTTCTGCGTTCCCAACCCCGACCAGCCGGGCTCCTAC

                      930              950                      97

TCGTGCATGTGCGAGACCGGCTACCGGCTGGCGGCCGACCAACACCGGTGC

      0                      990                      1010

GAGGACGTGGATGACTGCATACTGGAGCCCAGTCCGTGTCCGCAGCGCTGT

                      1030              1050                      1070

GTCAACACACAGGGTGGCTTCGAGTGCCACTGCTACCCTAACTACGACCTG

                      1090              1110

GTGGACGGCGAGTGTGTGGAGCCCGTGGACCCGTGCTTCAGAGCCAACTGC

      1130              1150                      1170

GAGTACCAGTGCCAGCCCCTGAACCAAACTAGCTACCTCTGCGTCTGCGCC

```
          1190                1210
           .       .       .       .       .
GAGGGCTTCGCGCCCATTCCCCACGAGCCGCACAGGTGCCAGATGTTTTGC

       1230              1250              1270
        .       .       .       .       .       .
AACCAGACTGCCTGTCCAGCCGACTGCGACCCCAACACCCAGGCTAGCTGT

          1290                1310
           .       .       .       .       .
GAGTGCCCTGAAGGCTACATCCTGGACGACGGTTTCATCTGCACGGACATC

   1330              1350              1370
    .       .       .       .       .       .
GACGAGTGCGAAAACGGCGGCTTCTGCTCCGGGGTGTGCCACAACCTCCCC

          1390                1410              14
           .       .       .       .   .   .
GGTACCTTCGAGTGCATCTGCGGGCCCGACTCGGCCCTTGCCCGCCACATT

   30               1450              1470
    .       .       .       .       .       .
GGCACCGACTGTGACTCCGGCAAGGTGGACGGTGGCGACAGCGGCTCTGGC

          1490                1510              1530
           .       .       .       .       .       .
GAGCCCCCGCCCAGCCCGACGCCCGGCTCCACCTTGACTCCTCCGGCCGTG

                    .
GGGCTCGTGCATTCG-3'
```

in which

R′ is 5′-ATGCTTGGGGTCCTGGTCCTTGGCGCGCTG GCCCTGGCCGGCCTGGGG-3′;

R¹′ is 5′-TTCCCC-3′;

x is 0 or 1;

y is 0 or 1, provided that if y = 0, then x must be 0, and if x = 1, then y must be 1;

A is deoxyadenyl,

G is deoxyguanyl,

C is deoxycytidyl, and T is thymidyl.

15. A DNA compound of Claim 14 which comprises the sequence:

```
          10                    30                    50
          .          .         .          .          .          .
GCACCCGCAGAGCCGCAGCCGGGTGGCAGCCAGTGCGTCGAGCACGACTGC

                    70                    90
          .          .         .          .          .          .
TTCGCGCTCTACCCGGGCCCCGCGACCTTCCTCAATGCCAGTCAGATCTGC

          110                   130                   150
          .          .         .          .          .          .
GACGGACTGCGGGGCCACCTAATGACAGTGCGCTCCTCGGTGGCTGCCGAT

                    170                   190
          .          .         .          .          .          .
GTCATTTCCTTGCTACTGAACGGCGACGGCGGCGTTGGCCGCCGGCGCCTC

          210                   230                   250
          .          .         .          .          .          .
TGGATCGGCCTGCAGCTGCCACCCGGCTGCGGCGACCCCAAGCGCCTCGGG

                    270                   290
          .          .         .          .          .          .
CCCCTGCGCGGCTTCCAGTGGGTTACGGGAGACAACAACACCAGCTATAGC

          310                   330                   350
          .          .         .          .          .          .
AGGTGGGCACGGCTCGACCTCAATGGGGCTCCCCTCTGCGGCCCGTTGTGC

                    370                   390                   4
          .          .         .          .          .          .
GTCGCTGTCTCCGCTGCTGAGGCCACTGTGCCCAGCGAGCCGATCTGGGAG

10                    430                   450
          .          .         .          .          .          .
GAGCAGCAGTGCGAAGTGAAGGCCGATGGCTTCCTCTGCGAGTTCCACTTC
```

                470                   490                   510
                 .         .          .         .          .         .
CCAGCCACCTGCAGGCCACTGGCTGTGGAGCCCGGCGCCGCGGCTGCCGCC

                     530                   550
                      .         .          .         .          .
GTCTCGATCACCTACGGCACCCCGTTCGCGGCCCGCGGAGCGGACTTCCAG

                570                   590                   610
                 .         .          .         .          .         .
GCGCTGCCGGTGGGCAGCTCCGCCGCGGTGGCTCCCCTCGGCTTACAGCTA

                     630                   650
                      .         .          .         .          .
ATGTGCACCGCGCCGCCCGGAGCGGTCCAGGGGCACTGGGCCAGGGAGGCG

                670                   690                   710
                 .         .          .         .          .         .
CCGGGCGCTTGGGACTGCAGCGTGGAGAACGGCGGCTGCGAGCACGCGTGC

                     730                   750
                      .         .          .         .          .
AATGCGATCCCTGGGGCTCCCCGCTGCCAGTGCCCAGCCGGCGCCGCCCTG

                770                   790                   810
                 .         .          .         .          .         .
CAGGCAGACGGGCGCTCCTGCACCGCATCCGCGACGCAGTCCTGCAACGAC

                     830                   850
                      .         .          .         .          .
CTCTGCGAGCACTTCTGCGTTCCCAACCCCGACCAGCCGGGCTCCTACTCG

870                   890                   910
 .         .          .         .          .         .
TGCATGTGCGAGACCGGCTACCGGCTGGCGGCCGACCAACACCGGTGCGAG

                930                   950                   97
                 .         .          .         .          .         .
GACGTGGATGACTGCATACTGGAGCCCAGTCCGTGTCCGCAGCGCTGTGTC

0                   990                   1010
 .         .          .         .          .         .
AACACACAGGGTGGCTTCGAGTGCCACTGCTACCCTAACTACGACCTGGTG

```
              1030                1050                1070
              .        .        .        .        .        .
GACGGCGAGTGTGTGGAGCCCGTGGACCCGTGCTTCAGAGCCAACTGCGAG

                        1090                1110
              .        .        .        .        .
TACCAGTGCCAGCCCCTGAACCAAACTAGCTACCTCTGCGTCTGCGCCGAG

              1130                1150                1170
              .        .        .        .        .        .
GGCTTCGCGCCCATTCCCCACGAGCCGCACAGGTGCCAGATGTTTTGCAAC

                        1190                1210
              .        .        .        .        .
CAGACTGCCTGTCCAGCCGACTGCGACCCCAACACCCAGGCTAGCTGTGAG

       1230                1250                1270
       .        .        .        .        .        .
TGCCCTGAAGGCTACATCCTGGACGACGGTTTCATCTGCACGGACATCGAC

                1290                1310
       .        .        .        .        .
GAGTGCGAAAACGGCGGCTTCTGCTCCGGGGTGTGCCACAACCTCCCCGGT

1330                1350                1370
       .        .        .        .        .
ACCTTCGAGTGCATCTGCGGGCCCGACTCGGCCCTTGCCCGCCACATTGGC

       1390                1410                14
       .        .        .        .        .        .
ACCGACTGTGACTCCGGCAAGGTGGACGGTGGCGACAGCGGCTCTGGCGAG

30                  1450                1470
       .        .        .        .        .
CCCCCGCCCAGCCCGACGCCCGGCTCCACCTTGACTCCTCCGGCCGTGGGG

       1490
       .        .
CTCGTGCATTCG-3',
```

wherein
A is deoxyadenyl,
G is deoxyguanyl,
C is deoxycytidyl, and
T is thymidyl.

16. A recombinant DNA vector comprising the DNA sequence of Claim 14 or Claim 15.

17. Plasmid pUC18TMD1 or plasmid phdTMD1.

18. A host cell transformed with a DNA sequence of any one of Claims 14 to 17.

19. The host cell of Claim 18 which is a prokaryotic host cell.

20. The host cell of Claim 18 which is a eukaryotic host cell.

21. The host cell of Claim 20 which is an AV12 or 293 cell.

22. A transformed host cell selected from the group consisting of E. coli/pUC18Tmlinker, E. coli/pUC18TM, E. coli/pUC18TMD1, and E. coli/phdTMD1.

23. A transformed host cell selected from the group consisting of AV12/phdTMD1 and 293/phdTMD1.

24. A pharmaceutical formulation which comprises, as an active ingredient, an amino acid sequence as claimed in any of Claims 1 to 13, associated with a pharmaceutically-acceptable carrier, diluent, or excipient therefor.

25. A polypeptide product, as defined in any one of Claims 1 to 13, for use in therapy.

Claims for the following Contracting State: ES

1. A process for preparing an amino acid sequence which, in order from the N-terminus, comprises:
a) the N-terminal;
b) epidermal growth factor homology; and
c) serine/threonine rich regions of human thrombomodulin, said amino acid sequence lacking the transmembrane and cytoplasmic domains of human thrombomodulin, which comprises culturing, under conditions suitable for expression, an AV12 or 293 host cell transformed with a recombinant DNA vector encoding said amino acid sequence.

2. A process for preparing an amino acid sequence which is:

$$H_2N-(R)_x(R^1)_y-\text{AlaProAlaGluProGlnProGlyGlySerGlnCysValGluHisAsp}$$

CysPheAlaLeuTyrProGlyProAlaThrPheLeuAsnAlaSerGlnIle

CysAspGlyLeuArgGlyHisLeuMetThrValArgSerSerValAlaAla

AspValIleSerLeuLeuLeuAsnGlyAspGlyGlyValGlyArgArgArg

LeuTrpIleGlyLeuGlnLeuProProGlyCysGlyAspProLysArgLeu

GlyProLeuArgGlyPheGlnTrpValThrGlyAspAsnAsnThrSerTyr

SerArgTrpAlaArgLeuAspLeuAsnGlyAlaProLeuCysGlyProLeu

CysValAlaValSerAlaAlaGluAlaThrValProSerGluProIleTrp

GluGluGlnGlnCysGluValLysAlaAspGlyPheLeuCysGluPheHis

PheProAlaThrCysArgProLeuAlaValGluProGlyAlaAlaAlaAla

```
AlaValSerIleThrTyrGlyThrProPheAlaAlaArgGlyAlaAspPhe

GlnAlaLeuProValGlySerSerAlaAlaValAlaProLeuGlyLeuGln

LeuMetCysThrAlaProProGlyAlaValGlnGlyHisTrpAlaArgGlu

AlaProGlyAlaTrpAspCysSerValGluAsnGlyGlyCysGluHisAla

CysAsnAlaIleProGlyAlaProArgCysGlnCysProAlaGlyAlaAla

LeuGlnAlaAspGlyArgSerCysThrAlaSerAlaThrGlnSerCysAsn

AspLeuCysGluHisPheCysValProAsnProAspGlnProGlySerTyr

SerCysMetCysGluThrGlyTyrArgLeuAlaAlaAspGlnHisArgCys

GluAspValAspAspCysIleLeuGluProSerProCysProGlnArgCys

ValAsnThrGlnGlyGlyPheGluCysHisCysTyrProAsnTyrAspLeu

ValAspGlyGluCysValGluProValAspProCysPheArgAlaAsnCys

GluTyrGlnCysGlnProLeuAsnGlnThrSerTyrLeuCysValCysAla

GluGlyPheAlaProIleProHisGluProHisArgCysGlnMetPheCys

AsnGlnThrAlaCysProAlaAspCysAspProAsnThrGlnAlaSerCys

GluCysProGluGlyTyrIleLeuAspAspGlyPheIleCysThrAspIle

AspGluCysGluAsnGlyGlyPheCysSerGlyValCysHisAsnLeuPro

GlyThrPheGluCysIleCysGlyProAspSerAlaLeuAlaArgHisIle

GlyThrAspCysAspSerGlyLysValAspGlyGlyAspSerGlySerGly

GluProProProSerProThrProGlySerThrLeuThrProProAlaVal

GlyLeuValHisSer-COOH
```

wherein
ALA is Alanine, ARG is Arginine, ASN is Asparagine, ASP is Aspartic Acid, CYS is Cysteine, GLN is Glutamine, GLU is Glutamic Acid, GLY is Glycine, HIS is Histidine, ILE is Isoleucine, LEU is Leucine, LYS is Lysine, MET is Methionine, PHE is Phenylalanine, PRO is Proline, SER is Serine, THR is Threonine, TRP is Tryptophan, TYR is Tyrosine, and VAL is Valine;
R is MetLeuGlyValLeuValLeuGlyAlaLeuAlaLeuAla GlyLeuGly-;
$R^1$ is PhePro-;
x is 0 or 1;
y is 0 or 1, provided that if y = 0, then x must be 0 and if x = 1, then y must be 1, which comprises culturing, under conditions suitable for expression, a host cell transformed with a recombinant DNA vector encoding said amino acid sequence.

3. A process as claimed in Claims 1 or 2 in which the amino acid sequence is glycosylated.

4. A process as claimed in Claims 1 or 2 in which the amino acid sequence is not glycosylated.

5. A process as claimed in Claims 1 or 2 in which the amino acid sequence is

$H_2$N-AlaProAlaGluProGlnProGlyGlySerGlnCysValGluHisAspCys
PheAlaLeuTyrProGlyProAlaThrPheLeuAsnAlaSerGlnIleCys
AspGlyLeuArgGlyHisLeuMetThrValArgSerSerValAlaAlaAsp
ValIleSerLeuLeuLeuAsnGlyAspGlyGlyValGlyArgArgArgLeu
TrpIleGlyLeuGlnLeuProProGlyCysGlyAspProLysArgLeuGly
ProLeuArgGlyPheGlnTrpValThrGlyAspAsnAsnThrSerTyrSer
ArgTrpAlaArgLeuAspLeuAsnGlyAlaProLeuCysGlyProLeuCys
ValAlaValSerAlaAlaGluAlaThrValProSerGluProIleTrpGlu
GluGlnGlnCysGluValLysAlaAspGlyPheLeuCysGluPheHisPhe
ProAlaThrCysArgProLeuAlaValGluProGlyAlaAlaAlaAlaAla
ValSerIleThrTyrGlyThrProPheAlaAlaArgGlyAlaAspPheGln
AlaLeuProValGlySerSerAlaAlaValAlaProLeuGlyLeuGlnLeu
MetCysThrAlaProProGlyAlaValGlnGlyHisTrpAlaArgGluAla
ProGlyAlaTrpAspCysSerValGluAsnGlyGlyCysGluHisAlaCys
AsnAlaIleProGlyAlaProArgCysGlnCysProAlaGlyAlaAlaLeu
GlnAlaAspGlyArgSerCysThrAlaSerAlaThrGlnSerCysAsnAsp


LeuCysGluHisPheCysValProAsnProAspGlnProGlySerTyrSer
CysMetCysGluThrGlyTyrArgLeuAlaAlaAspGlnHisArgCysGlu
AspValAspAspCysIleLeuGluProSerProCysProGlnArgCysVal
AsnThrGlnGlyGlyPheGluCysHisCysTyrProAsnTyrAspLeuVal
AspGlyGluCysValGluProValAspProCysPheArgAlaAsnCysGlu
TyrGlnCysGlnProLeuAsnGlnThrSerTyrLeuCysValCysAlaGlu
GlyPheAlaProIleProHisGluProHisArgCysGlnMetPheCysAsn
GlnThrAlaCysProAlaAspCysAspProAsnThrGlnAlaSerCysGlu
CysProGluGlyTyrIleLeuAspAspGlyPheIleCysThrAspIleAsp
GluCysGluAsnGlyGlyPheCysSerGlyValCysHisAsnLeuProGly
ThrPheGluCysIleCysGlyProAspSerAlaLeuAlaArgHisIleGly
ThrAspCysAspSerGlyLysValAspGlyGlyAspSerGlySerGlyGly
ProProProSerProThrProGlySerThrLeuThrProProAlaValGly
LeuValHisSer-COOH


wherein
ALA is Alanine, ARG is Arginine, ASN is Asparagine, ASP is Aspartic Acid, CYS is Cysteine, GLN is Glutamine, GLU is Glutamic Acid, GLY is Glycine, HIS is Histidine, ILE is Isoleucine, LEU is Leucine, LYS is Lysine, MET is Methionine, PHE is Phenylalanine, PRO is Proline, SER is Serine, THR is Threonine, TRP is Tryptophan, TYR is Tyrosine, and VAL is Valine.

6. A process as claimed in Claim 5 in which the amino acid sequence is glycosylated.

7. A process as claimed in Claim 5 in which the amino acid sequence is not glycosylated.

8. A process for preparing a polypeptide product which comprises culturing, under conditions suitable for expression, a host cell, said host cell transformed with a recombinant DNA expression vector comprising a DNA sequence, said DNA sequence being:

$$5'-(R')_x(R^{1'})_y-\text{GCACCCGCAGAGCCGCAGCCGGGTGGCAGCCAGTGCGTCGAGCACGAC}$$

TGCTTCGCGCTCTACCCGGGCCCCGCGACCTTCCTCAATGCCAGTCAGATC

TGCGACGGACTGCGGGGCCACCTAATGACAGTGCGCTCCTCGGTGGCTGCC

GATGTCATTTCCTTGCTACTGAACGGCGACGGCGGCGTTGGCCGCCGGCGC

CTCTGGATCGGCCTGCAGCTGCCACCCGGCTGCGGCGACCCCAAGCGCCTC

GGGCCCCTGCGCGGCTTCCAGTGGGTTACGGGAGACAACAACACCAGCTAT

AGCAGGTGGGCACGGCTCGACCTCAATGGGGCTCCCCTCTGCGGCCCGTTG

TGCGTCGCTGTCTCCGCTGCTGAGGCCACTGTGCCCAGCGAGCCGATCTGG

GAGGAGCAGCAGTGCGAAGTGAAGGCCGATGGCTTCCTCTGCGAGTTCCAC

TTCCCAGCCACCTGCAGGCCACTGGCTGTGGAGCCCGGCGCCGCGGCTGCC

GCCGTCTCGATCACCTACGGCACCCCGTTCGCGGCCCGCGGAGCGGACTTC

```
                    630                 650
CAGGCGCTGCCGGTGGGCAGCTCCGCCGCGGTGGCTCCCCTCGGCTTACAG

          670                 690                 710
CTAATGTGCACCGCGCCGCCCGGAGCGGTCCAGGGGCACTGGGCCAGGGAG

                730                 750
GCGCCGGGCGCTTGGGACTGCAGCGTGGAGAACGGCGGCTGCGAGCACGCG

          770                 790                 810
TGCAATGCGATCCCTGGGGCTCCCCGCTGCCAGTGCCCAGCCGGCGCCGCC

                830                 850
CTGCAGGCAGACGGGCGCTCCTGCACCGCATCCGCGACGCAGTCCTGCAAC

        870                 890                 910
GACCTCTGCGAGCACTTCTGCGTTCCCAACCCCGACCAGCCGGGCTCCTAC

                930                 950                 97
TCGTGCATGTGCGAGACCGGCTACCGGCTGGCGGCCGACCAACACCGGTGC

        0                 990                 1010
GAGGACGTGGATGACTGCATACTGGAGCCCAGTCCGTGTCCGCAGCGCTGT

              1030                1050                1070
GTCAACACACAGGGTGGCTTCGAGTGCCACTGCTACCCTAACTACGACCTG

                1090                1110
GTGGACGGCGAGTGTGTGGAGCCCGTGGACCCGTGCTTCAGAGCCAACTGC

          1130                1150                1170
GAGTACCAGTGCCAGCCCCTGAACCAAACTAGCTACCTCTGCGTCTGCGCC
```

61

```
          1190                    1210
           .          .          .          .
GAGGGCTTCGCGCCCATTCCCCACGAGCCGCACAGGTGCCAGATGTTTTGC

      1230           1250           1270
       .          .          .          .
AACCAGACTGCCTGTCCAGCCGACTGCGACCCCAACACCCAGGCTAGCTGT

      1290           1310
       .          .          .          .
GAGTGCCCTGAAGGCTACATCCTGGACGACGGTTTCATCTGCACGGACATC

   1330           1350           1370
    .          .          .          .
GACGAGTGCGAAAACGGCGGCTTCTGCTCCGGGGTGTGCCACAACCTCCCC

      1390           1410             14
       .          .          .          .
GGTACCTTCGAGTGCATCTGCGGGCCCGACTCGGCCCTTGCCCGCCACATT

30                1450           1470
    .          .          .          .          .
GGCACCGACTGTGACTCCGGCAAGGTGGACGGTGGCGACAGCGGCTCTGGC

      1490           1510           1530
       .          .          .          .          .
GAGCCCCCGCCCAGCCCGACGCCCGGCTCCACCTTGACTCCTCCGGCCGTG

                .
GGGCTCGTGCATTCG-3'
```

in which

R' is 5'-ATGCTTGGGGTCCTGGTCCTTGGCGCGCTG GCCCTGGCCGGCCTGGGG-3';

R¹' is 5'-TTCCCC-3';

x is 0 or 1;

y is 0 or 1, provided that if y = 0, then x must be 0, and if x = 1, then y must be 1;

A is deoxyadenyl,

G is deoxyguanyl,

C is deoxycytidyl, and T is thymidyl.

9. A process as claimed in Claim 8 in which the polypeptide product is expressed in a prokaryotic host cell.

10. A process as claimed in Claim 8 in which the polypeptide product is expressed in a eukaryotic host cell.

11. A process as claimed in Claim 10 in which the host cell is a 293 or AV12 cell.

12. A process as claimed in Claim 11 in which the polypeptide product has a molecular weight of about 110 kD (reducing conditions) and about 76-94 kD (non-reducing conditions).

13. A process as claimed in Claim 11 in which the polypeptide product has a molecular weight of about 73 to about 77 kD (reducing conditions) and about 58-66 kD (non-reducing conditions).

14. A process as claimed in any one of Claims 1 to 13, in which the host cell is cultured at a temperature of about 25° C to about 40° C.

15. A process for preparing a pharmaceutical formulation which comprises admixing a polypeptide, as defined in any one of Claims 1 to 13, with a pharmaceutically acceptable carrier, diluent, or excipient therefor.

Claims for the following Contracting State: GR

1. A process for preparing an amino acid sequence which, in order from the N-terminus, comprises:

a) the N-terminal;

b) epidermal growth factor homology; and

c) serine/threonine rich regions of human thrombomodulin, said amino acid sequence lacking the transmembrane and cytoplasmic domains of human thrombomodulin, which comprises culturing, under conditions suitable for expression, an AV12 or 293 host cell transformed with a recombinant DNA vector encoding said amino acid sequence.

2. A process for preparing an amino acid sequence which is:

$$H_2N-(R)_x(R^1)_y-AlaProAlaGluProGlnProGlyGlySerGlnCysValGluHisAsp$$

CysPheAlaLeuTyrProGlyProAlaThrPheLeuAsnAlaSerGlnIle

CysAspGlyLeuArgGlyHisLeuMetThrValArgSerSerValAlaAla

AspValIleSerLeuLeuLeuAsnGlyAspGlyGlyValGlyArgArgArg

LeuTrpIleGlyLeuGlnLeuProProGlyCysGlyAspProLysArgLeu

GlyProLeuArgGlyPheGlnTrpValThrGlyAspAsnAsnThrSerTyr

SerArgTrpAlaArgLeuAspLeuAsnGlyAlaProLeuCysGlyProLeu

CysValAlaValSerAlaAlaGluAlaThrValProSerGluProIleTrp

GluGluGlnGlnCysGluValLysAlaAspGlyPheLeuCysGluPheHis

PheProAlaThrCysArgProLeuAlaValGluProGlyAlaAlaAlaAla

AlaValSerIleThrTyrGlyThrProPheAlaAlaArgGlyAlaAspPhe

GlnAlaLeuProValGlySerSerAlaAlaValAlaProLeuGlyLeuGln

LeuMetCysThrAlaProProGlyAlaValGlnGlyHisTrpAlaArgGlu

AlaProGlyAlaTrpAspCysSerValGluAsnGlyGlyCysGluHisAla

CysAsnAlaIleProGlyAlaProArgCysGlnCysProAlaGlyAlaAla

LeuGlnAlaAspGlyArgSerCysThrAlaSerAlaThrGlnSerCysAsn

AspLeuCysGluHisPheCysValProAsnProAspGlnProGlySerTyr

SerCysMetCysGluThrGlyTyrArgLeuAlaAlaAspGlnHisArgCys

GluAspValAspAspCysIleLeuGluProSerProCysProGlnArgCys

ValAsnThrGlnGlyGlyPheGluCysHisCysTyrProAsnTyrAspLeu

ValAspGlyGluCysValGluProValAspProCysPheArgAlaAsnCys

GluTyrGlnCysGlnProLeuAsnGlnThrSerTyrLeuCysValCysAla

GluGlyPheAlaProIleProHisGluProHisArgCysGlnMetPheCys

AsnGlnThrAlaCysProAlaAspCysAspProAsnThrGlnAlaSerCys

GluCysProGluGlyTyrIleLeuAspAspGlyPheIleCysThrAspIle

AspGluCysGluAsnGlyGlyPheCysSerGlyValCysHisAsnLeuPro

GlyThrPheGluCysIleCysGlyProAspSerAlaLeuAlaArgHisIle

GlyThrAspCysAspSerGlyLysValAspGlyGlyAspSerGlySerGly

GluProProProSerProThrProGlySerThrLeuThrProProAlaVal

GlyLeuValHisSer-COOH

wherein

ALA is Alanine, ARG is Arginine, ASN is Asparagine, ASP is Aspartic Acid, CYS is Cysteine, GLN is Glutamine, GLU is Glutamic Acid, GLY is Glycine, HIS is Histidine, ILE is Isoleucine, LEU is Leucine, LYS is Lysine, MET is Methionine, PHE is Phenylalanine, PRO is Proline, SER is Serine, THR is Threonine, TRP is Tryptophan, TYR is Tyrosine, and VAL is Valine;

R is MetLeuGlyValLeuValLeuGlyAlaLeuAlaLeuAla GlyLeuGly-;

R' is PhePro-;

x is 0 or 1;

y is 0 or 1, provided that if y = 0, then x must be 0 and if x = 1, then y must be 1, which comprises culturing, under conditions suitable for expression, a host cell transformed with a recombinant DNA vector encoding said amino acid sequence.

3. A process as claimed in Claims 1 or 2 in which the amino acid sequence is glycosylated.

4. A process as claimed in Claims 1 or 2 in which the amino acid sequence is not glycosylated.

5. A process as claimed in Claims 1 or 2 in which the amino acid sequence is

$H_2N$-AlaProAlaGluProGlnProGlyGlySerGlnCysValGluHisAspCys

PheAlaLeuTyrProGlyProAlaThrPheLeuAsnAlaSerGlnIleCys

AspGlyLeuArgGlyHisLeuMetThrValArgSerSerValAlaAlaAsp

ValIleSerLeuLeuLeuAsnGlyAspGlyGlyValGlyArgArgArgLeu

TrpIleGlyLeuGlnLeuProProGlyCysGlyAspProLysArgLeuGly

ProLeuArgGlyPheGlnTrpValThrGlyAspAsnAsnThrSerTyrSer

ArgTrpAlaArgLeuAspLeuAsnGlyAlaProLeuCysGlyProLeuCys

ValAlaValSerAlaAlaGluAlaThrValProSerGluProIleTrpGlu

GluGlnGlnCysGluValLysAlaAspGlyPheLeuCysGluPheHisPhe

ProAlaThrCysArgProLeuAlaValGluProGlyAlaAlaAlaAlaAla

ValSerIleThrTyrGlyThrProPheAlaAlaArgGlyAlaAspPheGln

AlaLeuProValGlySerSerAlaAlaValAlaProLeuGlyLeuGlnLeu

MetCysThrAlaProProGlyAlaValGlnGlyHisTrpAlaArgGluAla

ProGlyAlaTrpAspCysSerValGluAsnGlyGlyCysGluHisAlaCys

AsnAlaIleProGlyAlaProArgCysGlnCysProAlaGlyAlaAlaLeu

GlnAlaAspGlyArgSerCysThrAlaSerAlaThrGlnSerCysAsnAsp

wherein

ALA is Alanine, ARG is Arginine, ASN is Asparagine, ASP is Aspartic Acid, CYS is Cysteine, GLN is Glutamine, GLU is Glutamic Acid, GLY is Glycine, HIS is Histidine, ILE is Isoleucine, LEU is Leucine, LYS is Lysine, MET is Methionine, PHE is Phenylalanine, PRO is Proline, SER is Serine, THR is Threonine, TRP is Tryptophan, TYR is Tyrosine, and VAL is Valine.

6. A process as claimed in Claim 5 in which the amino acid sequence is glycosylated.

7. A process as claimed in Claim 5 in which the amino acid sequence is not glycosylated.

8. A process for preparing a polypeptide product which comprises culturing, under conditions suitable for expression, a host cell, said host cell transformed with a recombinant DNA expression vector comprising a DNA sequence, said DNA sequence being:

$$5'-(R')_x(R^{1'})_y-\text{GCACCCGCAGAGCCGCAGCCGGGTGGCAGCCAGTGCGTCGAGCACGAC}$$

TGCTTCGCGCTCTACCCGGGCCCCGCGACCTTCCTCAATGCCAGTCAGATC

TGCGACGGACTGCGGGGCCACCTAATGACAGTGCGCTCCTCGGTGGCTGCC

GATGTCATTTCCTTGCTACTGAACGGCGACGGCGGCGTTGGCCGCCGGCGC

CTCTGGATCGGCCTGCAGCTGCCACCCGGCTGCGGCGACCCCAAGCGCCTC

GGGCCCCTGCGCGGCTTCCAGTGGGTTACGGGAGACAACAACACCAGCTAT

AGCAGGTGGGCACGGCTCGACCTCAATGGGGCTCCCCTCTGCGGCCCGTTG

TGCGTCGCTGTCTCCGCTGCTGAGGCCACTGTGCCCAGCGAGCCGATCTGG

GAGGAGCAGCAGTGCGAAGTGAAGGCCGATGGCTTCCTCTGCGAGTTCCAC

TTCCCAGCCACCTGCAGGCCACTGGCTGTGGAGCCCGGCGCCGCGGCTGCC

GCCGTCTCGATCACCTACGGCACCCCGTTCGCGGCCCGCGGAGCGGACTTC

630                              650

CAGGCGCTGCCGGTGGGCAGCTCCGCCGCGGTGGCTCCCCTCGGCTTACAG

670                  690                      710

CTAATGTGCACCGCGCCGCCCGGAGCGGTCCAGGGGCACTGGGCCAGGGAG

730                  750

GCGCCGGGCGCTTGGGACTGCAGCGTGGAGAACGGCGGCTGCGAGCACGCG

770                  790                      810

TGCAATGCGATCCCTGGGGCTCCCCGCTGCCAGTGCCCAGCCGGCGCCGCC

830                  850

CTGCAGGCAGACGGGCGCTCCTGCACCGCATCCGCGACGCAGTCCTGCAAC

870                  890                      910

GACCTCTGCGAGCACTTCTGCGTTCCCAACCCCGACCAGCCGGGCTCCTAC

930                  950                      97

TCGTGCATGTGCGAGACCGGCTACCGGCTGGCGGCCGACCAACACCGGTGC

0                    990                      1010

GAGGACGTGGATGACTGCATACTGGAGCCCAGTCCGTGTCCGCAGCGCTGT

1030                 1050                     1070

GTCAACACACAGGGTGGCTTCGAGTGCCACTGCTACCCTAACTACGACCTG

1090                 1110

GTGGACGGCGAGTGTGTGGAGCCCGTGGACCCGTGCTTCAGAGCCAACTGC

1130                 1150                     1170

GAGTACCAGTGCCAGCCCCTGAACCAAACTAGCTACCTCTGCGTCTGCGCC

1190    1210

GAGGGCTTCGCGCCCATTCCCCACGAGCCGCACAGGTGCCAGATGTTTTGC

1230    1250    1270

AACCAGACTGCCTGTCCAGCCGACTGCGACCCCAACACCCAGGCTAGCTGT

1290    1310

GAGTGCCCTGAAGGCTACATCCTGGACGACGGTTTCATCTGCACGGACATC

1330    1350    1370

GACGAGTGCGAAAACGGCGGCTTCTGCTCCGGGGTGTGCCACAACCTCCCC

1390    1410    14

GGTACCTTCGAGTGCATCTGCGGGCCCGACTCGGCCCTTGCCCGCCACATT

30    1450    1470

GGCACCGACTGTGACTCCGGCAAGGTGGACGGTGGCGACAGCGGCTCTGGC

1490    1510    1530

GAGCCCCCGCCCAGCCCGACGCCCGGCTCCACCTTGACTCCTCCGGCCGTG

GGGCTCGTGCATTCG-3'

in which

R' is 5'-ATGCTTGGGGTCCTGGTCCTTGGCGCGCTG GCCCTGGCCGGCCTGGGG-3';

R¹' is 5'-TTCCCC-3';

x is 0 or 1;

y is 0 or 1, provided that if y = 0, then x must be 0, and if x = 1, then y must be 1;

A is deoxyadenyl,

G is deoxyguanyl,

C is deoxycytidyl, and T is thymidyl.

9. A process as claimed in Claim 8 in which the polypeptide product is expressed in a prokaryotic host cell.

10. A process as claimed in Claim 8 in which the polypeptide product is expressed in a eukaryotic host cell.

11. A process as claimed in Claim 10 in which the host cell is a 293 or AV12 cell.

12. A process as claimed in Claim 11 in which the polypeptide product has a molecular weight of about 110 kD (reducing conditions) and about 76-94 kD (non-reducing conditions).

13. A process as cliamed in Claim 11 in which the polypeptide product has a molecular weight of about 73 to about 77 kD (reducing conditions) and about 58-66 kD (non-reducing conditions).

14. A constructed DNA compound which comprises the sequence:

$$5'-(R')_x(R^{1'})_y-\text{GCACCCGCAGAGCCGCAGCCGGGTGGCAGCCAGTGCGTCGAGCACGAC}$$

TGCTTCGCGCTCTACCCGGGCCCCGCGACCTTCCTCAATGCCAGTCAGATC

TGCGACGGACTGCGGGGCCACCTAATGACAGTGCGCTCCTCGGTGGCTGCC

GATGTCATTTCCTTGCTACTGAACGGCGACGGCGGCGTTGGCCGCCGGCGC

CTCTGGATCGGCCTGCAGCTGCCACCCGGCTGCGGCGACCCCAAGCGCCTC

GGGCCCCTGCGCGGCTTCCAGTGGGTTACGGGAGACAACAACACCAGCTAT

AGCAGGTGGGCACGGCTCGACCTCAATGGGGCTCCCCTCTGCGGCCCGTTG

TGCGTCGCTGTCTCCGCTGCTGAGGCCACTGTGCCCAGCGAGCCGATCTGG

GAGGAGCAGCAGTGCGAAGTGAAGGCCGATGGCTTCCTCTGCGAGTTCCAC

TTCCCAGCCACCTGCAGGCCACTGGCTGTGGAGCCCGGCGCCGCGGCTGCC

GCCGTCTCGATCACCTACGGCACCCCGTTCGCGGCCCGCGGAGCGGACTTC

68

630                     650

CAGGCGCTGCCGGTGGGCAGCTCCGCCGCGGTGGCTCCCCTCGGCTTACAG

670                     690                     710

CTAATGTGCACCGCGCCGCCCGGAGCGGTCCAGGGGCACTGGGCCAGGGAG

730                     750

GCGCCGGGCGCTTGGGACTGCAGCGTGGAGAACGGCGGCTGCGAGCACGCG

770                     790                     810

TGCAATGCGATCCCTGGGGCTCCCCGCTGCCAGTGCCCAGCCGGCGCCGCC

830                     850

CTGCAGGCAGACGGGCGCTCCTGCACCGCATCCGCGACGCAGTCCTGCAAC

870                     890                     910

GACCTCTGCGAGCACTTCTGCGTTCCCAACCCCGACCAGCCGGGCTCCTAC

930                     950                     97

TCGTGCATGTGCGAGACCGGCTACCGGCTGGCGGCCGACCAACACCGGTGC

0                       990                     1010

GAGGACGTGGATGACTGCATACTGGAGCCCAGTCCGTGTCCGCAGCGCTGT

1030                    1050                    1070

GTCAACACACAGGGTGGCTTCGAGTGCCACTGCTACCCTAACTACGACCTG

1090                    1110

GTGGACGGCGAGTGTGTGGAGCCCGTGGACCCGTGCTTCAGAGCCAACTGC

1130                    1150                    1170

GAGTACCAGTGCCAGCCCCTGAACCAAACTAGCTACCTCTGCGTCTGCGCC

```
                    1190                    1210
                      .           .                    .
      GAGGGCTTCGCGCCCATTCCCCACGAGCCGCACAGGTGCCAGATGTTTTGC

        1230              1250                    1270
          .           .           .           .           .
      AACCAGACTGCCTGTCCAGCCGACTGCGACCCCAACACCCAGGCTAGCTGT

              1290                    1310
                .           .           .           .
      GAGTGCCCTGAAGGCTACATCCTGGACGACGGTTTCATCTGCACGGACATC

        1330              1350                    1370
          .           .           .           .           .
      GACGAGTGCGAAAACGGCGGCTTCTGCTCCGGGGTGTGCCACAACCTCCCC

              1390                    1410                    14
                .           .           .           .           .
      GGTACCTTCGAGTGCATCTGCGGGCCCGACTCGGCCCTTGCCCGCCACATT

        30                1450                    1470
          .           .           .           .           .
      GGCACCGACTGTGACTCCGGCAAGGTGGACGGTGGCGACAGCGGCTCTGGC

              1490                    1510                    1530
                .           .           .           .           .
      GAGCCCCCGCCCAGCCCGACGCCCGGCTCCACCTTGACTCCTCCGGCCGTG

                      .
      GGGCTCGTGCATTCG-3'
```

in which
R′ is 5′-ATGCTTGGGGTCCTGGTCCTTGGCGCGCTG GCCCTGGCCGGCCTGGGG-3′;
R¹′ is 5′-TTCCCC-3′;
x is 0 or 1;
y is 0 or 1, provided that if y = 0, then x must be 0, and if x = 1, then y must be 1;
A is deoxyadenyl,
G is deoxyguanyl,
C is deoxycytidyl, and T is thymidyl.
15. A DNA compound of Claim 14 which comprises the sequence:

```
                10                      30                      50
                 .                       .                       .
GCACCCGCAGAGCCGCAGCCGGGTGGCAGCCAGTGCGTCGAGCACGACTGC

                          70                      90
                 .         .             .         .             .
TTCGCGCTCTACCCGGGCCCCGCGACCTTCCTCAATGCCAGTCAGATCTGC

                110                     130                     150
                 .         .             .         .             .
GACGGACTGCGGGGCCACCTAATGACAGTGCGCTCCTCGGTGGCTGCCGAT

                          170                     190
                 .         .             .         .             .
GTCATTTCCTTGCTACTGAACGGCGACGGCGGCGTTGGCCGCCGGCGCCTC

                210                     230                     250
                 .         .             .         .             .
TGGATCGGCCTGCAGCTGCCACCCGGCTGCGGCGACCCCAAGCGCCTCGGG

                          270                     290
                 .         .             .         .             .
CCCCTGCGCGGCTTCCAGTGGGTTACGGGAGACAACAACACCAGCTATAGC

                310                     330                     350
                 .         .             .         .             .
AGGTGGGCACGGCTCGACCTCAATGGGGCTCCCCTCTGCGGCCCGTTGTGC

                          370                     390                      4
                 .         .             .         .             .
GTCGCTGTCTCCGCTGCTGAGGCCACTGTGCCCAGCGAGCCGATCTGGGAG

                10                      430          .          450
                 .         .             .         .             .
GAGCAGCAGTGCGAAGTGAAGGCCGATGGCTTCCTCTGCGAGTTCCACTTC
```

71

```
                470                 490                 510
         .       .        .       .        .        .       .
  CCAGCCACCTGCAGGCCACTGGCTGTGGAGCCCGGCGCCGCGGCTGCCGCC

                    530                 550
         .       .        .       .        .        .       .
  GTCTCGATCACCTACGGCACCCCGTTCGCGGCCCGCGGAGCGGACTTCCAG

                570                 590                 610
         .       .        .       .        .        .       .
  GCGCTGCCGGTGGGCAGCTCCGCCGCGGTGGCTCCCCTCGGCTTACAGCTA

                    630                 650
         .       .        .       .        .        .       .
  ATGTGCACCGCGCCGCCCGGAGCGGTCCAGGGGCACTGGGCCAGGGAGGCG

                670                 690                 710
         .       .        .       .        .        .       .
  CCGGGCGCTTGGGACTGCAGCGTGGAGAACGGCGGCTGCGAGCACGCGTGC

                    730                 750
         .       .        .       .        .        .       .
  AATGCGATCCCTGGGGCTCCCCGCTGCCAGTGCCCAGCCGGCGCCGCCCTG

                770                 790                 810
         .       .        .       .        .        .       .
  CAGGCAGACGGGCGCTCCTGCACCGCATCCGCGACGCAGTCCTGCAACGAC

                    830                 850
         .       .        .       .        .        .       .
  CTCTGCGAGCACTTCTGCGTTCCCAACCCCGACCAGCCGGGCTCCTACTCG

             870                 890                 910
         .       .        .       .        .        .       .
  TGCATGTGCGAGACCGGCTACCGGCTGGCGGCCGACCAACACCGGTGCGAG

                    930                 950                  97
         .       .        .       .        .        .       .
  GACGTGGATGACTGCATACTGGAGCCCAGTCCGTGTCCGCAGCGCTGTGTC

             0                  990                 1010
         .       .        .       .        .        .       .
  AACACACAGGGTGGCTTCGAGTGCCACTGCTACCCTAACTACGACCTGGTG
```

```
                    1030              1050              1070
                     .        .       .        .        .
         GACGGCGAGTGTGTGGAGCCCGTGGACCCGTGCTTCAGAGCCAACTGCGAG

                           1090              1110
                     .        .       .        .        .
         TACCAGTGCCAGCCCCTGAACCAAACTAGCTACCTCTGCGTCTGCGCCGAG

                    1130              1150              1170
                     .        .       .        .        .
         GGCTTCGCGCCCATTCCCCACGAGCCGCACAGGTGCCAGATGTTTTGCAAC

                           1190              1210
                     .        .       .        .        .
         CAGACTGCCTGTCCAGCCGACTGCGACCCCAACACCCAGGCTAGCTGTGAG

                    1230              1250              1270
                     .        .       .        .        .
         TGCCCTGAAGGCTACATCCTGGACGACGGTTTCATCTGCACGGACATCGAC

                           1290              1310
                     .        .       .        .        .
         GAGTGCGAAAACGGCGGCTTCTGCTCCGGGGTGTGCCACAACCTCCCCGGT

         1330              1350              1370
          .        .       .        .        .
         ACCTTCGAGTGCATCTGCGGGCCCGACTCGGCCCTTGCCCGCCACATTGGC

                    1390              1410              14
                     .        .       .        .        .
         ACCGACTGTGACTCCGGCAAGGTGGACGGTGGCGACAGCGGCTCTGGCGAG

         30                1450              1470
          .        .       .        .        .
         CCCCCGCCCAGCCCGACGCCCGGCTCCACCTTGACTCCTCCGGCCGTGGGG

                    1490
                     .        .
         CTCGTGCATTCG-3',
```

wherein
A is deoxyadenyl,
G is deoxyguanyl,
C is deoxycytidyl, and
T is thymidyl.

16. A recombinant DNA vector comprising the DNA sequence of Claim 14 or Claim 15.

17. Plasmid pUC18TMD1 or plasmid phdTMD1.

18. A host cell transformed with a DNA sequence of any one of Claims 14 to 17.

19. The host cell of Claim 18 which is a prokaryotic host cell.

20. The host cell of Claim 18 which is a eukaryotic host cell.

21. The host cell of Claim 20 which is an AV12 or 293 cell.

22. A transformed host cell selected from the group consisting of E. coli/pUC18TMlinker, E. coli/pUC18TM, E. coli/pUC18TMD1, and E. coli/phdTMD1.

23. A transformed host cell selected from the group consisting of AV12/phdTMD1 and 293/phdTMD1.

24. A pharmaceutical formulation which comprises, as an active ingredient, an amino acid sequence as defined in any of Claims 1 to 13, associated with a pharmaceutically-acceptable carrier, diluent, or excipient therefor.

# FIG.I

EP 0 412 841 A1

# FIG.2

Restriction and Function Map of
Plasmid pUC18
(~ 2.7 kb)

# FIG.3

Restriction and Function Map of
Plasmid   pUC18TMlinker
(~ 2.7   kb)

# FIG.4

Restriction and Function Map of
Plasmid pUC18TM

(~ 4.7   kb)

# FIG.5

Restriction and Function Map of
Plasmid pUC18TMD1
(~4.2 kb)

# FIG.6

Restriction and Function Map of
Plasmid phd
(~ 8.8 kb)

# FIG.7

Restriction and Function Map of
Plasmid phdTMD1
(~10.4 kb)

European
Patent Office

**EUROPEAN SEARCH
REPORT**

Application Number

**EP 90 30 8826**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | WO-A-8 809 811   (NOVO INDUSTRI)<br>* Claims 1,2,4,16-18,20,31-38; page 13, lines 12-30 *<br>— — — | 1,3,24-25 | C 12 P 21/02<br>C 12 N 15/12<br>C 12 N 1/21 |
| X | EP-A-0 312 598   (ASAHI KASEI KOGYO)<br>* Claims; page 14, lines 2-17 *<br><br>— — — | 1-10,<br>14-16,<br>18-20,<br>24-25 | C 12 N 5/10<br>A 61 K 37/02 |
| X | THE JOURNAL OF BIOLOGICAL CHEMISTRY, vol. 264, no. 9, March 1989, pages 4872-4876; K. SUZUKI et al.: "A domain composed of epidermal growth factor-like structures of human thrombomodulin is essential for thrombin binding and for protein C activation"<br>* Whole article *<br>— — — | 1-3,8,10,<br>14,16,18,<br>20 | |
| P,X | THE JOURNAL OF BIOLOGICAL CHEMISTRY, vol. 265, no. 21, July 1990, pages 12602-12610; J.F. PARKINSON et al.: "Stable expression of a secretable deletion mutant of re-combinant human thrombomodulin in mammalian cells"<br>* Whole article *<br>— — — — — | 1-25 | |

| TECHNICAL FIELDS SEARCHED (Int. Cl.5) |
|---|
| C 12 N |

The present search report has been drawn up for all claims

| Place of search | Date of completion of search | Examiner |
|---|---|---|
| The Hague | 18 November 90 | HUBER A. |

CATEGORY OF CITED DOCUMENTS

X : particularly relevant if taken alone
Y : particularly relevant if combined with another
    document of the same catagory
A : technological background
O : non-written disclosure
P : intermediate document
T : theory or principle underlying the invention

E : earlier patent document, but published on, or after
    the filing date
D : document cited in the application
L : document cited for other reasons

&: member of the same patent family, corresponding
   document